# EUROPEAN PATENT APPLICATION

(11) **EP 2 359 825 A2**
(43) Date of publication of application: **24.08.2011**
(21) Application number: 11156096.7
(22) Date of filing: 05.10.2007
(51) Int. Cl.: A61K 31/337, A61K 31/395, A61K 31/4745, A61K 31/505, A61K 31/517, A61K 31/519, A61K 31/5377, A61K 31/565, A61K 31/704, A61P 35/00

(54) **Combination drug**

(30) Priority: 06.10.2006 JP 2006275841; 07.03.2007 JP 2007057902
(62) Divisional of application: 07829761.1
(71) Applicant: Takeda Pharmaceutical Company Limited, Osaka-shi, Osaka 541-0045 (JP)
(72) Inventor: Ohta, Yoshikazu, Ibaraki 300-4293 (JP); Tamura, Toshiya, Ibaraki 300-4293 (JP); Takagi, Shinji, Ibaraki 300-4293 (JP)
(74) Representative: Jones, Nicholas Andrew

(57) **Abstract**

The present invention provides a pharmaceutical agent containing an HER2 inhibitor and a hormonal therapeutic agent or anti-cancer agent in combination. The present invention provides a pharmaceutical agent containing (1) an HER2 inhibitor having a pyrrolopyrimidine skeleton or pyrazolopyrimidine skeleton, and (2) a hormonal therapeutic agent or an anti-cancer agent in combination.

## Description

### Technical Field

The present invention relates to a pharmaceutical agent comprising (1) an HER2 inhibitor having a pyrrolopyrimidine skeleton or pyrazolopyrimidine skeleton and (2) a hormonal therapeutic agent or anti-cancer agent in combination.

### Background of The Invention

W02005/010451 describes an HER2 inhibitor having a pyrrolopyrimidine skeleton or pyrazolopyrimidine skeleton.

W02005/120512 describes a therapeutic use of a combination of a compound represented by the formula 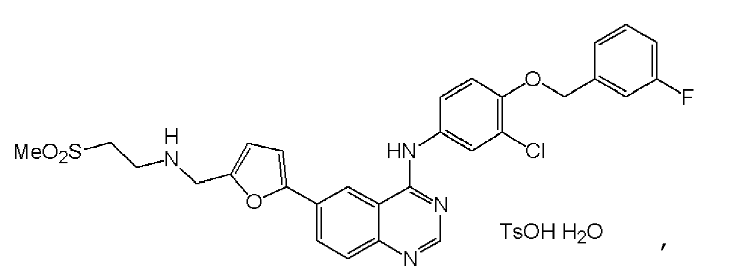 which is an HER2 inhibitor, and trastuzumab etc. for breast cancer.

### Disclosure of the Invention

The present invention aims at providing a pharmaceutical agent comprising (1) an HER2 inhibitor having a pyrrolopyrimidine skeleton or pyrazolopyrimidine skeleton and (2) a hormonal therapeutic agent or anti-cancer agent in combination (hereinafter sometimes to be abbreviated as the combination drug of the present invention), which is useful as an agent for the prophylaxis or treatment of cancer.

The present inventors have conducted intensive studies and found that the use of (1) an HER2 inhibitor having a pyrrolopyrimidine skeleton or pyrazolopyrimidine skeleton and (2) a hormonal therapeutic agent or anti-cancer agent in combination shows a significant anti-cancer action as compared to use as a single agent and use of other combination pharmaceutical agents, and further investigation resulted in the completion of the present invention. Accordingly, the present invention relates to
[1] a pharmaceutical agent comprising (1) an HER2 inhibitor having a pyrrolopyrimidine skeleton or pyrazolopyrimidine skeleton and (2) a hormonal therapeutic agent or anti-cancer agent in combination;
[2] the pharmaceutical agent of the above-mentioned [1], wherein the HER2 inhibitor having a pyrrolopyrimidine skeleton or pyrazolopyrimidine skeleton is a compound represented by the formula: 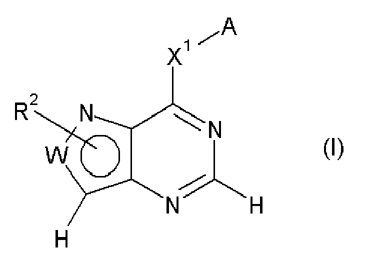 wherein W is C (R¹) or N,
   A is an optionally substituted aryl group or an optionally substituted heteroaryl group,
   X¹ is -NR³-Y¹-, -O-, -S-, -SO-, -SO₂- or -CHR³-
   wherein R³ is a hydrogen atom or an optionally substituted aliphatic hydrocarbon group, or R³ is optionally bonded to a carbon atom or a hetero atom on the aryl group or the heteroaryl group represented by A to form an optionally substituted ring structure, and
   Y¹ is a single bond or an optionally substituted C₁₋₄ alkylene or an optionally substituted -O-(C₁₋₄ alkylene)-,
   R¹ is a hydrogen atom or an optionally substituted group bonded via a carbon atom, a nitrogen atom or an oxygen atom, and R² is a hydrogen atom or an optionally substituted group bonded via a carbon atom or a sulfur atom, or
   R¹ and R², or R² and R³ are optionally bonded to form an optionally substituted ring structure, (sometimes referred to as compound (I) in the present specification), provided that the compounds represented by the formulas: 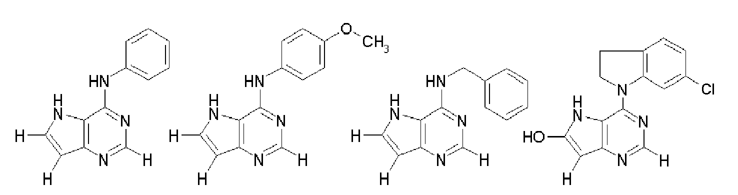 are excluded, or a salt thereof or a prodrug thereof;
[3] the pharmaceutical agent of the above-mentioned [1], wherein the HER2 inhibitor having a pyrrolopyrimidine skeleton or pyrazolopyrimidine skeleton is a compound represented by formula: 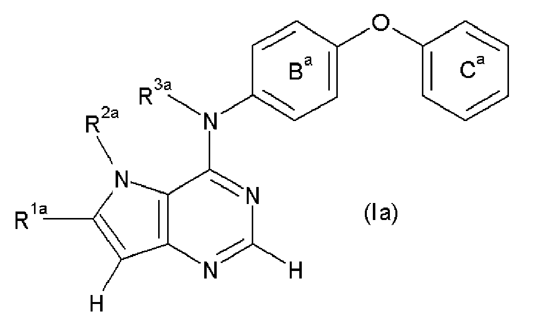 wherein R^{1a} is a hydrogen atom or an optionally substituted group bonded via a carbon atom, a nitrogen atom or an oxygen atom,
   R^{2a} is an optionally substituted group bonded via a carbon atom or a sulfur atom, or
   R^{1a} and R^{2a}, or R^{2a} and R^{3a} are optionally bonded to form an optionally substituted ring structure,
   R^{3a} is a hydrogen atom or an optionally substituted aliphatic hydrocarbon group, or
   R^{3a} is optionally bonded to a carbon atom of the adjacent phenyl group to form an optionally substituted ring structure, B^{a} is an optionally substituted benzene ring, and
   C^{a} is an optionally substituted C₆₋₁₈ aryl group, (sometimes referred to as compound (Ia) in the present specification) or a salt thereof or a prodrug thereof;
[4] the pharmaceutical agent of the above-mentioned [1], wherein the HER2 inhibitor having a pyrrolopyrimidine skeleton or pyrazolopyrimidine skeleton is N-{2-[4-({3-chloro-4-[3-(trifluoromethyl)phenoxy]phenyl}amino)-5H-pyrrolo[3,2-dlpyrimidin-5-yllethyll-3-hydroxy-3-methylbutanamide or a salt thereof or a prodrug thereof;
[5] the pharmaceutical agent of the above-mentioned [1], wherein the anti-cancer agent is an HER2 antibody, an EGFR antibody, an EGFR inhibitor, a VEGFR inhibitor or a chemotherapeutic agent;
[6] the pharmaceutical agent of the above-mentioned [1], wherein the anti-cancer agent is trastuzumab, cetuximab, erlotinib, gefitinib or paclitaxel;
[7] the pharmaceutical agent of the above-mentioned [1], wherein the anti-cancer agent is doxorubicin hydrochloride, irinotecan hydrochloride, 5FU, docetaxel or methotrexate;
[8] the pharmaceutical agent of the above-mentioned [1], wherein the HER2 inhibitor having a pyrrolopyrimidine skeleton or pyrazolopyrimidine skeleton is N-{2-[4-({3-chloro-4-[3-(trifluoromethyl)phenoxy]phenyl}amino)-5H-pyrrolo[3,2-dlpyrimidin-5-yllethyll-3-hydroxy-3-methylbutanamide or a salt thereof or a prodrug thereof, and the anti-cancer agent is an HER2 antibody, an EGFR antibody, an EGFR inhibitor, a VEGFR inhibitor or a chemotherapeutic agent;
[9] the pharmaceutical agent of the above-mentioned [1], wherein the HER2 inhibitor having a pyrrolopyrimidine skeleton or pyrazolopyrimidine skeleton is N-{2-[4-({3-chloro-4-[3-(trifluoromethyl)phenoxy]phenyl}amino)-5H-pyrrolo[3,2-dlpyrimidin-5-yllethyll-3-hydroxy-3-methylbutanamide or a salt thereof or a prodrug thereof, and the anti-cancer agent is trastuzumab, cetuximab, erlotinib, gefitinib or paclitaxel;
[10] the pharmaceutical agent of the above-mentioned [1], wherein the HER2 inhibitor having a pyrrolopyrimidine skeleton or pyrazolopyrimidine skeleton is N-{2-[4-({3-chloro-4-[3-(trifluoromethyl)phenoxy]phenyl}amino)-5H-pyrrolo[3,2-dlpyrimidin-5-yl]ethyll-3-hydroxy-3-methylbutanamide or a salt thereof or a prodrug thereof, and the anti-cancer agent is doxorubicin hydrochloride, irinotecan hydrochloride, 5FU, docetaxel or methotrexate;
[11] the pharmaceutical agent of the above-mentioned [1], wherein the hormonal therapeutic agent is an ER down-regulator;
[12] the pharmaceutical agent of the above-mentioned [1], wherein the hormonal therapeutic agent is fulvestrant;
[13] the pharmaceutical agent of the above-mentioned [1], wherein the HER2 inhibitor having a pyrrolopyrimidine skeleton or pyrazolopyrimidine skeleton is N-{2-[4-({3-chloro-4-[3-(trifluoromethyl)phenoxy]phenyl}amino)-5H-pyrrolo[3,2-dlpyrimidin-5-yllethyll-3-hydroxy-3-methylbutanamide or a salt thereof or a prodrug thereof, and the hormonal therapeutic agent is fulvestrant;
[14] the pharmaceutical agent of the above-mentioned [1], which is an agent for the prophylaxis or treatment of cancer;
[15] the pharmaceutical agent of the above-mentioned [14], wherein the cancer is breast cancer, ovarian cancer, prostate cancer, lung cancer, pancreatic cancer, kidney cancer, colorectal cancer, small intestinal cancer, esophagus cancer or gastric cancer;
[16] a method for the prophylaxis or treatment of cancer, which comprises administering (1) an effective amount of an HER2 inhibitor having a pyrrolopyrimidine skeleton or pyrazolopyrimidine skeleton and (2) an effective amount of a hormonal therapeutic agent or anti-cancer agent to a mammal;
[17] use of (1) an HER2 inhibitor having a pyrrolopyrimidine skeleton or pyrazolopyrimidine skeleton and (2) a hormonal therapeutic agent or anti-cancer agent for the production of an agent for the prophylaxis or treatment of cancer, and the like.

In addition, the present invention relates to
[18] the pharmaceutical agent of the above-mentioned [1], wherein the HER2 inhibitor having a pyrrolopyrimidine skeleton or pyrazolopyrimidine skeleton is a compound represented by the formula: 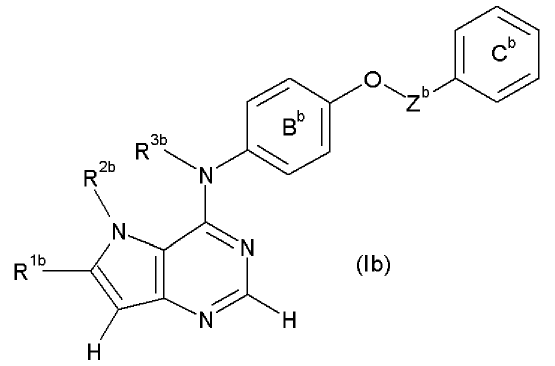 wherein R^{1b} is a hydrogen atom or an optionally substituted group bonded via a carbon atom, a nitrogen atom or an oxygen atom,
   R^{2b} is an optionally substituted group bonded via a carbon atom or a sulfur atom, or
   R^{1b} and R^{2b}, or R^{2b} and R^{3b} are optionally bonded to form an optionally substituted ring structure,
   R^{3b} is a hydrogen atom or an optionally substituted aliphatic hydrocarbon group, or
   R^{3b} is optionally bonded to a carbon atom of the adjacent phenyl group to form an optionally substituted ring structure, B^{b} is an optionally substituted benzene ring,
   C^{b} is an optionally substituted C₆₋₁₈ aryl group, and
   Z^{b} is an optionally substituted C₁₋₃ alkylene group (hereinafter sometimes to be referred to as compound (Ib) in the present specification) or a salt thereof or a prodrug thereof;
[19] the pharmaceutical agent of the above-mentioned [1], wherein the HER2 inhibitor having a pyrrolopyrimidine skeleton or pyrazolopyrimidine skeleton is a compound represented by the formula: 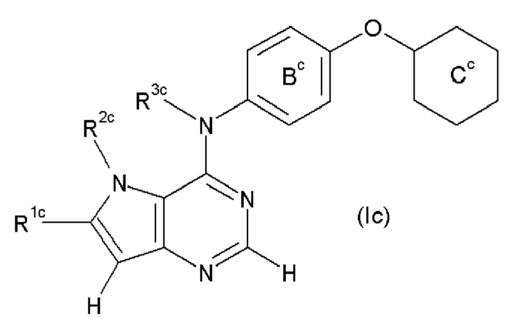 wherein R^{1c} is a hydrogen atom or an optionally substituted group bonded via a carbon atom, a nitrogen atom or an oxygen atom,
   R^{2c} is an optionally substituted group bonded via a carbon atom or a sulfur atom, or
   R^{1c} and R^{2c}, or R^{2c} and R^{3c} are optionally bonded to form an optionally substituted ring structure,
   R^{3c} is a hydrogen atom or an optionally substituted aliphatic hydrocarbon group, or
   R^{3c} is optionally bonded to a carbon atom of the adjacent phenyl group to form an optionally substituted ring structure, B^{c} is an optionally substituted benzene ring, and
   C^{c} is an optionally substituted heterocyclic group (hereinafter sometimes to be referred to as compound (Ic) in the present specification) or a salt thereof or a prodrug thereof;
[20] the pharmaceutical agent of the above-mentioned [1], wherein the HER2 inhibitor having a pyrrolopyrimidine skeleton or pyrazolopyrimidine skeleton is a compound represented by the formula: 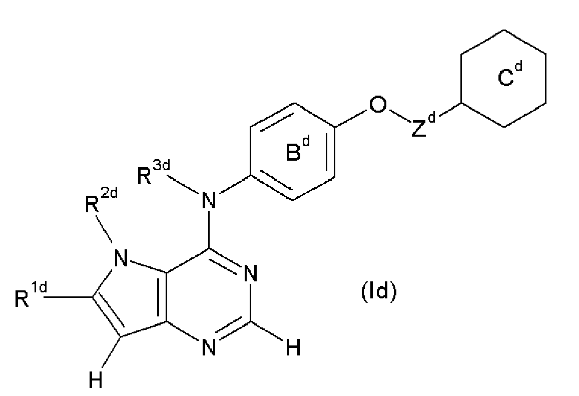 wherein R^{1d} is a hydrogen atom or an optionally substituted group bonded via a carbon atom, a nitrogen atom or an oxygen atom,
   R^{2d} is an optionally substituted group bonded via a carbon atom or a sulfur atom, or
   R^{1d} and R^{2d}, or R^{2d} and R^{3d} are optionally bonded to form an optionally substituted ring structure,
   R^{3d} is a hydrogen atom or an optionally substituted aliphatic hydrocarbon group, or
   R^{3d} is optionally bonded to a carbon atom of the adjacent phenyl group to form an optionally substituted ring structure, B^{d} is an optionally substituted benzene ring,
   C^{d} is an optionally substituted heterocyclic group, and
   2^{d} is an optionally substituted C₁₋₃ alkylene group, (hereinafter sometimes to be referred to as compound (Id) in the present specification) or a salt thereof or a prodrug thereof;
[21] the pharmaceutical agent of the above-mentioned [1], wherein the HER2 inhibitor having a pyrrolopyrimidine skeleton or pyrazolopyrimidine skeleton is a compound represented by the formula: 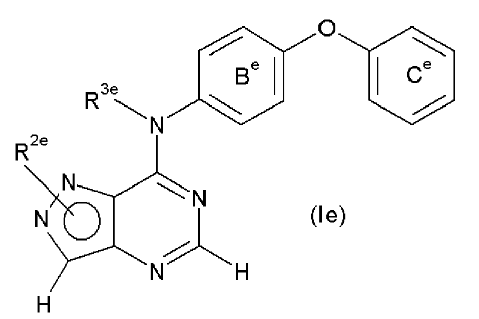 wherein R^{2e} is an optionally substituted group bonded via a carbon atom or a sulfur atom, or
   R^{2e} and R^{3e} are optionally bonded to form an optionally substituted ring structure,
   R^{3e} is a hydrogen atom or an optionally substituted aliphatic hydrocarbon group, or
   R^{3e} is optionally bonded to a carbon atom of the adjacent phenyl group to form an optionally substituted ring structure, Be is an optionally substituted benzene ring, and
   C^{e} is an optionally substituted C₆₋₁₈ aryl group, (hereinafter sometimes to be referred to as compound (Ie) in the present specification) or a salt thereof or a prodrug thereof;
[22] the pharmaceutical agent of the above-mentioned [1], wherein the HER2 inhibitor having a pyrrolopyrimidine skeleton or pyrazolopyrimidine skeleton is a compound represented by the formula: 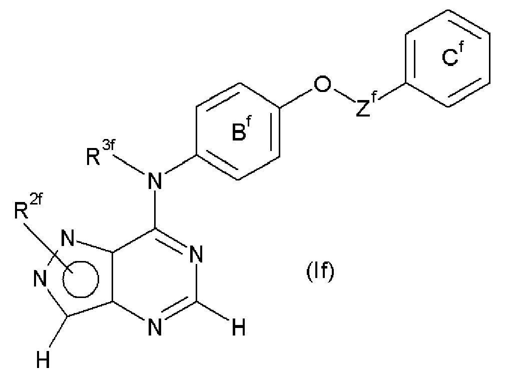 wherein R^{2f} is an optionally substituted group bonded via a carbon atom or a sulfur atom, or
   R^{2f} and R^{3f} are optionally bonded to form an optionally substituted ring structure,
   R^{3f} is a hydrogen atom or an optionally substituted aliphatic hydrocarbon group, or
   R^{3f} is optionally bonded to a carbon atom of the adjacent phenyl group to form an optionally substituted ring structure, B^{f} is an optionally substituted benzene ring,
   C^{f} is an optionally substituted C₆₋₁₈ aryl group, and
   Z^{f} is an optionally substituted C₁₋₃ alkylene group, (hereinafter sometimes to be referred to as compound (If) in the present specification) or a salt thereof or a prodrug thereof;
[23] the pharmaceutical agent of the above-mentioned [1], wherein the HER2 inhibitor having a pyrrolopyrimidine skeleton or pyrazolopyrimidine skeleton is a compound represented by the formula: 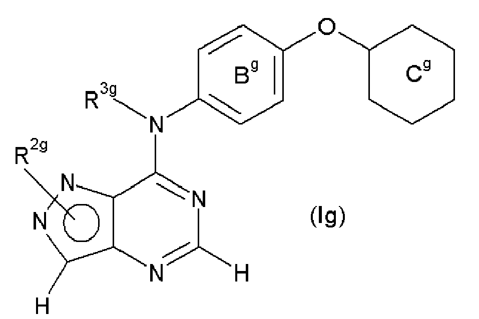 wherein R^{2g} is an optionally substituted group bonded via a carbon atom or a sulfur atom, or
   R^{2g} and R^{3g} are optionally bonded to form an optionally substituted ring structure,
   R^{3g} is a hydrogen atom or an optionally substituted aliphatic hydrocarbon group, or
   R^{3g} is optionally bonded to a carbon atom of the adjacent phenyl group to form an optionally substituted ring structure, B^{g} is an optionally substituted benzene ring, and
   C^{g} is an optionally substituted heterocyclic group, (hereinafter sometimes to be referred to as compound (Ig) in the present specification) or a salt thereof or a prodrug thereof, and the like.

The present invention is explained in detail in the following.

### Effect of the Invention

A pharmaceutical agent comprising (1) an HER2 inhibitor having a pyrrolopyrimidine skeleton or pyrazolopyrimidine skeleton and (2) a hormonal therapeutic agent or anti-cancer agent in combination is useful as a safe agent for the prophylaxis or treatment of cancer since it shows a significant anti-cancer action as compared to use as a single agent and use of other combination pharmaceutical agents.

A hormonal therapeutic agent increases the HER family molecule and enhances stimulation of the HER family molecule, thereby possibly limiting the effect. In contrast, an HER2 inhibitor having a pyrrolopyrimidine skeleton or pyrazolopyrimidine skeleton blocks HER signals. Accordingly, a pharmaceutical agent particularly comprising (1) an HER2 inhibitor having a pyrrolopyrimidine skeleton or pyrazolopyrimidine skeleton and (2) a hormonal therapeutic agent in combination prevents an increase in the HER family molecule as compared to use of a hormonal therapeutic agent as a single agent, and permits use of a pharmaceutical agent with a different mechanism in combination.

### Brief Description of the Drawings

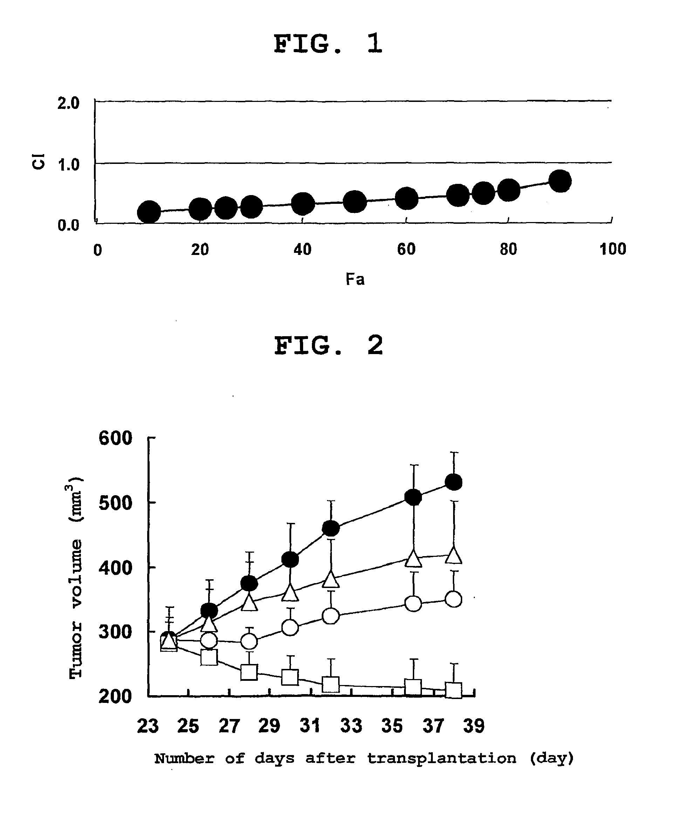

Fig. 1 shows the effect of a combined use of compound A and trastuzumab for the growth of HER2 high expressing human breast cancer cell line, wherein the vertical axis shows the Combination Index and the transverse axis shows Fraction affected; Fa. The effect of the combined use when Combination Index (CI) is 1 is an additive effect, when it is less than 1 is a synergistic effect, and when it is not less than 1 is a counteracting effect.
Fig. 2 shows the effect of a combined use of compound A and trastuzumab for the tumor growth of HER2 high expressing human breast cancer cell line, wherein the vertical axis shows the tumor volume and the transverse axis shows the number of days after transplantation. The effect of the combined use was analyzed by two-way analysis of variance. (●; control group, o; compound A, Δ; trastuzumab, □; compound A+trastuzumab)

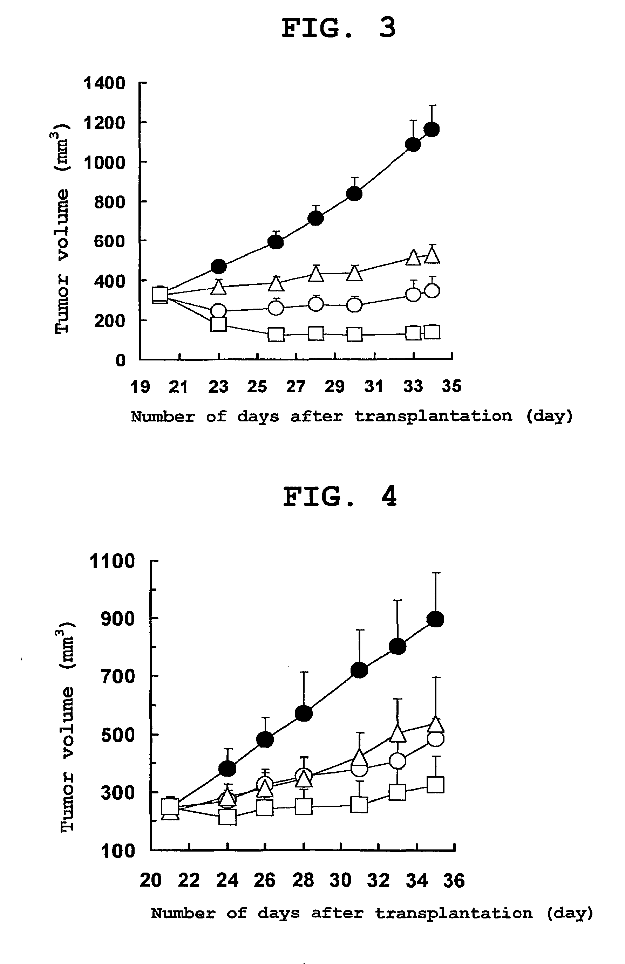

Fig. 3 shows the effect of a combined use of compound A and trastuzumab for the tumor growth of HER2 high expressing 4-1ST gastric cancer tumor, wherein the vertical axis shows the tumor volume and the transverse axis shows the number of days after transplantation. The effect of the combined use was analyzed by two-way analysis of variance. (●; control group, ; compound A, Δ; trastuzumab, □; compound A+trastuzumab)
Fig. 4 shows the effect of a combined use of compound A and cetuximab for the tumor growth of HER2 high expressing 4-1ST gastric cancer tumor, wherein the vertical axis shows the tumor volume and the transverse axis shows the number of days after transplantation. The effect of the combined use was analyzed by two-way analysis of variance. (●; control group, o; compound A, Δ; cetuximab, □; compound A+cetuximab)

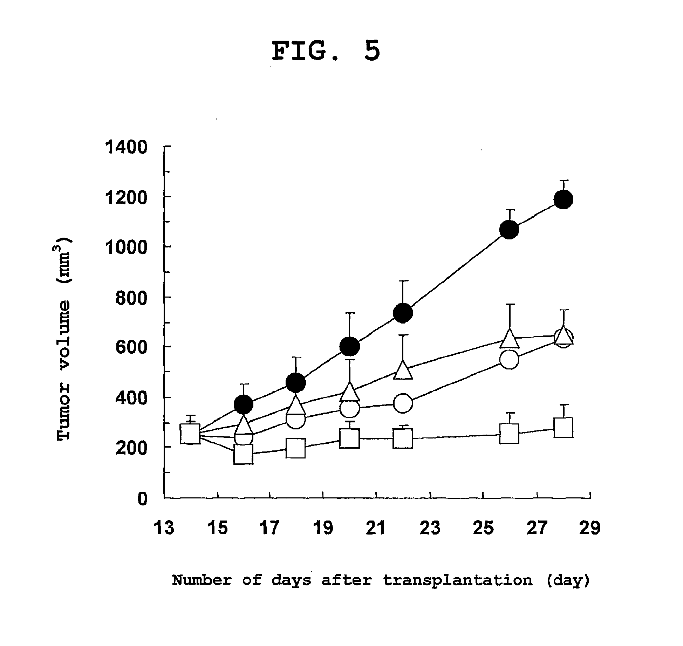

Fig. 5 shows the effect of a combined use of compound A and erlotinib for the tumor growth of HER2 high expressing 4-1ST gastric cancer tumor, wherein the vertical axis shows the tumor volume and the transverse axis shows the number of days after transplantation. The effect of the combined use was analyzed by two-way analysis of variance. (●; control group, o; compound A, Δ; erlotinib, □; compound A+erlotinib)

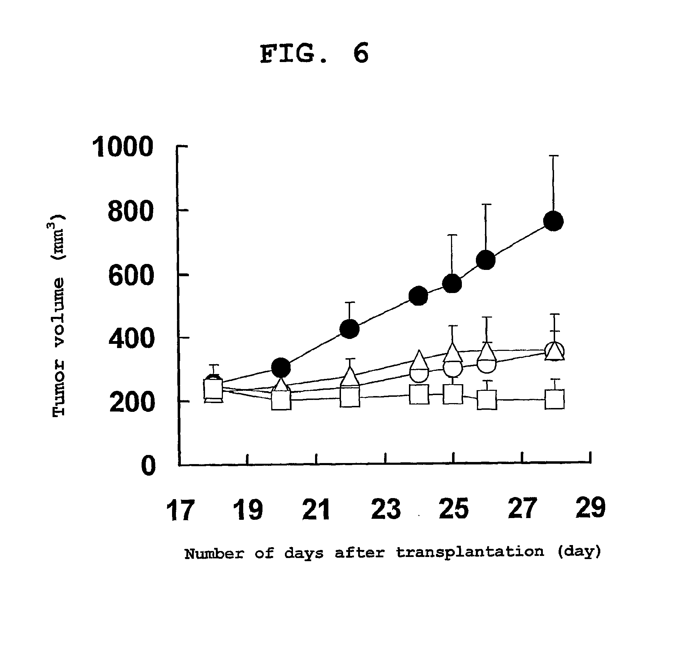

Fig. 6 shows the effect of a combined use of compound A and paclitaxel for the tumor growth of HER2 high expressing human breast cancer cell line, wherein the vertical axis shows the tumor volume and the transverse axis shows the number of days after transplantation. The effect of the combined use was analyzed by two-way analysis of variance. (●; control group, ο; compound A, Δ; paclitaxel, □; compound A+paclitaxel)

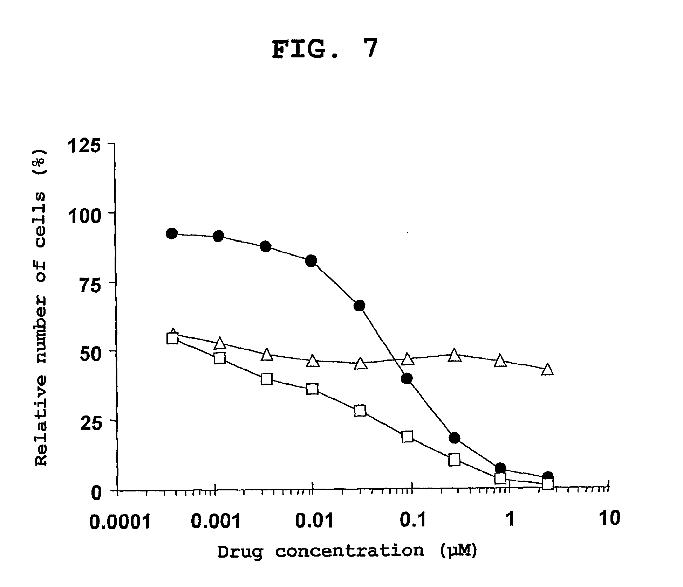

Fig. 7 shows inhibition of BT-474 cell growth by a combined use of compound A and fulvestrant as compared to each use as a single agent. Using SRB staining method, a cell growth inhibitory assay was performed. The transverse axis shows the concentration of each pharmaceutical agent and the vertical axis shows the number of cells (%) when a group free of addition of a pharmaceutical agent as 100%. (●; compound A alone, Δ; fulvestrant alone, □; compound A+fulvestrant)

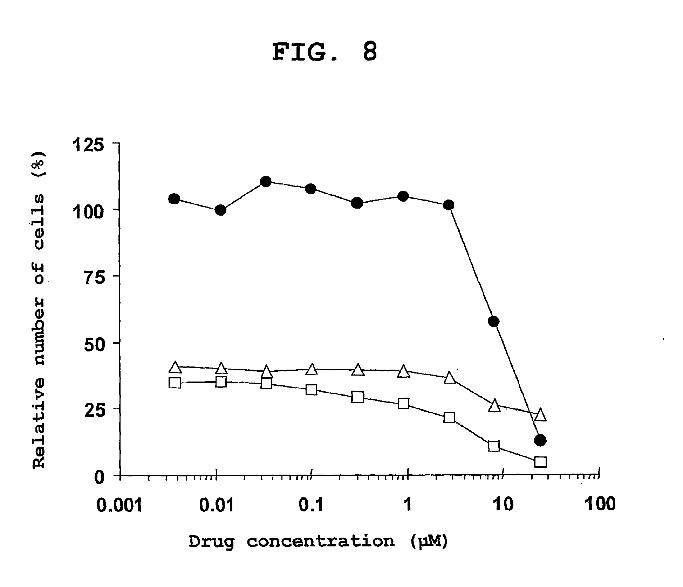

Fig. 8 shows inhibition of MCF-7 cell growth by a combined use of compound A and fulvestrant as compared to each use as a single agent. Using SRB staining method, a cell growth inhibitory assay was performed. The transverse axis shows the concentration of each pharmaceutical agent and the vertical axis shows the number of cells (%) when a group free of addition of a pharmaceutical agent as 100%. (●; compound A alone, Δ; fulvestrant alone, □; compound A+fulvestrant)

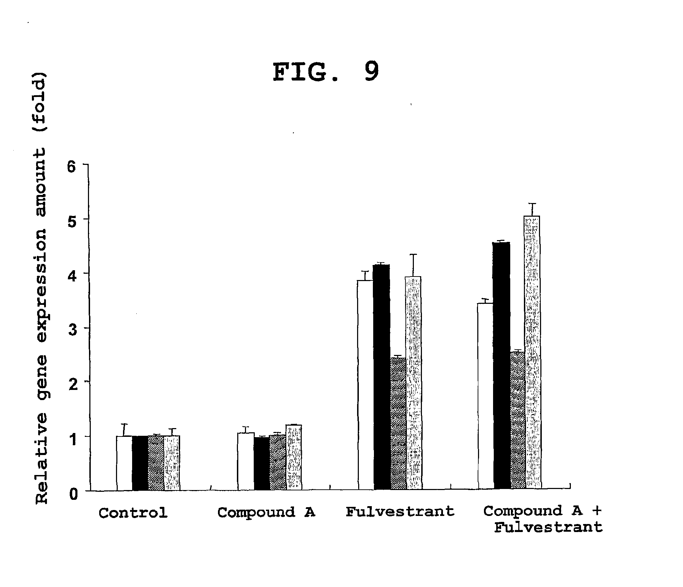

Fig. 9 shows an increase in the HER family gene expression by a treatment with fulvestrant, where in the vertical axis shows the expression amount of each gene and the expression amount of the control group of each gene is 1. In the Figure, 4 bars in each graph for the groups of Control, compound A, fulvestrant and compound A+fulvestrant show the expression amounts of EGFR, HER2, HER3 and HER4 from the left. (open: EGFR, closed: HER2, hatched: HER3, dotted: HER4)

In the present specification, unless otherwise specified, the "aryl" in the "aryl group" and the substituents includes a monocyclic aryl group and a fused polycyclic aryl group. As the "aryl group", for example, a C₆₋₁₈ aryl group can be mentioned. As the "C₆₋₁₈ aryl group", for example, phenyl, biphenylyl, naphthyl, anthryl, phenanthryl and acenaphthylenyl can be mentioned.

In the present specification, unless otherwise specified, the "heterocyclyl-" in the "heterocyclic group" and substituent encompasses a heteroaryl group and a saturated or unsaturated aliphatic heterocyclic group. In the present specification, as the "heterocyclic group" (and "heterocyclyl-" in the substituents), for example, a 3- to 12-membered (preferably 5-to 8-membered) heterocyclic group (for example, heteroaryl group or a saturated or unsaturated aliphatic heterocyclic group) containing, as an atom (ring atom) constituting a ring system, one or more (preferably 1 to 4, more preferably 1 to 3, further preferably 1 or 2) hetero atoms selected from an oxygen atom, an optionally oxidized sulfur atom and a nitrogen atom and the like (preferably, an oxygen atom, a sulfur atom and a nitrogen atom etc.) can be mentioned.

In the present specification, unless otherwise specified, as the "aliphatic hydrocarbon group", a linear or branched aliphatic hydrocarbon group having 1 to 15 carbon atom (preferably, 1 to 8 carbon atom) can be mentioned. As such "aliphatic hydrocarbon group", for example, a C₁₋₈ alkyl group, a C₂₋₈ alkenyl group, a C₂₋₈ alkynyl group, a C₃₋₈ cycloalkyl group and the like can be mentioned.

In the present specification, unless otherwise specified, as the "heteroaryl group", an aromatic monocyclic heterocyclic group (e.g., 5- or 6-membered aromatic monocyclic heterocyclic group such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl and the like) and an aromatic fused heterocyclic group (e.g., 8- to 12-membered aromatic fused heterocyclic group such as benzofuranyl, isobenzofuranyl, benzothienyl, indolyl, isoindolyl, 1H-indazolyl, benzindazolyl, benzoxazolyl, 1,2-benzisoxazolyl, benzothiazolyl, benzopyranyl, 1,2-benzisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acrydinyl, phenoxazinyl, phenothiazinyl, phenazinyl, phenoxathiinyl, thianthrenyl, phenathridinyl, phenathrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-blpyridazinyl, imidazo[1,2-a]pyrimidinyl, 1,2,4-triazolo[4,3-a]pyridyl, 1,2,4-triazolo[4,3-b]pyridazinyl and the like) and the like can be mentioned. As the aromatic fused heterocyclic group, a heterocycle wherein the aforementioned 5- or 6-membered aromatic monocyclic heterocyclic group is fused with a benzene ring and a heterocycle wherein the same or different two heterocycles of the aforementioned 5- or 6-membered aromatic monocyclic heterocyclic group are fused are preferable.

In the present specification, unless otherwise specified, as the "aliphatic heterocyclic group", for example, a 3- to 8-membered (preferably 5- or 6-membered) saturated or unsaturated (preferably saturated) aliphatic heterocyclic group such as oxiranyl, azetidinyl, oxetanyl, thietanyl, pyrrolidinyl, tetrahydrofuryl, thiolanyl, piperidyl, tetrahydropyranyl, morpholinyl, thiomorpholinyl, piperazinyl, dihydro-1,2,4-oxadiazolyl and the like, and the like can be mentioned.

In the present specification, unless otherwise specified, as the "C₁₋₈ alkyl group", for example, methyl, ethyl, n-propyl, i-propyl, n-butyl, i-butyl, s-butyl, t-butyl, n-pentyl, i-pentyl, t-pentyl, neopentyl, n-hexyl, i-hexyl, n-heptyl and n-octyl and the like can be mentioned, with preference given to a C₁₋₆ alkyl group. In the present specification, moreover, unless otherwise specified, as the "C₁₋₄ alkyl group", for example, methyl, ethyl, n-propyl, i-propyl, n-butyl and i-butyl can be mentioned.

In the present specification, unless otherwise specified, as the "C₂₋₈ alkenyl group", for example, vinyl, (1- or 2-)propenyl, (1-, 2- or 3-)butenyl, pentenyl, octenyl and (1, 3-)butadienyl can be mentioned, with preference given to a C₂₋₄ alkenyl group.

In the present specification, unless otherwise specified, as the "C₂₋₈ alkynyl group", for example, ethynyl, (1- or 2-)propynyl, (1-, 2- or 3-)butynyl, pentynyl and octynyl can be mentioned, with preference given to a C₂₋₄ alkynyl group.

In the present specification, unless otherwise specified, as the "C₃₋₈ cycloalkyl group", for example, cyclopropyl, cyclobutyl, cyclopentyl, cyclohexyl, cycloheptyl and cyclooctyl can be mentioned, with preference given to a C₃₋₆ cycloalkyl group.

In the present specification, unless otherwise specified, as the "C₁₋₄ alkylene", for example, methylene, ethylene, trimethylene, tetramethylene and propylene and the like can be mentioned.

In the present specification, unless otherwise specified, as the "-O-(C₁₋₄ alkylene)-", for example, -OCH₂-, -OCH₂CH₂-, -O(CH₂)₃-, -O(CH₂)₄-, -OCH(CH₃)-,-OC(CH₃)₂-, -OCH(CH₃)CH₂-, -OCH₂CH(CH₃)-, -OC(CH₃)2CH₂-and -OCH₂C(CH₃)₂-and the like can be mentioned.

In the present specification, unless otherwise specified, as the "C₆₋₁₈ aryl-carbonyl group", for example, benzoyl, naphthoyl, anthrylcarbonyl, phenanthrylcarbonyl and acenaphthylenylcarbonyl and the like can be mentioned.

In the present specification, unless otherwise specified, as the "C₆₋₁₈ aryl-C₁₋₄ alkyl-carbonyl group", for example, benzylcarbonyl, 3-phenylpropionyl, 2-phenylpropionyl, 4-phenylbutyryl and 5-phenylpentanoyl and the like can be mentioned.

In the present specification, unless otherwise specified, as the "halogen", fluorine, chlorine, bromine and iodine can be mentioned.

As the "5- to 8-membered heterocyclyl-carbonyl group containing 1 to 3 hetero atoms selected from a nitrogen atom, an oxygen atom and a sulfur atom", "a 5- to 8-membered cyclic amino-carbonyl group optionally having 1 or 2 hetero atoms selected from a nitrogen atom, an oxygen atom and a sulfur atom" is preferable, for example, pyrrolidin-1-ylcarbonyl, piperidin-1-ylcarbonyl, piperazin-1-ylcarbonyl, morpholin-4-ylcarbonyl, thiomorpholin-4-ylcarbonyl and the like can be mentioned.

In the above-mentioned formula, as the "aryl group" for A, a C₆₋₁₈ aryl group is preferable, and phenyl is more preferable.

The "aryl group" and "heteroaryl group" for A is optionally substituted by a group represented by the formula - Y²-B wherein Y² is a single bond, -O-, -O-(C₁₋₃ alkylene)-(preferably -OCH₂-), -NH- or -S-, and B is an aryl group, a heterocyclic group, a C₃₋₈ cycloalkyl group, a carbamoyl group, a ureido group, a C₆₋₁₈ aryl-carbonyl group or a C₆₋₁₈ aryl-C₁₋₄ alkyl-carbonyl group, each of which is optionally substituted.

As Y², a single bond, -O- or -OCH₂- is preferable, and -O- or -OCH₂- is more preferable. particularly -O- is preferable.

As the "aryl group" for B, a C₆₋₁₈ aryl group is preferable, and phenyl is more preferable.

As the "heterocyclic group" for B, the aforementioned "5-or 6-membered aromatic monocyclic heterocyclic group" is preferable, and pyridyl is more preferable.

The "aryl group", "heterocyclic group", "C₆₋₁₈ aryl-carbonyl group" or "C₆₋₁₈ aryl-C₁₋₄ alkyl-carbonyl group" for B may have, for example, 1 to 5, the same or different substituents selected from halogen, optionally halogenated C₁₋₄ alkyl, hydroxy, optionally halogenated C₁₋₄ alkoxy, C₁₋₄ alkoxymethyl, hydroxyl-C₁₋₄ alkyl, C₁₋₄ alkyl-carbonyl, carboxy, C₁₋₄ alkoxy-carbonyl, cyano, carbamoyl, sulfamoyl, nitro, amino, C₁₋₄ alkyl-carbonylamino, C₁₋₄ alkoxy-carbonylamino and C₁₋₄ alkyl-sulfonylamino, at any substitutable position(s). As the substituent of the "aryl group" for B, halogen, optionally halogenated C₁₋₄ alkyl, optionally halogenated C₁₋₄ alkoxy and the like are preferable and, for example, fluorine, chlorine, trifluoromethyl, trifluoromethoxy and the like can be mentioned. Of these, optionally halogenated C₁₋₄ alkyl (e.g., trifluoromethyl) and the like are preferable.

The "aryl group" and "heteroaryl group" for A may further have, besides the above-mentioned a group represented by the formula -Y²-B, 1 to 5, the same or different substituents at substitutable optional position(s). As such substituent, substituents similar to those exemplified for the "aryl group" or "heterocyclic group" for B can be mentioned. As such substituent, halogen, optionally halogenated C₁₋₄ alkyl and the like are preferable, such as chlorine, methyl and the like can be mentioned. Of these, halogen (e.g., chlorine) is preferable.

As A, for example, 3-chloro-4-[3-(trifluoromethyl)phenoxy]phenyl, 3-chloro-4-[(3-fluorobenzyl)oxy]phenyl, 3-methyl-4-[3-(trifluoromethoxy)phenoxy]phenyl, 3-chloro-4-(3-chlorophenoxy)phenyl, 3-chloro-4-[3-(trifluoromethoxy)phenoxy]phenyl and the like can be mentioned. Of these, 3-chloro-4-[3-(trifluoromethyl)phenoxy]phenyl and the like are preferable.

As the "aliphatic hydrocarbon group" for R³, a C₁₋₈ alkyl group, a C₂₋₈ alkenyl group, a C₂₋₈ alkynyl group and a C₃₋₈ cycloalkyl group are preferable.

The "aliphatic hydrocarbon group" for R³ is optionally substituted by 1 to 3 substituents selected from halogen, hydroxy, C₁₋₄ alkoxy, C₁₋₄ alkyl-carbonyl, carboxy, C₁₋₄ alkoxy-carbonyl, cyano, carbamoyl, sulfamoyl, nitro, amino, C₁₋₄ alkyl-carbonylamino, C₁₋₄ alkoxy-carbonylamino and C₁₋₄ alkyl-sulfonylamino.

The "C₁₋₄ alkylene" and "-O-(C₁₋₄alkylene)-" for Y¹ are optionally substituted by 1 to 3 substituents selected from halogen, hydroxy, C₁₋₄ alkoxy, C₁₋₄ alkyl-carbonyl, carboxy, C₁₋₄ alkoxy-carbonyl, cyano, carbamoyl, sulfamoyl, nitro, amino, C₁₋₄ alkyl-carbonylamino, C₁₋₄ alkoxy-carbonylamino and C₁₋₄ alkyl-sulfonylamino.

As X¹, -NR³- wherein R³ is as defined above is preferable. As R³, a hydrogen atom is preferable.

As W, C (R¹) is preferable.

As R¹, a hydrogen atom is preferable.

As the "optionally substituted group bonded via a carbon atom, a nitrogen atom or an oxygen atom" for R¹, a group of the formula -X²-R⁴ can be mentioned, wherein X² is a single bond, -NH- or -O-, and R⁴ is a hydrogen atom, a cyano group, or a C₁₋₈ alkyl group, a C₂₋₈ alkenyl group, a C₂₋₈ alkynyl group, a carbamoyl group, a C₁₋₈ alkyl-carbonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted.

The "C₁₋₈ alkyl group", "C₂₋₈ alkenyl group", "C₂₋₈ alkynyl group", "C₁₋₈ alkyl-carbonyl group", "C₃₋₈ cycloalkyl group", "C₆₋₁₈ aryl group", "C₆₋₁₈ aryl-C₁₋₄ alkyl group", "C₆₋₁₈ aryl-carbonyl group", "C₆₋₁₈ aryl-C₁₋₄ alkyl-carbonyl group", "heterocyclic group", "heterocyclyl-C₁₋₄ alkyl group", "heterocyclyl-carbonyl group" and "heterocyclyl-C₁₋₄ alkyl-carbonyl group" are, for example, optionally substituted by one or more (preferably 1 to 5, more preferably 1 to 3) substituent(s) selected from the group consisting of
(a) halogen,
(b) oxo,
(c) optionally halogenated C₁₋₄ alkyl,
(d) - (CH₂)ₘ-Q,
(e) - (CH₂)ₘ-Z¹- (optionally halogenated C₁₋₄ alkyl) ,
(f) -(CH₂)ₘ-Z¹-C₃₋₈ cycloalkyl,
(g) - (CH₂) ₘ-Z²- (CH₂) ₙ⁻Q,
(h) -(CH₂)m-Z²-(CH2)ₙ-Z¹-(optionally halogenated C₁₋₄ alkyl) ,
(i) - (CH₂) ₘ-Z²- (CH₂) ₙ-Z¹-C₃₋₈ cycloalkyl,
(j) -(CH₂)ₘ-Z¹-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom)
(k) -(CH₂)ₘ-Z²-C₁₋₄ alkoxy, and
(l) - (CH₂)ₘ-Z²-(CH₂)ₘ-Z²-(CH₂)ₙ-Z¹-C₁₋₄alkyl (hereinafter to be sometimes to be referred to as substituent group T).

In these formulas, m is an integer of 0 to 4, n is an integer of 1 to 4,
Q is hydroxy, carboxy, cyano, nitro, -NR⁶R⁷, -CONR⁶R⁷, -OCONH₂ or -SO₂NR⁶R⁷,
Z¹ is -O-, -CO-, -C(OH)R⁸-, -C(=N-OR⁸)-, -S-, -SO-, -SO₂-, -N(COR⁸) -, -N(CO₂R⁹) -, -N(SO₂R⁹) -, -CO-O-, -O-CO-, -CO-NR⁸-, -NR⁸-CO-, -NR⁸-CO²-, -NR⁸-CO-NH-, -NR⁸-SO²-, or -NR⁸-C(=NH)-NH-,
and
z² is -O-, -CO-, -C(OH)R⁸-, -C(=N-OR⁸) -, -S-, -SO-, -SO₂-, -NR⁸-, -N(COR⁸)-, -N(CO₂R⁹)-, -N(SO₂R⁹)-, -CO-O-, -O-CO-, -CO-NR⁸-, -NR⁸-CO-, -NR⁸-CO₂-, -NR⁸-CO-NH-, -NR⁸-C(=NH) -NH-, -NR⁸-SO₂-, or -S02-NR⁸-. In these formulas, (CH₂)ₘ and (CH₂)ₙ are optionally substituted by one or more (preferably 1 to 5, more preferably 1 to 3) substituents selected from, for example, halogen, optionally halogenated C₁₋₄ alkyl and hydroxy, and when m or n is not less than 2, a subset -CH₂CH₂- of (CH₂)ₘ and (CH₂)ₙ is optionally replaced by -CH=CH- or -C≡C-.

In these formulas, R⁶ and R⁷are the same or different and each is a hydrogen atom or C₁₋₄ alkyl, or R⁶ and R⁷ form a ring together with a nitrogen atom. In these formulas, moreover, R⁸ is a hydrogen atom or C₁₋₄ alkyl and R⁹ is C₁₋₄ alkyl. When R⁶ and R⁷ form a ring together with a nitrogen atom, as the nitrogen-containing heterocyclic group, for example, a 3- to 8-membered (preferably 5- or 6-membered) saturated or unsaturated (preferably saturated) aliphatic heterocyclic group such as azetidinyl, pyrrolidinyl, piperidinyl, homopiperidinyl, heptamethyleneimino, morpholinyl, thiomorpholinyl, piperazinyl, homopiperazinyl and the like, and the like can be mentioned.

As X², a single bond is preferable.

As R⁴, a hydrogen atom or a C₁₋₈ alkyl group, a C₂₋₈ alkenyl group, a C₆₋₁₈ aryl group or heterocyclic group, each of which is optionally substituted is preferable. As the "C₆₋₁₈ aryl group" for R⁴, phenyl is preferable. As the "heterocyclic group" for R⁴, the aforementioned "5- or 6-membered aromatic monocyclic heterocyclic group" is preferable, and furyl is more preferable.

As the "optionally substituted group bonded via a carbon atom or a sulfur atom" for R², a C₁₋₈ alkyl group, a C₂₋₈ alkenyl group, a C₂₋₈ alkynyl group, a carbamoyl group, a C₁₋₈ alkyl-carbonyl group, a C₁₋₈ alkyl-sulfonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a C₆₋18 aryl-sulfonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted, can be mentioned.

The "C₁₋₈ alkyl group", "C₂₋₈ alkenyl group", "C₂₋₈ alkynyl group", "C₁₋₈ alkyl-carbonyl group", "C₁₋₈ alkyl-sulfonyl group", "C₃₋₈ cycloalkyl group", "C₆₋₁₈ aryl group", "C₆₋₁₈ aryl-C₁₋₄ alkyl group", "C₆₋₁₈ aryl-carbonyl group", "C₆₋₁₈ aryl-C₁₋₄ alkyl-carbonyl group", "C₆₋₁₈ aryl-sulfonyl group", "heterocyclic group", "heterocyclyl-C₁₋₄ alkyl group", "heterocyclyl-carbonyl group" and "heterocyclyl-C₁₋₄ alkyl-carbonyl group" are optionally substituted by, for example, one or more (preferably 1 to 5, more preferably 1 to 3) substituents selected from the above-mentioned substituent group T.

As R², a hydrogen atom or a C₁₋₈ alkyl group, a C₆₋₁₈ aryl group, a C₆₋₁₈ aryl-C₁₋₄ alkyl group, a C₆₋₁₈ aryl-carbonyl group, a C₆₋₁₈ aryl-sulfonyl group or heterocyclyl-C₁₋₄ alkyl group, each of which is optionally substituted, is preferable. Particularly, optionally substituted C₁₋₈ alkyl group and the like are preferable and, for example, ethyl substituted at the 2-position and the like can be mentioned. As the substituent of the optionally substituted C₁₋₈ alkyl group, (g) -(CH₂)ₘ-Z²-(CH₂)ₙ-Q in the above-mentioned substituent group T and the like are preferable, particularly that wherein m is 0, Z² is - NR⁸-CO- or -O- and Q is hydroxy, namely, -0-(CH₂)ₙ-OH or -NR⁸-CO-(CH₂)ₙ-OH ((CH₂)ₙ is optionally substituted by C₁₋₄ alkyl (e.g., methyl)) and the like are preferable. Particularly, - NR⁸-CO-(CH₂)ₙ-OH((CH₂)ₙ is optionally substituted by C₁₋₄ alkyl (e.g., methyl)) is preferable. In these formulas, R⁸ is preferably a hydrogen atom and n is preferably 2. As the (CH₂) moiety, -CH₂-C(CH₃)₂-, -CH₂-CH₂- and the like can be mentioned and -CH₂-C(CH₃)₂- and the like are preferable.

As the substituent of the optionally substituted C₁₋₈ alkyl group for R², (h) -(CH₂)ₘ-Z²-(CH₂)ₙ-Z¹-(optionally halogenated C₁₋₄ alkyl) of the above-mentioned substituent group T and the like are preferable, particularly that wherein m is 0, Z₂ is -NR⁸-CO- or -O-, and Z₁ is -SO₂-, namely, -O- (CH₂) ₙ-SO₂-(optionally halogenated C₁₋₄ alkyl) or -NR⁸-CO-(CH₂)ₙ-SO₂-(optionally halogenated C₁₋₄ alkyl) ((CH₂)ₙ is optionally substituted by C₁₋₄ alkyl (e.g., methyl)) and the like are preferable. In these formulas, R⁸ is preferably a hydrogen atom, and n is preferably 1 or 2. As the (CH₂)ₙ moiety, -CH₂-, -CH₂-CH₂-, -C(CH₃₎₂- and the like can be mentioned. As the optionally halogenated C₁₋₄ alkyl moiety, for example, methyl, tert-butyl and the like can be mentioned.

As the substituent of the optionally substituted C₁₋₈ alkyl group for R², for example, -NH-CO-CH₂-C(CH₃)₂-OH, -O-CH₂-CH₂-OH, -NH-CO-CH₂-SO₂-CH₃, -NH-CO-C(CH₃)₂-SO₂-CH₃, -O-CH₂-CH₂-SO₂-C(CH₃)₃ and the like can be mentioned, and -NH-CO-CH₂-C(CH₃)₂-OH and the like are preferable.

As the "C₆₋₁₈ aryl group" for R², phenyl is preferable. As the "C₆₋₁₈ aryl-C₁₋₄ alkyl group" for R², benzyl is preferable. As the "C₆₋₁₈ aryl-carbonyl group" for R², benzoyl is preferable. As the "C₆₋₁₈ aryl-sulfonyl group" for R² phenylsulfonyl is preferable. As the "heterocyclic group" or "heterocyclyl-" of "heterocyclyl-C₁₋₄ alkyl group", "heterocyclyl-carbonyl group" and "heterocyclyl-C₁₋₄ alkyl-carbonyl group" for R², the aforementioned "5- or 6-membered aromatic monocyclic heterocyclic group" or the aforementioned "aliphatic heterocyclic group" is preferable, and furyl or tetrahydrofuryl is more preferable.

In the substituents that a group represented by R² may have, when R⁶ and R⁷ form a ring together with a nitrogen atom, the "ring" optionally further has 1 to 5 (preferably 1 to 3) the same or different substituents. As such substituents, substituents similar to those exemplified for "aryl group" or "heterocyclic group" for B can be mentioned.

The aforementioned "carbamoyl group" and "ureido group" optionally have 1 or 2 optionally substituted C₁₋₈ alkyl group (s). Alternatively, the "carbamoyl group" and "ureido group" may have two substituents and they may form an optionally substituted ring, together with the adjacent nitrogen atom. As the "ring" of the "optionally substituted ring", rings similar to those formed by R⁶ and R⁷ together with a nitrogen atom as exemplified above can be mentioned. As the "substituent" of the "optionally substituted C₁₋₈ alkyl group" and as the "substituent" of the "optionally substituted ring", groups similar to the substituents of the above-mentioned substituent group T can be mentioned.

As the "optionally substituted carbamoyl group", carbamoyl, C₁₋₈ alkylcarbamoyl, di (C₁₋₈ alkyl) carbamoyl, C₆₋₁₈ aryl-C₁₋₄ alkylcarbamoyl, azetidin-1-ylcarbonyl, pyrrolidin-1-ylcarbonyl, piperidin-1-ylcarbonyl, piperazin-1-ylcarbonyl, morpholin-4-ylcarbonyl, thiomorpholin-4-ylcarbonyl, (C₁₋₄ alkyl)piperidin-1-ylcarbonyl, (C₆₋₁₈ aryl-C₁₋₄ alkyl)piperidin-1-ylcarbonyl and the like can be mentioned.

As the "optionally substituted ureido group", ureido, 3-(C₁₋₈ alkyl) ureido, 3,3-di(C₁₋₈ alkyl) ureido, 3-(C₆₋₁₈ aryl-C₁₋₄ alkyl)ureido, azetidine-1-ylcarbonylamino, pyrrolidin-1-ylcarbonylamino, piperidin-1-ylcarbonylamino, piperazin-1-ylcarbonylamino, morpholin-4-ylcarbonylamino, thiomorpholin-4-ylcarbonylamino, (C₁₋₄ alkyl)piperidin-1-ylcarbonylamino, (C₆₋₁₈ aryl-C₁₋₄ alkyl) piperidin-1-ylcarbonylamino and the like can be mentioned.

As the "ring structure" of the optionally substituted ring structure formed by R³ bonded to a carbon atom or a hetero atom on the aryl group or the heteroaryl group represented by A, a saturated or unsaturated (preferably saturated) 4- to 8-membered (preferably 5- or 6-membered) nitrogen-containing heterocycle can be mentioned. Specifically, 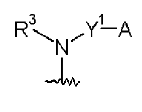 is 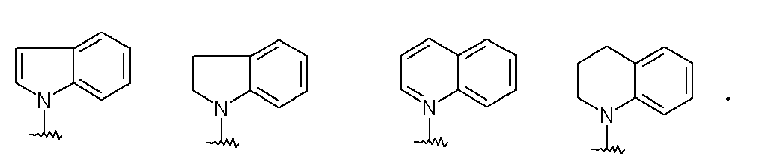 The "ring structure" may have 1 to 5 (preferably 1 to 3, more preferably 1 or 2) the same or different substituents at any substitutable position(s). As such substituents, substituents similar to those exemplified for "aryl group" or "heterocyclic group" for B can be mentioned.

As the "ring structure" of the optionally substituted ring structure formed by R¹ and R² bonded to each other, a saturated or unsaturated (preferably saturated) 4- to 8-membered (preferably 5- or 6-membered) heterocycle can be mentioned. When R¹ and R² are bonded to form an optionally substituted ring structure, for example, 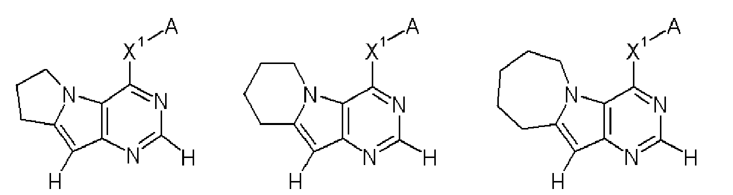 wherein each symbol is as defined above, and the like can be mentioned.

As the "ring structure" of the optionally substituted ring structure formed by R² and R³ bonded to each other, a saturated or unsaturated (preferably saturated) 4- to 8-membered (preferably 5- to 7-membered) heterocycle can be mentioned. When R² and R³ are bonded to form an optionally substituted ring structure, for example, 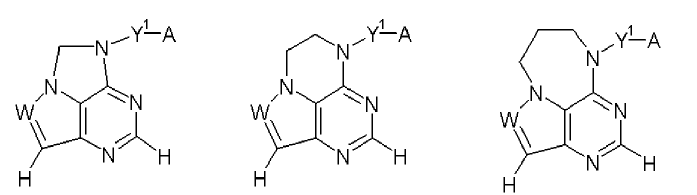 wherein each symbol is as defined above, and the like can be mentioned. The "ring structure" formed by R¹ and R², or R² and R³ bonded to each other may have 1 to 5 (preferably 1 to 3, more preferably 1 or 2) the same or different substituents selected from the above-mentioned substituent group T at any substitutable position(s).

When W is C(R¹), compound (I) is represented by the following formula (IA): 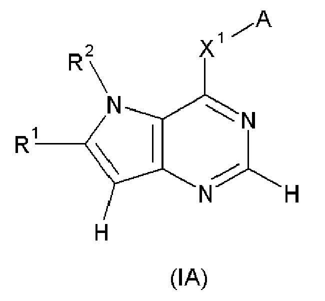 wherein each symbol is as defined above.

When W is N, compound (I) is represented by the following formula (IB) or (IC): 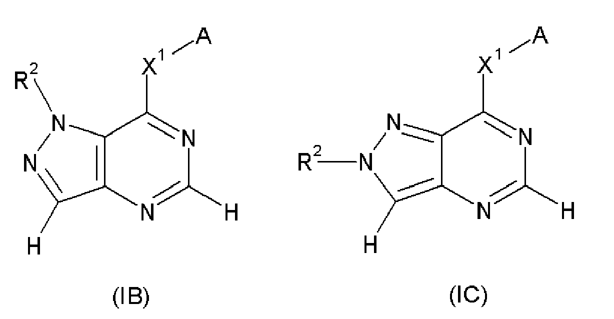 wherein each symbol is as defined above.

Specifically, as compound (I) or a salt thereof or a prodrug thereof, the following compounds (Ia) - (Ik) or a salt thereof or a prodrug thereof and the like are preferably used.

### [Compound (Ia)

A compound represented by the formula: 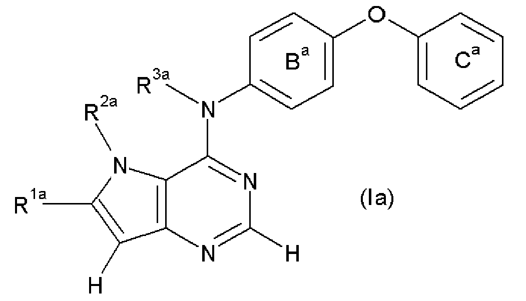 wherein R^{1a} is a hydrogen atom or an optionally substituted group bonded via a carbon atom, a nitrogen atom or an oxygen atom,
R^{2a} is an optionally substituted group bonded via a carbon atom or a sulfur atom, or
R^{1a} and R^{2a}, or R^{2a} and R^{3a} are optionally bonded to form an optionally substituted ring structure,
R^{3a} is a hydrogen atom or an optionally substituted aliphatic hydrocarbon group, or
R^{3a} is optionally bonded to a carbon atom of the adjacent phenyl group to form an optionally substituted ring structure, B^{a} is an optionally substituted benzene ring, and
C^{a} is an optionally substituted C₆₋₁₈ aryl group.

As the "optionally substituted group bonded via a carbon atom, a nitrogen atom or an oxygen atom" for R^{1a}, those similar to the "optionally substituted group bonded via a carbon atom, a nitrogen atom or an oxygen atom" for R¹ can be used.

As R^{1a}, a hydrogen atom is preferable.

As the "optionally substituted group bonded via a carbon atom or a sulfur atom" for R^{2a}, those similar to the "optionally substituted group bonded via a carbon atom or a sulfur atom" for R² can be used.

As the "optionally substituted ring structure" constructed by R^{1a} and R^{2a}, or R^{2a} and R^{3a} bonded to each other, those similar to the "optionally substituted ring structure" constructed by R¹ and R², or R² and R³ bonded to each other can be used.

As the "optionally substituted aliphatic hydrocarbon group" for R^{3a}, those similar to the "optionally substituted aliphatic hydrocarbon group" for R³ can be used.

As the "optionally substituted ring structure" constructed by R^{3a} bonded to a carbon atom of the adjacent phenyl group, "optionally substituted ring structure" constructed by R³ bonded to a carbon atom of the adjacent phenyl group can be used.

As R^{3a}, a hydrogen atom is preferable.

As the substituent of the "optionally substituted benzene ring" for B^{a}, for example, 1 to 5, the same or different substituents selected from halogen, optionally halogenated C₁₋₄ alkyl, hydroxy, optionally halogenated C₁₋₄ alkoxy, C₁₋₄ alkoxymethyl, hydroxyl-C₁₋₄ alkyl, C₁₋₄ alkyl-carbonyl, carboxy, C₁₋₄ alkoxy-carbonyl, cyano, carbamoyl, sulfamoyl, nitro, amino, C₁₋₄ alkyl-carbonylamino, C₁₋₄ alkoxy-carbonylamino and C₁₋₄ alkyl-sulfonylamino are used.

As the substituent of the "optionally substituted benzene ring" for B^{a}, halogen, optionally halogenated C₁₋₄ alkyl and the like are preferable and, for example, chlorine, methyl and the like can be mentioned. Of these, halogen (e.g., chlorine) is preferable. As B^{a}, a benzene ring wherein the 1-position of the ring is the carbon atom bonded to N and the 3-position is substituted by chlorine or methyl (preferably chlorine) and the like can be mentioned.

As the "C₆₋₁₈ aryl group" of the "optionally substituted C₆₋₁₈ aryl group" for C^{a}, for example, phenyl, biphenylyl, naphthyl, anthryl, phenanthryl, acenaphthylenyl and the like are used. Of these, phenyl is preferable.

As the "substituent" of the "optionally substituted C₆₋₁₈ aryl group" for C^{a}, those similar to the "optionally substituted benzene ring" for B^{a} can be used.

As the "substituent" of the "optionally substituted C₆₋₁₈ aryl group" for C^{a}, halogen, optionally halogenated C₁₋₄ alkyl, optionally halogenated C₁₋₄ alkoxy and the like are preferable and, for example, chlorine, trifluoromethyl, trifluoromethoxy and the like can be mentioned. Of these, optionally halogenated C₁₋₄ alkyl (e.g., trifluoromethyl) and the like are preferable.

As C^{a}, for example, 3-(trifluoromethyl)phenyl, 3-(trifluoromethoxy)phenyl, 3-chlorophenyl and the like can be mentioned. Of these, 3-(trifluoromethyl)phenyl and the like are preferable.

As R^{2a}, a C₁₋₈ alkyl group, a C₂₋₈ alkenyl group, a C₂₋₈ alkynyl group, a carbamoyl group, a C₁₋₈ alkyl-carbonyl group, a C₁₋₈ alkyl-sulfonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a C₆₋₁₈ aryl-sulfonyl group, a heterocyclic group, a heterocyclyl-C₁₋₄ alkyl group, a heterocyclyl-carbonyl group or a heterocyclyl-C₁₋₄ alkyl-carbonyl group, each optionally substituted by 1 to 5 substituents selected from the group consisting of
(a) halogen,
(b) oxo,
(c) optionally halogenated C₁₋₄ alkyl,
(d) - (CH₂)ₘ-Q,
(e) - (CH₂)ₘ-Z¹- (optionally halogenated C₁₋₄ alkyl) ,
(f) - (CH₂)ₘ-Z¹-C₃₋₈ cycloalkyl,
(g) - (CH₂)ₘ-Z²-(CH₂)ₙ-Q,
(h) -(CH₂)ₘ-Z²-(CH₂)ₙ-Z¹-(optionally halogenated C₁₋₄ alkyl),
(i) - (CH₂)ₘ-Z²-(CH₂)ₙ-Z¹-C₃₋₈ cycloalkyl,
(j) -(CH₂)ₘ-Z¹-(optionally substituted heterocyclic group) (preferably, 5- to 8-membered heterocyclic group having 1 to 3 hetero atoms selected from a nitrogen atom, an oxygen atom and an optionally oxidized sulfur atom),
(k) -(CH₂)ₘ-Z²-C₁₋₄ alkoxy, and
(l) - (CH₂)ₘ-Z²- (CH₂)ₙ-Z¹-(CH₂)ₙ-Z¹-C₁₋₄ alkyl
wherein m is an integer of 0 to 4, n is an integer of 1 to 4,
Q is hydroxy, carboxy, cyano, nitro, -NR⁶R⁷, -CONR⁶R⁷, -OCONH₂ or -SO₂NR⁶R⁷,
Z¹ is -O-, -CO-, -C(OH)R⁸-, -C(=N-OR⁸)-, -S-, -SO-, -SO₂-, - N(COR⁸)-, -N(CO₂R⁹)-, -N(SO₂R⁹)-, -CO-O-, -O-CO-, -CO-NR⁸-, -NR⁸-CO-, -NR⁸-CO₂-_{,} -NR⁸-CO-NH-, -NR⁸-SO₂- or -NR⁸-C(=NH)-NH-,
Z² is -O-, -CO-, -C(OH)R⁸-, -C(=N-OR⁸)-, -S-, -SO-, -SO₂-, -NR⁸- ,-N(COR⁸)-, -N(CO₂R⁹)-, -N(SO₂R⁹)-, -CO-O-, -O-CO-, -CO-NR⁸-, - NR⁸-CO-, -NR8-CO₂-_{,} -NR⁸-CO-NH-, -NR⁸-C(=NH)-NH-, -NR⁸-SO₂- or - SO₂-NR⁸-,
(CH₂)ₘ and (CH₂)ₙ is optionally substituted by 1 to 5 substituents selected from halogen, optionally halogenated C₁₋₄ alkyl and hydroxy, when m or n is not less than 2, a subset - CH₂CH₂- of (CH₂)ₘ and (CH₂)ₙ may be replaced by -CH=CH- or -C≡C-, R⁶ and R⁷ are the same or different and each is a hydrogen atom or a C₁₋₄ alkyl group, or R⁶ and R⁷ are bond to form, together with a nitrogen atom, a 3- to 8-membered saturated or unsaturated aliphatic heterocyclic group,
R⁸ is a hydrogen atom or C₁₋₄ alkyl and R⁹ is C₁₋₄ alkyl, is preferable.

As R^{2a}, optionally substituted C₁₋₈ alkyl group and the like are preferable and, for example, ethyl substituted at the 2-position and the like can be mentioned. As the substituent of the optionally substituted C₁₋₈ alkyl group, (g) - (CH₂)ₘ-Z²-(CH₂)ₙ-Q in the above-mentioned substituent group and the like are preferable, particularly that wherein m is 0, Z² is -NR⁸-CO- or -O- and Q is hydroxy, namely, -O-(CH₂)ₙ-OH or -NR⁸-CO-(CH₂)ₙ-OH ((CH₂)ₙ is optionally substituted by C₁₋₄ alkyl (e.g., methyl)) and the like are preferable. Particularly, -NR⁸-CO-(CH₂)ₙ-OH ((CH₂) is optionally substituted by C₁₋₄ alkyl (e.g., methyl)) is preferable. In these formulas, R⁸ is preferably a hydrogen atom and n is preferably 2. As the (CH₂)ₙ moiety, - CH₂-C(CH₃)₂-, -CH₂-CH₂- and the like can be mentioned and -CH₂-C(CH₃)₂- and the like are preferable.

As the substituent of the optionally substituted C₁₋₈ alkyl group for R^{2a}, (h) -(CH₂)ₘ-Z²-(CH₂)ₙ-Z¹-(optionally halogenated C₁₋₄ alkyl) of the above-mentioned substituent group and the like are preferable, particularly that wherein m is 0, Z₂ is -NR⁸-CO- or -O-, and Z₁ is -SO₂-, namely, -O- (CH₂)ₙ-SO₂-(optionally halogenated C₁₋₄ alkyl) or -NR⁸-CO-(CH₂)ₙ-SO₂-(optionally halogenated C₁₋₄ alkyl) ((CH₂)ₙ is optionally substituted by C₁₋₄ alkyl (e.g., methyl)) and the like are preferable. In these formulas, R⁸ is preferably a hydrogen atom, and n is preferably 1 or 2. As the (CH₂)ₙ moiety, -CH₂-, -CH₂-CH₂-, -C(CH₃)₂- and the like can be mentioned. As the optionally halogenated C₁₋₄ alkyl moiety, for example, methyl, tert-butyl and the like can be mentioned.

As the substituent of the optionally substituted C₁₋₈ alkyl group for R^{2a}, for example, -NH-CO-CH₂-C (CH₃)₂-OH, -O-CH₂-CH₂-OH, -NH-CO-CH₂-SO₂-CH₃, -NH-CO-C(CH₃)₂-SO₂-CH₃, -O-CH₂-CH₂-SO₂-C(CH₃)₃ and the like can be mentioned, and -NH-CO-CH₂-C(CH₃)₂-OH and the like are preferable.

As compound (Ia), a compound wherein
B^{a} is a benzene ring optionally substituted by 1 to 4 substituents selected from halogen, C₁₋₄ alkyl, hydroxy-C₁₋₄ alkyl and C₁₋₄ alkoxy;
C^{a} is a phenyl group optionally substituted by 1 to 5 substituents selected from (i) halogen, (ii) optionally halogenated C₁₋₄ alkyl, (iii) hydroxy-C₁₋₄ alkyl, (iv) heterocyclyl-C₁₋₄ alkyl (preferably, 5- to 8-membered heterocyclyl-C₁₋₄ alkyl, said 5- to 8-membered heterocycle has 1 to 3 hetero atoms selected from a nitrogen atom, an oxygen atom and an optionally oxidized sulfur atom, such as imidazolyl, triazolyl and the like), (v) optionally halogenated C₁₋₄ alkoxy, (vi) C₁₋₄ alkyl-carbonyl, (vii) cyano, (viii) carbamoyl optionally substituted by C₁₋₈ alkyl and (ix) C₁₋₄ alkoxy-carbonyl;
R^{1a} is
(i) a hydrogen atom,
(ii) a cyano group, or
(iii) a C₁₋₄ alkyl group or a C₂₋₄ alkenyl group, each of which is optionally substituted by -NR⁸-CO- (CH₂) -NR⁶R⁷ wherein n is an integer of 1 to 4, R⁶ and R⁷ are the same or different and each is a hydrogen atom or a C₁₋₄ alkyl group, R⁸ is a hydrogen atom or a C₁₋₄ alkyl group, and when n is not less than 2, a subset -CH₂CH₂- of (CH₂)ₙ is optionally replaced by - CH=CH-;
   R^{2a} is a C₁₋₈ alkyl group, a C₂₋₈ alkenyl group or a C₂₋₈ alkynyl group, each of which is optionally substituted by substituent(s) selected from
   (a) hydroxy,
   (b) carboxy,
   (c) cyano,
   (d) optionally halogenated C₁₋₄ alkoxy,
   (e) -O-(CH₂)ₙ-OH,
   (f) -0- (CH₂)ₙ-O-CO-NH₂,
   (g) -O- (CH₂)ₙ-O- (optionally halogenated C₁₋₄ alkyl) ,
   (h) -O- (CH₂)ₙ-SO₂- (optionally halogenated C₁₋₄ alkyl) ,
   (i) -O- (CH₂)ₙ-SO₂-C₆₋₁₈ aryl,
   (j) -O- (CH₂)ₙ-SO₂- (CH₂)ₙ-OH,
   (k) -O- (CH₂)ₙ-NR⁸-CO-C₁₋₄ alkyl,
   (l) -O-(CH₂)ₙ-NR⁸-CO-(CH₂)ₙ-SO₂-C₁₋₄ alkyl,
   (m) -O- (CH₂)ₙ-NR⁸-SO₂- (optionally halogenated C₁₋₄ alkyl) ,
   (n) -CO-NR⁸- (CH₂)ₙ-OH,
   (o) -CO-NR⁸- (CH₂)ₙ-SO₂- (optionally halogenated C₁₋₄ alkyl) ,
   (p) -CO-NR⁸-O-C₁₋₄ alkyl,
   (q) -NR⁶R⁷ ,
   (r) -NR⁸- (CH₂)ₙ-OH,
   (s) -NR⁸- (CH₂)ₙ-SO₂-C₁₋₄ alkyl,
   (t) -NR⁸-CO- (optionally halogenated C₁₋₄ alkyl),
   (u) -NR⁸-CO- (CH₂)ₙ-OH,
   (v) -NR⁸-CO- (CH₂)ₙ-CN,
   (w) -NR⁸-CO- (CH₂)ₙ-NR⁶R⁷,
   (x) -NR⁸-CO- (CH₂)ₙ-O-C₁₋₄ alkyl,
   (y) -NR⁸-CO- (CH₂)ₙ-SO- (optionally halogenated C₁₋₄ alkyl) ,
   (z) -NR⁸-CO- (CH₂)ₙ-SO₂- (optionally halogenated C₁₋₄ alkyl) ,

   (aa) -NR⁸-CO- (CH₂)ₙ-SO₂-C₃₋₈ cycloalkyl,
   (bb) -NR⁸-CO- (CH₂)ₙ-NR⁸-SO²-C₁₋₄ alkyl,
   (cc) -NR⁸-CO²- (CH₂)ₙ-SO₂-C₁₋₄ alkyl,
   (dd) -NR⁸-CO-NH- (CH₂)ₙ-SO₂-C₁₋₄ alkyl,
   (ee) -NR⁸-CO-NH-O-C₁₋₄ alkyl,
   (ff) -NR⁸-CO-NH-(CH₂)ₙ-O-C₁₋₄ alkyl,
   (gg) -NR⁸-C (=NH) -NH-C₁₋₄ alkyl,
   (hh) -NR⁸-SO₂- (CH₂)ₙ-SO₂-C₁₋₄ alkyl,
   (ii) -S-(CH₂₎ₙ-OH,
   (jj) -SO-(CH₂)ₙ-OH,
   (kk) -SO₂-(CH₂)ₙ-OH, and
   (11) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-O-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
   wherein n is an integer of 1 to 4, R⁶ and R⁷ are the same or different and each is a hydrogen atom or a C₁₋₄ alkyl group, R⁸ is a hydrogen atom or a C₁₋₄ alkyl group, (CH₂)ₙ is optionally substituted by optionally halogenated C₁₋₄ alkyl or hydroxy, and when n is not less than 2, a subset -CH₂CH₂- of (CH₂)ₙ is optionally replaced by -CH=CH-; and
   R^{3a} is a hydrogen atom or a C₁₋₆ alkyl group; or R^{1a} and R^{2a} are optionally bonded to form 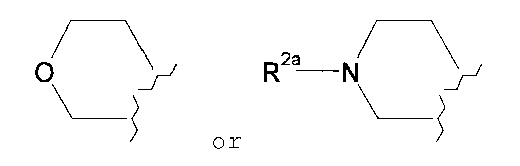 R^{2a} and R^{3a} are optionally bonded to form C₂₋₄ alkylene optionally substituted by an imino group is preferable.

As R⁸, a hydrogen atom, methyl, ethyl and the like are preferable, and a hydrogen atom is particularly preferable.

As R^{2a}, a C₁₋₈ alkyl group, a C₂₋₈ alkenyl group or a C₂₋₈ alkynyl group, each of which is optionally substituted by substituent(s) selected from
(a) hydroxy,
(b) carboxy,
(c) cyano,
(d) optionally halogenated C₁₋₄ alkoxy,
(e) -O-(CH₂)ₙ-OH (wherein (CH₂)ₙ is optionally substituted by hydroxy),
(f) -O- (CH₂)ₙ-O-CO-NH₂,
(g) -O- (CH₂)ₙ-0- (optionally halogenated C₁₋₄ alkyl) ,
(h) -O- (CH₂)ₙ-SO₂- (optionally halogenated C₁₋₄ alkyl) ,
(i) -O- (CH₂)ₙ-SO₂-C₆₋₁₈ aryl,
(j) -O- (CH₂)ₙ-SO₂- (CH₂)ₙ-OH,
(k) -O- (CH₂)ₙ-NR⁸-CO-C₁₋₄ alkyl,
(l) -O-(CH₂)ₙ-NR⁸-CO-(CH₂)ₙ-SO₂-C₁₋₄ alkyl,
(m) -O- (CH₂)ₙ-NR⁸-SO₂- (optionally halogenated C₁₋₄ alkyl) ,
(n) -CO-NR⁸- (CH₂)ₙ-OH,
(o) -CO-NR⁸- (CH₂)ₙ-SO₂- (optionally halogenated C₁₋₄ alkyl) ,
(p) -CO-NR⁸-O-C₁₋₄ alkyl,
(q) -NR⁶R⁷ ,
(r) -NR⁸- (CH₂)ₙ-OH,
(s) -NR⁸-(CH₂)ₙ-SO₂-C₁₋₄alkyl,
(t) -NR⁸-CO- (optionally halogenated C₁₋₄ alkyl) ,
(u) -NR⁸-CO-(CH₂)ₙ-OH (wherein (CH₂) is optionally substituted by optionally halogenated C₁₋₄ alkyl or hydroxy),
(v) -NR⁸-CO- (CH₂)ₙ-CN,
(w) -NR⁸-CO-(CH₂)ₙ-NR⁶R⁷ (when n is not less than 2, a subset -CH₂CH₂- of (CH₂)ₙ is optionally replaced by -CH=CH-),
(x) -NR⁸-CO-(CH₂)ₙ-O-C₁₋₄ alkyl,
(y) -NR⁸-CO- (CH₂)ₙ-SO- (optionally halogenated C₁₋₄ alkyl) ,
(z) -NR⁸- CO-(CH₂),-SO₂- (optionally halogenated C₁₋₄ alkyl)
   (wherein (CH₂)ₙ is optionally substituted by C₁₋₄ alkyl),

(aa) -NR⁸-CO- (CH₂)ₙ-SO₂-C₃₋₈ cycloalkyl,
(bb) -NR⁸-CO- (CH₂)-NR⁸-SO₂-C₁₋₄ alkyl,
(cc) -NR⁸-CO₂-(CH₂)ₙ-SO₂-C₁₋₄ alkyl,
(dd) -NR⁸-CO-NH- (CH₂)ₙ-SO₂-C₁₋₄ alkyl,
(ee) -NR⁸-CO-NH-O-C₁₋₄ alkyl,
(ff) -NR⁸-CO-NH-(CH₂)ₙ-O-C₁₋₄ alkyl,
(gg) -NR⁸-C(=NH) -NH-C₁₋₄ alkyl,
(hh) -NR⁸-SO₂- (CH₂)ₙ-SO₂-C₁₋₄ alkyl,
(ii) -S-(CH₂)ₙ-OH,
(jj) -SO- (CH₂)ₙ-OH,
(kk) -SO₂-(CH₂)ₙ-OH, and
(11) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-O-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
wherein n is an integer of 1 to 4, R⁶ and R⁷ are the same or different and each is a hydrogen atom or a C₁₋₄ alkyl group, and R⁸ is a hydrogen atom or a C₁₋₄ alkyl group, is preferable.

As R⁸, a hydrogen atom, methyl, ethyl and the like are preferable, and a hydrogen atom is particularly preferable.

As compound (Ia), moreover, a compound wherein B^{a} is a benzene ring optionally substituted by 1 to 4 substituents selected from halogen and optionally halogenated C₁₋₄ alkyl;
C^{a} is a phenyl group substituted by 1 to 5 substituents selected from (i) halogen, (ii) optionally halogenated C₁₋₄ alkyl, (iii) hydroxy-C₁₋₄ alkyl, (iv) heterocyclyl-C₁₋₄ alkyl (preferably, 5- to 8-membered heterocyclyl-C₁₋₄ alkyl, said 5-to 8-membered heterocycle has 1 to 3 hetero atoms selected from a nitrogen atom, an oxygen atom and an optionally oxidized sulfur atom, such as imidazolyl and the like), (v) optionally halogenated C₁₋₄ alkoxy, (vi) cyano, and (vii) carbamoyl optionally substituted by C₁₋₈ alkyl;
_{R}^{1a} is a hydrogen atom;
R^{2a} is a C₁₋₈ alkyl group, a C₂₋₈ alkenyl group or a C₂₋₈ alkynyl group, each of which is substituted by substituent(s) selected from
(a) hydroxy,
(b) optionally halogenated C₁-₄ alkoxy,
(c) -O-(CH₂) ₙ-OH,
(d) -O- (CH₂) ₙ-O-CO-NH₂,
(e) -O- (CH₂) ₙ-O-C₁₋₄ alkyl,
(f) -O- (CH₂) ₙ-SO₂- (optionally halogenated C₁₋₄ alkyl) ,
(g) -O- (CH₂) ₙ-SO₂-C₆₋₁₈ aryl
(h) -O- (CH₂) ₙ-SO₂- (CH₂) ₙ-OH,
(i) -O- (CH₂) ₙ-NR⁸-SO₂- (optionally halogenated C₁₋₄ alkyl) ,
(j) -CO-NR⁸- (CH₂) ₙ-OH,
(k) -CO-NR⁸-(CH₂) ₙ-SO₂- (optionally halogenated C₁₋₄ alkyl),
(l) -NR⁶R⁷,
(m) -NR⁸- (CH₂) ₙ-OH,
(n) -NR⁸- (CH₂) ₙ-SO₂-C₁₋₄ alkyl,
(o) -NR⁸-CO- ( CH₂) ₙ-OH,
(p) -NR⁸-CO**-** (CH₂) ₙ-O-C₁₋₄ alkyl,
(q) -NR⁸-CO- (CH₂) ₙ-SO- (optionally halogenated C₁₋₄ alkyl) ,
(r) -NR⁸-CO- (CH₂) ₙ-SO₂-(optionally halogenated C₁₋₄ alkyl) ,
(s) -NR⁸-CO- (CH₂)ₙ-SO₂-C₃-₈ cycloalkyl,
(t) -NR⁸-CO₂- (CH₂)ₙ-SO₂-C₁₋₄ alkyl,
(u) -NR⁸-CO-NH- (CH₂) ₙ-SO₂-C₁₋₄ alkyl,
(v) -NR⁸-SO₂- (CH₂) ₙ-SO₂-C₁₋₄ alkyl,
(w) -S- (CH₂) ₙ-OH,
(x) -SO- (CH₂) ₙ-OH,
(y) -SO₂- (CH₂) ₙ-OH, and
(z) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
wherein n is an integer of 1 to 4, R⁶ and R⁷are the same or different and each is a hydrogen atom or a C₁₋₄ alkyl group, R⁸ is a hydrogen atom or a C₁₋₄ alkyl group, and (CH₂) is optionally substituted by C₁₋₄ alkyl or hydroxy;
R^{3a} is a hydrogen atom or a C₁₋₆ alkyl group; or
R^{1a} and R^{2a} are optionally bonded to form 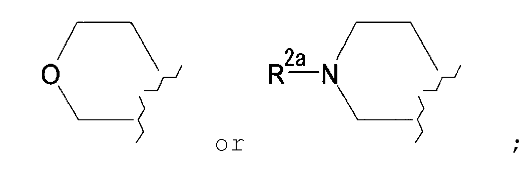 and
R^{2a} and R^{3a} are optionally bonded to form C₂₋₄ alkylene, is preferable.

Of these, as R^{2a}, a C₁₋₈ alkyl group, a C₂₋₈ alkenyl group or a C₂-₈ alkynyl group (particularly, a C₁₋₈ alkyl group), each of which is substituted by substituent(s) selected from
(a) hydroxy,
(b) optionally halogenated C₁₋₄ alkoxy,
(c) -O- (CH_{2) n}-OH (wherein (CH₂) is optionally substituted by hydroxy),
(d) -O- (CH₂) ₙ-O-CO-NH₂,
(e) -O- (CH₂),-O-C₁₋₄ alkyl,
(f) -O- (CH₂)ₙ-SO2- (optionally halogenated C₁₋₄ alkyl) ,
(g) -O- (CH₂) ₙ-SO₂-C₆₋₁₈ aryl,
(h) -O- (CH₂)ₙ-SO2- (CH₂)ₙ-OH,
(i) -O- (CH₂)ₙ-NR⁸-SO₂- (optionally halogenated C₁₋₄ alkyl) ,
(j) -CO-NR⁸-( CH₂)ₙ-OH,
(k) -CO-NR⁸- (CH₂) ₙ-SO₂- (optionally halogenated C₁₋₄ alkyl) ,
(l) - NR⁶R⁷,
(m) -NR⁸-(CH₂) ₙ-OH,
(n) -NR⁸- (CH₂) ₙ-SO₂-C₁₋₄ alkyl,
(o) -NR⁸-CO- (CH₂) ₙ-OH (wherein (CH₂)ₙ is optionally substituted by C₁-₄ alkyl) ,
(p) -NR ⁸- CO- (CH₂) ₙ-O-C₁₋₄ alkyl,
(q) -NR⁸-CO- (CH₂) ₙ-SO- (optionally halogenated C₁₋₄ alkyl),
(r) -NR⁸-CO- (CH₂) ₙ-SO₂- (optionally halogenated C₁₋₄ alkyl) (wherein (CH₂)ₙ is optionally substituted by C₁₋₄ alkyl),
(s) -NR⁸-CO- (CH₂) ₙ-SO₂-C₃₋₈ cycloalkyl,
(t) -NR⁸-CO₂- (CH₂) ₙ-SO₂-C₁₋₄ alkyl,
(u) -NR⁸-CO-NH- (CH₂) ₙ-SO₂-C₁₋₄ alkyl,
(v) -NR⁸-SO₂- (CH₂) ₙ-SO₂-C₁₋₄ alkyl,
(w) -S-(CH₂) ₙ-OH,
(x) -SO- (CH₂)ₙ-OH,
(y) -SO₂- (CH₂) ₙ-OH, and
(z) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
   wherein n is an integer of 1 to 4, R⁶ and R⁷ are the same or different and each is a hydrogen atom or a C₁₋₄ alkyl group, R⁸ is a hydrogen atom or a C₁₋₄ alkyl group, is preferable.

As R^{2a}, (i) a C₅₋₈ alkyl group substituted by hydroxy, (ii) a C₁-₈ alkyl group substituted by substituent(s) selected from
(a) halogenated C₁₋₄ alkoxy,
(b) -O- ( CH₂) ₙ-OH,
(c) -O- ( CH₂ ) ₙ-O-CO-NH₂ ,
(d) -O- (CH₂) ₙ-O- (optionally halogenated C₁₋₄ alkyl) ,
(e) -O- (CH₂) ₙ-SO₂- (optionally halogenated C₁₋₄ alkyl),
(f) -O- (CH₂) ₙ-SO₂-C₆₋₁₈ aryl,
(g) -O- (CH₂) ₙ-NR⁸-SO₂- (optionally halogenated C₁₋₄ alkyl),
(h) -CO-NR⁸- (CH₂) ₙ-OH,
(i) -CO-NR⁸- (CH₂) ₙ-SO2- (optionally halogenated C₁₋₄ alkyl) ,
(j) -NR⁸- (CH₂) ₙ-SO₂-C₁₋₄ alkyl,
(k) -NR⁸-CO- (CH₂ ) ₙ-OH,
(l) -NR⁸-CO- (CH₂) ₙ-O-C₁₋₄ alkyl,
(m) -NR⁸-CO- (CH₂) ₙ-SO- (optionally halogenated C₁₋₄ alkyl),
(n) -NR⁸-CO- (CH₂) ₙ-SO₂- (optionally halogenated C₁₋₄ alkyl),
(o) -NR⁸-CO- (CH₂) ₙ-SO₂-C₃₋₈ cycloalkyl,
(p) -NR⁸-CO₂- (CH₂) ₙ-SO₂-C₁-₄ alkyl,
(q) -NR⁸-CO-NH- (CH₂) ₙ-SO₂-C₁₋₄ alkyl,
(r) -NR⁸-SO₂- (CH₂) ₙ-SO₂-C₁₋₄ alkyl,
(s) -S-(CH₂)ₙ-OH,
(t) -SO- (CH₂) ₙ-OH,
(u) -SO₂- (C_{H}2_{) n}-OH, and
(v) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁-₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
   wherein n is an integer of 1 to 4, R⁸ is a hydrogen atom or a C₁₋₄ alkyl group, and (CH₂) is optionally substituted by C₁₋₄ alkyl or hydroxy,
   (iii) a C₂₋₈ alkenyl group optionally substituted by hydroxy, or (iv) a C₂₋₈ alkynyl group optionally substituted by hydroxy is preferable, and particularly,
      as R^{2a}, (i) a C₅₋₈ alkyl group substituted by hydroxy,
   (ii) a C₁₋₈ alkyl group substituted by substituent(s) selected from
      (a) halogenated C₁₋₄ alkoxy,
      (b) -O- (CH₂) ₙ-OH (wherein (CH₂) is optionally substituted by hydroxy),
      (c) -O- ( CH₂ )ₙ-O-CO-NH₂ ,
      (d) -O- (CH₂) ₙ-O- (optionally halogenated C₁₋₄ alkyl),
      (e) -O- (CH₂) ₙ-SO₂- (optionally halogenated C₁₋₄ alkyl),
      (f) -O- (CH₂) ₙ-SO₂-C₆₋₁₈ aryl,
      (g) -O- (CH₂) ₙ-NR⁸-SO₂- (optionally halogenated C₁₋₄ alkyl),
      (h) -CO-NR⁸- (CH₂) ₙ-OH,
      (i) -CO-NR⁸- (CH₂) ₙ-SO₂- (optionally halogenated C₁₋₄ alkyl),
      (j) -NR⁸- (CH₂) ₙ-SO₂-C₁₋₄ alkyl,
      (k) -NR⁸-CO- (CH₂) ₙ-OH (wherein (CH₂)ₙ is optionally substituted by C₁₋₄ alkyl),
      (l) -NR8⁻CO- (CH₂) ₙ-O-C₁₋₄ alkyl,
      (m) -NR⁸-CO- (CH₂) ₙ-SO- (optionally halogenated C₁₋₄ alkyl),
      (n) -NR⁸-CO-(CH₂),-SO₂- (optionally halogenated C₁₋₄ alkyl) (wherein (CH₂)ₙ is optionally substituted by C₁₋₄ alkyl),
      (o) -NR⁸-CO- (CH₂) ₙ-SO₂-C₃₋₈ cycloalkyl,
      (p) -NR⁸-CO₂- (CH₂) ₙ-SO₂-C₁₋₄ alkyl,
      (q) -NR⁸-CO-NH- (CH₂)ₙ-SO₂-C₁₋₄ alkyl,
      (r) -NR⁸-SO₂- (CH₂)ₙ-SO₂-C₁₋₄ alkyl,
      (s) -S-(CH₂)ₙ-OH,
      (t) -SO-(CH₂) ₙ-OH,
      (u) -SO₂- (CH₂) ₙ-OH, and
      (v) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like), wherein n is an integer of 1 to 4, and R⁸ is a hydrogen atom or a C₁₋₄ alkyl group,
   (iii) a C₂₋₈ alkenyl group optionally substituted by hydroxy, or
   (iv) a C₂₋₈ alkynyl group optionally substituted by hydroxy is preferable, and as R⁸, a hydrogen atom, methyl, ethyl and the like are preferable, and a hydrogen atom is particularly preferable.

### [Compound (Ib)]

A compound represented by the formula: 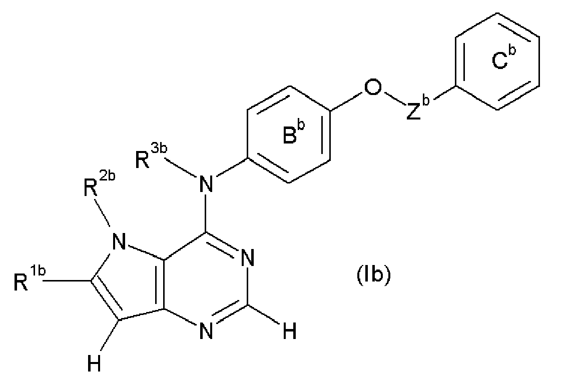 wherein R^{1b} is a hydrogen atom or an optionally substituted group bonded via a carbon atom, a nitrogen atom or an oxygen atom,
_{R}^{2b} is an optionally substituted group bonded via a carbon atom or a sulfur atom, or
_{R}^{1b} and _{R}^{2b}, or _{R}^{2b} and _{R}^{3b} are optionally bonded to form an optionally substituted ring structure,
_{R}^{3b} is a hydrogen atom or an optionally substituted aliphatic hydrocarbon group, or R^{3b} is optionally bonded to a carbon atom of the adjacent phenyl group to form an optionally substituted ring structure,
B^{b} is an optionally substituted benzene ring, C^{b} is an optionally substituted C₆₋₁₈ aryl group, and
z^{b} is an optionally substituted C₁₋₃ alkylene group.

As the "optionally substituted group bonded via a carbon atom, a nitrogen atom or an oxygen atom" for R^{1b}, those similar to the "optionally substituted group bonded via a carbon atom, a nitrogen atom or an oxygen atom" for R¹ can be used.

As _{R}^{1b}, a hydrogen atom is preferable.

As the "optionally substituted group bonded via a carbon atom or a sulfur atom" for R^{2b}, those similar to the "optionally substituted group bonded via a carbon atom or a sulfur atom" for R² can be used.

As the "optionally substituted ring structure" formed by _{R} ^{1b} and _{R}^{2b}, or _{R}^{2b} and _{R}^{3b} bonded to each other, those similar to the "optionally substituted ring structure" formed by R¹ and _{R}², or R² and R³ bonded to each other can be used.

As the "optionally substituted aliphatic hydrocarbon group" for R^{3b}, those similar to the "optionally substituted aliphatic hydrocarbon group" for R³ can be used.

As the "optionally substituted ring structure" formed by _{R}^{3b} and a carbon atom of the adjacent phenyl group, those similar to the "optionally substituted ring structure" formed by R³ and a carbon atom of the adjacent phenyl group can be used.

As _{R}^{3b}, a hydrogen atom is preferable.

As the "optionally substituted benzene ring" for B^{b}, those similar to the "optionally substituted benzene ring" for B^{a} can be used.

As the substituent of the "optionally substituted benzene ring" for B^{b}, halogen and the like are preferable and, for example, chlorine and the like can be mentioned, and halogen (e.g., chlorine) is preferable. As B^{b}, a benzene ring wherein the 1-position of the ring is the carbon atom bonded to N and the 3-position is substituted by chlorine and the like can be mentioned.

As the "optionally substituted C₆-₁₈ aryl group" for C^{b}, those similar to the "optionally substituted C₆-₁₈ aryl group" for C^{a} can be used. As the "C₆-₁₈ aryl group" of the "optionally substituted C₆-₁₈ aryl group" for C^{b}, phenyl is preferable.

As the substituent of the "optionally substituted C₆-₁₈ aryl group" for C^{b}, halogen and the like are preferable and, for example, fluorine and the like can be mentioned, and halogen (e.g., fluorine) is preferable.

As C^{b}, for example, 3-fluorophenyl and the like can be mentioned.

As the "C₁-₃ alkylene group" of the "optionally substituted C₁-₃ alkylene group" for 2^{b}, methylene, ethylene, trimethylene and propylene can be used. Of these, methylene is preferable.

As the "substituent" of the "optionally substituted C₁-₃ alkylene group" for Z^{b}, 1 to 3 substituents selected from halogen, hydroxy, C₁₋₄ alkoxy, C₁₋₄ alkyl-carbonyl, carboxy, C₁₋₄ alkoxy-carbonyl, cyano, carbamoyl, sulfamoyl, nitro, amino, C₁₋₄ alkyl-carbonylamino, C₁₋₄ alkoxy-carbonylamino and C₁₋₄ alkyl-sulfonylamino can be used.

As R^{2b}, a C₁₋₈ alkyl group, a C₂₋₈ alkenyl group, a C₂₋₈ alkynyl group, a carbamoyl group, a C₁₋₈ alkyl-carbonyl group, a C₁₋₈ alkyl-sulfonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a C₆₋₁₈ aryl-sulfonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted by 1 to 5 substituents selected from
(a) halogen,
(b) oxo,
(c) optionally halogenated C₁₋₄ alkyl,
(d) - (CH₂) ₘ-Q,
(e) - (CH₂) ₘ-Z¹- (optionally halogenated C₁₋₄ alkyl) ,
(f) -(CH₂)ₘ-Z¹-C₃₋₈ cycloalkyl,
(g) - (CH₂) ₘ-Z²- (CH₂) ₙ-Q,
(h) -(CH₂)ₘ-Z²-(CH₂)ₙ-Z¹-(optionally halogenated C₁₋₄ alkyl),
(i) - (CH₂) ₘ-Z²- (CH₂) ₙ-Z¹-C₃₋₈ cycloalkyl,
(j) - (CH₂) ₘ-Z¹- (optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom),
(k) -(CH₂)ₘ-Z²-C₁₋₄ alkoxy, and
(l) - (CH₂)ₘ-Z²- (CH₂)ₙ-Z¹- (CH₂)ₙ-Z¹-C₁-₄ alkyl
wherein m is an integer of 0 to 4, n is an integer of 1 to 4, Q is hydroxy, carboxy, cyano, nitro, -NR⁶R⁷, -CONR⁶R⁷, -OCONH₂ or -SO₂NR⁶R⁷
Z¹ is -O-, -CO-, -C (OH) R⁸-, -C(=N-OR⁸)_, -S-, -SO-, -SO₂-, -N(COR⁸)-, -N (CO₂R⁹) -, -N (SO₂R⁹) -, -CO-0-, -O-CO-, -CO-NR⁸-, -NR⁸-CO-, -NR⁸-CO₂-, -NR⁸-CO-NH-, -NR⁸-SO₂-_{,} or -NR⁸-C (=NH) -NH-, z² is -O-, -CO-, -C (OH) R⁸-, -C (=N-OR⁸) -, -S-, -SO-, -SO₂-, -NR⁸-, -N(COR⁸)-, -N (CO₂R⁹ ) -, -N (SO₂R⁹ ) -, -CO-O-, -O-CO-, -CO-NR⁸-, -NR⁸-CO-, -NR⁸-CO2-_{,} -NR⁸-CO-NH-, -NR⁸-C (=NH) -NH-, -NR⁸-SO₂-_{,} or -SO₂-NR⁸-,
(CH₂)ₘ and (CH₂)ₙ are optionally substituted by 1 to 5 substituents selected from halogen, optionally halogenated C₁-₄ alkyl and hydroxy, and when m or n is not less than 2, a subset -CH₂CH₂- of (CH₂)ₘ and (CH₂)ₙ is optionally replaced by -CH=CH- or -C=_C-,
R⁶ and R⁷ are the same or different and each is a hydrogen atom or a C₁-₄ alkyl group, or R⁶ and R⁷ are bonded to form, together with a nitrogen atom, a 3- to 8-membered saturated or unsaturated aliphatic heterocyclic group,
R⁸ is a hydrogen atom or C₁₋₄ alkyl, and R⁹ is C₁-₄ alkyl, is preferable.

As R^{2b}, optionally substituted C₁-₈ alkyl group and the like are preferable and, for example, ethyl wherein the 2-position is substituted and the like can be mentioned. As the substituent of the optionally substituted C₁-₈ alkyl group, (g) - (CH₂) ₘ-2²- (CH₂) ₙ-Q in the above-mentioned substituent group and the like are preferable, and particularly, that wherein m is 0, 2² is -O- and Q is hydroxyl, namely, -O-(CH₂)ₙ-OH and the like are preferable. In the formula, n is preferably 2.

As the substituent of the optionally substituted C₁-₈ alkyl group for R^{2b}, for example, -O-CH₂-CH₂-OH and the like can be mentioned.

As compound (Ib), a compound wherein
B^{b} is a benzene ring optionally substituted by halogen;
c^{b} is a phenyl group optionally substituted by 1 to 5 substituents selected from halogen, optionally halogenated C₁-₄ alkyl and cyano;
R^{1b} is (i) a hydrogen atom, or
(ii) a C₂-₄ alkenyl group optionally substituted by hydroxy; R^{2b} is
(i) a C₁-₈ alkyl group optionally substituted by substituent(s) selected from
   (a) halogen,
   (b) hydroxy,
   (c) C1-₄ alkoxy,
   (d) -O- (CH₂) ₙ-OH,
   (e) -O- (CH₂)ₙ-O-C₁-₄ alkyl,
   (f) -CO-NR⁸- (CH₂) ₙ-OH,
   (g) -NR⁶R⁷, and
   (h) -NR⁸- (CH₂) ₙ-OH,
      wherein n is an integer of 1 to 4, R⁶ and R⁷ are the same or different and each is a hydrogen atom or a C₁₋₄ alkyl group, and R⁸ is a hydrogen atom or a C₁-₄ alkyl group,
(ii) a C₆-₁₈ aryl-C₁-₄ alkyl group optionally substituted by substituent(s) selected from
   (a) C₁₋₄ alkyl optionally having hydroxy,
   (b) carboxy,
   (c) C₁-₄ alkoxy-carbonyl,
   (d) 5- to 8-membered heterocyclyl-carbonyl having 1 to 3 hetero atoms selected from a nitrogen atom, an oxygen atom and a sulfur atom, which optionally has substituent(s) selected from hydroxy and C₁-₄ alkyl, and
   (e) C₁-₄ alkyl-carbamoyl optionally having substituent(s) selected from hydroxy and carbamoyl,
(iii) a C₆-₁₈ aryl-carbonyl group optionally substituted by C₁-₄ alkoxy,
(iv) a C₆-₁₈ aryl-sulfonyl group optionally substituted by C₁-₄ alkoxy, or
(v) a 5- to 8-membered heterocyclyl-C₁-₄ alkyl group having 1 to 3 hetero atoms selected from a nitrogen atom, an oxygen atom and a sulfur atom, which is optionally substituted by substituent(s) selected from
   (a) carboxy, and
   (b) C₁-₄ alkoxy-carbonyl;
   R^{3b} is a hydrogen atom or a C₁-₆ alkyl group; or
   R^{2b} and R^{3b} are optionally bonded to form C₂-₄ alkylene; and
   Z^{b} is a C₁-₃ alkylene group, is preferable.

Moreover, as compound (Ib), a compound wherein
B^{b} is a benzene ring optionally substituted by halogen;
C^{b} is a phenyl group optionally substituted by 1 to 5 substituents selected from halogen and optionally halogenated C₁-₄ alkyl;
_{R}^{1b} is a hydrogen atom;
R^{2b} is a C₁-₈ alkyl group optionally substituted by substituent(s) selected from
(a) hydroxy,
(b) -O- (CH₂) ₙ-OH,
(c) -O- (CH₂) -O-C₁-₄ alkyl,
(d) -CO-NR⁸- (CH₂) ₙ-OH,
(e) -NR⁶R⁷, and
(f) -NR⁸- (CH₂)ₙ -OH,
wherein n is an integer of 1 to 4, R⁶ and R⁷are the same or different and each is a hydrogen atom or a C₁-₄ alkyl group, and R⁸ is a hydrogen atom or a C₁-₄ alkyl group;
R^{3b} is a hydrogen atom or a C₁-₆ alkyl group; and
2^{b} is a C₁-₃ alkylene group is preferable.

Particularly, as compound (Ib), a compound wherein B^{b} is a benzene ring optionally substituted by halogen; C^{b} is a phenyl group optionally substituted by 1 to 5 substituents selected from halogen and optionally halogenated C₁-₄ alkyl;
R^{1b} is a hydrogen atom;
R^{2b} is a C₁-₈ alkyl group substituted by substituent(s) selected from
(a) -O- (CH₂) ₙ-OH,
(b) -O-(CH₂) ₙ-O-C₁₋₄ alkyl, and
(c) -CO-NR⁸- (CH₂)ₙ-OH,
wherein n is an integer of 1 to 4, and R⁸ is a hydrogen atom or a C₁-₄ alkyl group;
R^{3b} is a hydrogen atom or a C₁-₆ alkyl group; and
2^{b} is a methylene group is preferable.

As R⁸, a hydrogen atom, methyl, ethyl and the like are preferable, and a hydrogen atom is particularly preferable.

### [Compound (Ic)]

A compound represented by the formula: 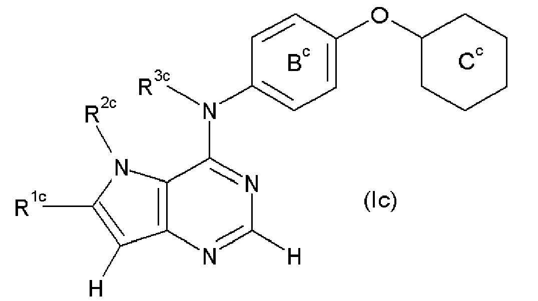 wherein R^{1c} is a hydrogen atom or an optionally substituted group bonded via a carbon atom, a nitrogen atom or an oxygen atom,
R^{2c} is an optionally substituted group bonded via a carbon atom or a sulfur atom, or
R^{1c} and R^{2c}, or R^{2c} and R^{3c} are optionally bonded to form an optionally substituted ring structure,
R^{3c} is a hydrogen atom or an optionally substituted aliphatic hydrocarbon group, or R^{3c} is optionally bonded to a carbon atom of the adjacent phenyl group to form an optionally substituted ring structure,
B^{C} is an optionally substituted benzene ring, and C^{c} is an optionally substituted heterocyclic group.

As the "optionally substituted group bonded via a carbon atom, a nitrogen atom or an oxygen atom" for R^{1c}, those similar to the "optionally substituted group bonded via a carbon atom, a nitrogen atom or an oxygen atom" for R¹ can be used.

As the "optionally substituted group bonded via a carbon atom or a sulfur atom" for R^{2c}, those similar to the "optionally substituted group bonded via a carbon atom or a sulfur atom" for R² can be used.

As the "optionally substituted ring structure" formed by R^{1c} and R^{2c}, or R^{2c} and R^{3c} bonded to each other, those similar to the "optionally substituted ring structure" formed by R¹ and R² or R² and R³ bonded to each other can be used.

As the "optionally substituted aliphatic hydrocarbon group" for R^{3c}, those similar to the "optionally substituted aliphatic hydrocarbon group" for R³ can be used.

As the "optionally substituted ring structure" formed by R^{3c} and a carbon atom of the adjacent phenyl group, those similar to the "optionally substituted ring structure" formed by R³ and a carbon atom of the adjacent phenyl group can be used.

As the "optionally substituted benzene ring" for B^{c}, those similar to the "optionally substituted benzene ring" for B^{a} can be used.

As the "heterocyclic group" of the "optionally substituted heterocyclic group" for C^{C}, the aforementioned "heterocyclic group" can be used, and a 5- to 8-membered heterocyclic group having 1 to 3 hetero atoms selected from a nitrogen atom, an oxygen atom and an optionally oxidized sulfur atom can be particularly preferably used. Specifically, 5- or 6-membered aromatic monocyclic heterocyclic groups such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl and the like, 3- to 8-membered (preferably 5- or 6-membered) saturated or unsaturated (preferably saturated) aliphatic heterocyclic groups such as oxiranyl, azetidinyl, oxetanyl, thietanyl, pyrrolidinyl, tetrahydrofuryl, thiolanyl, piperidyl, tetrahydropyranyl, morpholinyl, thiomorpholinyl, piperazinyl, dihydro-1,2,4-oxadiazolyl and the like can be used, and particularly, pyridyl, pyrimidinyl, piperidyl (particularly, 4-piperidyl) and the like are preferable.

As the "substituent" of the "optionally substituted heterocyclic group" for C^{c}, those similar to the "substituent" of the "optionally substituted C₆-₁₈ aryl group" for C^{a} can be used.

As R ^{2c} , a C₁-₈ alkyl group, a C₂₋₈ alkenyl group, a C₂₋₈ alkynyl group, a carbamoyl group, a C₁-₈ alkyl-carbonyl group, a C₁-₈ alkyl-sulfonyl group, a C₃-₈ cycloalkyl group, a C₆-₁₈ aryl group, a C₆-₁₈ aryl-C₁-₄ alkyl group, a C₆-₁₈ aryl-carbonyl group, a C₆-₁₈ aryl-C₁-₄ alkyl-carbonyl group, a C₆-₁₈ aryl-sulfonyl group, a heterocyclic group, a heterocyclyl-C₁-₄ alkyl group, a heterocyclyl-carbonyl group or a heterocyclyl-C₁-₄ alkyl-carbonyl group, each of which is optionally substituted by 1 to 5 substituents selected from the group consisting of
(a) halogen,
(b) oxo,
(c) optionally halogenated C₁₋₄ alkyl,
(d) - (CH₂) ₘ-Q,
(e) - (CH₂) ₘ-Z¹- (optionally halogenated C₁₋₄ alkyl) ,
(f) -(CH₂)ₘ-Z¹-C₃-₈ cycloalkyl,
(g) - (CH₂) ₘ-Z²- (CH₂) ₙ-Q
(h) -(CH₂)ₘ-Z²-(CH2)ₙ-Z¹-(optionally halogenated C₁₋₄ alkyl) ,
(i) - (CH₂) ₘ-Z²- (CH₂) ₙ-Z¹-C₃-₈ cycloalkyl,
(j) -(CH₂) ₘ-Z¹- (optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom),
(k) -(CH₂)ₘ-Z²-C₁-₄ alkoxy, and
(l) - (CH₂)ₘ-Z²- (CH₂)ₙ-Z¹- (CH₂)ₙ-Z¹-C₁-₄ alkyl
wherein m is an integer of 0 to 4, n is an integer of 1 to 4, Q is hydroxy, carboxy, cyano, nitro, -NR ⁶R⁷, -CONR⁶R⁷ or -S0₂NR⁶R⁷,
Z¹ is -O-, -CO-, -C (OH) R⁸-, -C(=N-OR⁸)_, -S-, -SO-, -SO₂-, -N(COR⁸)-, -N (CO₂R⁹) -, -N (SO₂R⁹) -, -CO-O-, -O-CO-, -CO-NR⁸-, -NR⁸-CO-, -NR⁸-CO₂-, -NR⁸-CO-NH-, -NR⁸-SO₂-, or -NR⁸-C (=NH) -NH-, z² is -O-, -CO-, -C (OH) R⁸-, -C(=N-OR⁸)-, -S-, -SO-, -SO₂-, -NR⁸-, -N(COR⁸)-, -N (CO₂R⁹) -, -N (SO₂R⁹) -, -CO-O-, -O-CO-, -CO-NR⁸-, -NR⁸-CO-, -NR⁸-CO₂-, -NR⁸-CO-NH-, -NR⁸-C (=NH) -NH-, -NR⁸-SO₂-, or -SO₂-NR⁸-,
(CH₂)ₘ and (CH₂)ₙ are optionally substituted by 1 to 5 substituents selected from halogen, optionally halogenated C₁-₄ alkyl and hydroxy, and when m or n is not less than 2, a subset -CH₂CH₂- of (CH₂)ₘ and (CH₂)ₙ is optionally replaced by -CH=CH-,
R⁶ and R⁷ are the same or different and each is a hydrogen atom or a C₁-₄ alkyl group, or R⁶ and R⁷ are bonded to form, together with a nitrogen atom, a 3- to 8-membered saturated or unsaturated aliphatic heterocyclic group,
R⁸ is a hydrogen atom or C₁-₄ alkyl, and R⁹ is C₁-₄ alkyl, is preferable.

As compound (Ic), a compound wherein
B^{c} is a benzene ring optionally substituted by 1 to 4 substituents selected from halogen and optionally halogenated C₁-₄ alkyl;
C^{c} is a 5- to 8-membered heterocyclic group having 1 to 3 hetero atoms selected from a nitrogen atom, an oxygen atom and a sulfur atom (e.g., pyridyl, pyrimidyl, 4-piperidyl), which is optionally substituted by 1 to 5 substituents selected from
(i) halogen,
(ii) C₁₋₄ alkyl,
(iii) C₁₋₄ alkyl-carbonyl,
(iv) optionally halogenated C₁₋₄ alkoxy-carbonyl,
(v) C₃₋₈ cycloalkyl-carbonyl, and
(vi) a carbamoyl group optionally substituted by substituent(s) selected from
   (a) optionally halogenated C₁-₈ alkyl,
   (b) C₃₋₈ cycloalkyl, and
   (c) C₆₋₁₈ aryl optionally substituted by substituent(s) selected from halogen, C₁₋₄ alkyl and C₁₋₄ alkoxy;
      R^{1c} is (i) a hydrogen atom,

(ii) a C₂-₄ alkenyl group optionally substituted by hydroxy, or
(iii) a 5- to 8-membered heterocyclic group having 1 to 3 hetero atoms selected from a nitrogen atom, an oxygen atom and a sulfur atom;
   R^{2c} is
(i) a C₁-₄ alkyl group optionally substituted by substituent(s) selected from
   (a) halogen,
   (b) hydroxy,
   (c) C₁-₄ alkoxy,
   (d) carboxy,
   (e) C₁-₄ alkoxy-carbonyl,
   (f) -O-(CH₂)ₙ-OH,
   (g) -0- (CH₂) ₙ-O-C₁-₄ alkyl,
   (h) -CO-NR⁸- (CH₂) ₙ-OH, and
   (i) -NR⁸-CO- (CH₂ ) ₙ-SO₂-C₁-₄ alkyl
      wherein n is an integer of 1 to 4, and R⁸ is a hydrogen atom or a C₁-₄ alkyl group, or
(ii) a C₆-₁₈ aryl-C₁-₄ alkyl group optionally substituted by ₁-₄ alkyl optionally having hydroxy; and
   R^{3c} is a hydrogen atom or a C₁-₆ alkyl group; or
   R^{2c} and R^{3c} are optionally bonded to form C₂-₄ alkylene, is preferable.

Moreover, as compound (Ic), a compound wherein B^{c} is a benzene ring optionally substituted by 1 to 4 substituents selected from halogen and C₁-₄ alkyl;
C^{c} is a 5- to 8-membered heterocyclic group having 1 to 3 hetero atoms selected from a nitrogen atom, an oxygen atom and a sulfur atom, which is optionally substituted by 1 to 5 substituents selected from
(i) C₁-₄ alkyl,
(ii) C₁-₄ alkyl-carbonyl,
(iii) optionally halogenated C₁-₄ alkoxy-carbonyl,
(iv) C₃-₈ cycloalkyl-carbonyl, and
(v) a carbamoyl group optionally substituted by substituent(s) selected from
   (a) optionally halogenated C₁₋₈ alkyl,
   (b) C₃₋₈ cycloalkyl, and
   (c) C₆₋₁₈ aryl optionally substituted by halogen;
      R^{1c} is a hydrogen atom;
      R^{2c} is a C₁₋₄ alkyl group optionally substituted by substituent(s) selected from
   (a) hydroxy,
   (b) C₁-₄ alkoxy,
   (c) -O-(CH₂)ₙ-OH,
   (d) -O- (CH₂) ₙ-O-C₁-₄ alkyl, and
   (e) -NR⁸-CO- (CH₂) ₙ-SO₂-C₁₋₄ alkyl
      wherein n is an integer of 1 to 4, and R⁸ is a hydrogen atom or a C₁₋₄ alkyl group; and
      R^{3c} is a hydrogen atom or a C₁-₆ alkyl group, is preferable, particularly, a compound wherein R^{2c} is a C₁-₄ alkyl group optionally substituted by substituent(s) selected from
   (a) -O-(CH₂)ₙ-OH, and
   (b) -O-(CH₂)ₙ-O-C₁-₄ alkyl ,
wherein n is an integer of 1 to 4, is preferable.

### [Compound (Id)]

A compound represented by the formula 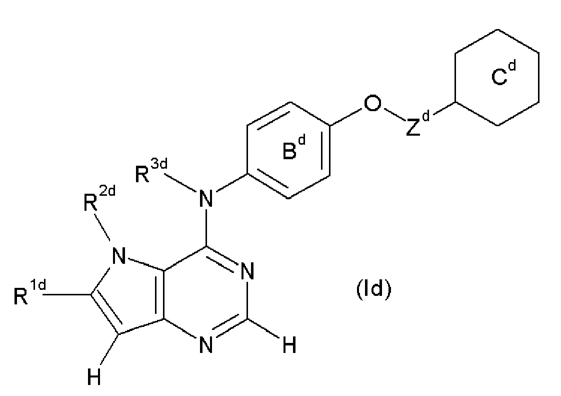 wherein R^{1d} is a hydrogen atom or an optionally substituted group bonded via a carbon atom, a nitrogen atom or an oxygen atom,
R^{2d} is an optionally substituted group bonded via a carbon atom or a sulfur atom, or
R^{1d} and R^{2d}, or R^{2d} and R^{3d} are optionally bonded to form an optionally substituted ring structure,
R^{3d} is a hydrogen atom or an optionally substituted aliphatic hydrocarbon group, or R^{3d} is optionally bonded to a carbon atom of the adjacent phenyl group to form an optionally substituted ring structure,
B^{d} is an optionally substituted benzene ring, C^{d} is an optionally substituted heterocyclic group, and z^{d} is an optionally substituted C₁-₃ alkylene group.

As the "optionally substituted group bonded via a carbon atom, a nitrogen atom or an oxygen atom" for R^{1d}, those similar to the "optionally substituted group bonded via a carbon atom, a nitrogen atom or an oxygen atom" for R¹ can be used.

As the "optionally substituted group bonded via a carbon atom or a sulfur atom" for R^{2d}, those similar to the "optionally substituted group bonded via a carbon atom or a sulfur atom" for R² can be used.

As the "optionally substituted ring structure" formed by R^{1d} and R^{2d}, or R^{2d} and R^{3d} bonded to each other, those similar to the "optionally substituted ring structure" formed by R¹ and R², or R² and R³ bonded to each other can be used.

As the "optionally substituted aliphatic hydrocarbon group" for R^{3d}, those similar to the "optionally substituted aliphatic hydrocarbon group" for R³ can be used.

As the "optionally substituted ring structure" formed by R^{3d} and a carbon atom of the adjacent phenyl group, those similar to the "optionally substituted ring structure" formed by R³ and a carbon atom of the adjacent phenyl group can be used.

As the "optionally substituted benzene ring" for B^{d} those similar to the "optionally substituted benzene ring" for B^{a} can be used.

As the "optionally substituted heterocyclic group" for C^{d}, those similar to the "optionally substituted heterocyclic group" for C^{c} can be used.

As the "optionally substituted C₁-₃ alkylene ring" for 2^{d}, those similar to the "optionally substituted C₁-₃ alkylene ring" for Z^{b} can be used.

As R^{2d}, a C₁-₈ alkyl group, a C₂₋₈ alkenyl group, a C₂₋₈ alkynyl group, a carbamoyl group, a C₁-₈ alkyl-carbonyl group, a C₁-₈ alkyl-sulfonyl group, a C₃-₈ cycloalkyl group, a C₆-₁₈ aryl group, a C₆-₁₈ aryl-C₁-₄ alkyl group, a C₆-₁₈ aryl-carbonyl group, a C₆-₁₈ aryl-C₁-₄ alkyl-carbonyl group, a C₆-₁₈ aryl-sulfonyl group, a heterocyclic group, a heterocyclyl-C₁₋₄ alkyl group, a heterocyclyl-carbonyl group or a heterocyclyl-C₁-₄ alkyl-carbonyl group, each of which is optionally substituted by 1 to 5 substituents selected from
(a) halogen,
(b) oxo,
(c) optionally halogenated C₁₋₄ alkyl,
(d) - (CH₂) ₘ-Q,
(e) - (CH₂) ₘ-Z¹- (optionally halogenated C₁-₄ alkyl) ,
(f) -(CH₂)ₘ-Z¹-C₃-₈ cycloalkyl,
(g) -(CH₂)ₘ-Z²-(CH₂)ₙ-Q,
(h) -(CH₂)m-Z²-(CH₂)ₙ-Z¹-(optionally halogenated C₁₋₄ alkyl),
(i) - (CH₂) ₘ-Z²- (CH₂) ₙ-Z¹-C₃₋₈ cycloalkyl,
(j) - (CH₂) ₘ-2¹- (optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom),
(k) -(CH₂)ₘ-2²-C₁-₄ alkoxy, and
(l) - (CH₂)ₘ-Z²- (CH₂)ₙ-Z¹- (CH₂)ₙ-Z¹-Cₗ-₄ alkyl
wherein m is an integer of 0 to 4, n is an integer of 1 to 4, Q is hydroxy, carboxy, cyano, nitro, -NR ⁶R⁷, -CONR ⁶R⁷ or -SO₂NR⁶R⁷,
Z¹ is -0-, -CO-, -C (OH) R⁸-, -C(=N-OR⁸)-, -S-, -SO-, -SO₂-, -N(COR⁸ )-, -N (CO₂R⁹) -, -N (SO₂R⁹) -, -CO-O-, -O-CO-, -CO-NR⁸-, -NR⁸-CO-, -NR⁸-CO₂-, -NR⁸-CO-NH-, -NR⁸-SO₂-, or -NR⁸-C (=NH) -NH-, z² is -0-, -CO-, -C (OH) R⁸-, -C(=N-OR⁸)-, -S-, -SO-, -SO₂-, -NR⁸-, -N(COR⁸)-, -N(CO₂R⁹)-, -N(SO₂R⁹)-, -CO-O-, -O-CO-, -CO-NR⁸-, -NR⁸-CO-, -NR⁸-CO₂-, -NR⁸-CO-NH-, -NR⁸-C (=NH) -NH-, -NR⁸-SO₂-, or -SO₂-NR⁸-,
(CH₂)ₘ and (CH₂)ₙ are optionally substituted by 1 to 5 substituents selected from halogen, optionally halogenated C₁-₄ alkyl and hydroxy, and when m or n is not less than 2, a subset -CH₂CH₂- of (CH₂)ₘ and (CH₂)ₙ is optionally replaced by -CH=CH-,
R⁶ and R⁷ are the same or different and each is a hydrogen atom, or a C₁-₄ alkyl group, or R⁶ and R⁷ are bonded to form, together with a nitrogen atom, a 3- to 8-membered saturated or unsaturated aliphatic heterocyclic group,
R⁸ is a hydrogen atom or C₁-₄ alkyl, and R⁹ is C₁-₄ alkyl, is preferable.

As compound (Id), a compound wherein
B^{d} is a benzene ring optionally substituted by halogen;
c^{d} is a 5- to 8-membered heterocyclic group having 1 to 3 hetero atoms selected from a nitrogen atom, an oxygen atom and a sulfur atom;
R^{1d} is a hydrogen atom;
R^{2d} is
(i) C₁₋₄ alkyl optionally substituted by substituent(s) selected from
   (a) C₁₋₄ alkoxy
   (b) -O-(CH₂)ₙ-OH, and
   (c) -NR⁸-CO- (CH₂)ₙ-SO₂-C₁-₄ alkyl
      wherein n is an integer of 1 to 4, and R⁸ is a hydrogen atom or a C₁-₄ alkyl group, or
(ii) a 5- to 8-membered heterocyclyl-C₁-₄ alkyl group having 1 to 3 hetero atoms selected from a nitrogen atom, an oxygen atom and a sulfur atom, which is optionally substituted by substituent(s) selected from
   (a) carboxy, and
   (b) C₁-₄ alkoxy-carbonyl;
   R^{3d} is a hydrogen atom or a C₁-₆ alkyl group; and
   2^{d} is a C₁-₃ alkylene group, is preferable.

Moreover, as compound (Id), a compound wherein
B^{d} is a benzene ring optionally substituted by halogen;
C^{d} is a 5- to 8-membered heterocyclic group having 1 to 3 hetero atoms selected from a nitrogen atom, an oxygen atom and a sulfur atom;
R^{1d} is a hydrogen atom,
R^{2d} is a C₁-₄ alkyl group optionally substituted by C₁-₄ alkoxy,
R^{3d} is a hydrogen atom or a C₁-₆ alkyl group; and
Z^{d} is a methylene group, is preferable.

### [Compound (Ie)]

A compound represented by the formula: 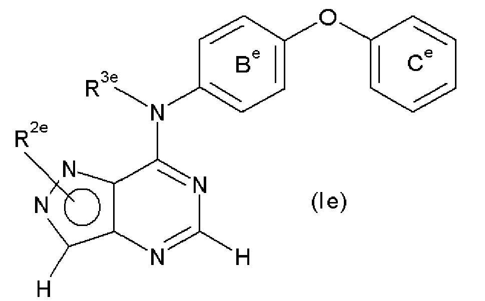 wherein R^{2e} is an optionally substituted group bonded via a carbon atom or a sulfur atom, or
R^{2e} and R^{3e} are optionally bonded to form an optionally substituted ring structure,
R^{3e} is a hydrogen atom or an optionally substituted aliphatic hydrocarbon group, or R^{3e} is optionally bonded to a carbon atom of the adjacent phenyl group to form an optionally substituted ring structure,
Be is an optionally substituted benzene ring, and C^{e} is an optionally substituted C₆-₁₈ aryl group.

As the "optionally substituted group bonded via a carbon atom or a sulfur atom" for R^{2e}, those similar to the "optionally substituted group bonded via a carbon atom or a sulfur atom" for R² can be used.

As the "optionally substituted ring structure" formed by R^{2e} and R^{3e} bonded to each other, those similar to the "optionally substituted ring structure" formed by R² and R³ bonded to each other can be used.

As the "optionally substituted aliphatic hydrocarbon group" for R^{3e}, those similar to the "optionally substituted aliphatic hydrocarbon group" for R³ can be used.

As the "optionally substituted ring structure" formed by R^{3e} and a carbon atom of the adjacent phenyl group, those similar to the "optionally substituted ring structure" formed by R³ and a carbon atom of the adjacent phenyl group can be used.

As the "optionally substituted benzene ring" for B^{e}, those similar to the "optionally substituted benzene ring" for B^{a} can be used.

As the "optionally substituted C₆-₁₈ aryl group" for C^{e}, those similar to the "optionally substituted C₆-₁₈ aryl group" for C^{a} can be used.

As R^{2e}, a C₁-₈ alkyl group, a C₂₋₈ alkenyl group, a C₂₋₈ alkynyl group, a carbamoyl group, a C₁₋₈ alkyl-carbonyl group, a C₁-₈ alkyl-sulfonyl group, a C₃-₈ cycloalkyl group, a C₆-₁₈ aryl group, a C₆₋₁₈ aryl-C₁-₄ alkyl group, a C₆-₁₈ aryl-carbonyl group, a C₆-₁₈ aryl-C₁-₄ alkyl-carbonyl group, a C₆-₁₈ aryl-sulfonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted by 1 to 5 substituents selected from
(a) halogen,
(b) oxo,
(c) optionally halogenated C₁₋₄ alkyl,
(d) - (CH₂) ₘ-Q,
(e) - (CH₂) ₘ-Z¹- (optionally halogenated C₁₋₄ alkyl) ,
(f) -(CH₂) ₘ-Z¹-C₃₋₈ cycloalkyl,
(g) -(CH₂) ₘ-Z²-(CH₂) ₙ-Q,
(h) -(CH₂) ₘ-Z²-(CH₂) ₙ-Z¹-optionally halogenated C₁₋₄ alkyl,
(i) - (CH₂) ₘ-Z²- (CH₂) ₙ-Z¹-C₃₋₈ cycloalkyl,
(j) - (CH₂) ₘ-Z¹- (optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom),
(k) -(CH₂) ₘ-Z²-C₁₋₄ alkoxy, and
(l) - (CH₂) ₘ-Z²- (CH₂) ₙ-Z¹- (CH₂) ₙ-Z¹-C₁₋₄ alkyl
wherein m is an integer of 0 to 4, n is an integer of 1 to 4, Q is hydroxy, carboxy, cyano, nitro, -NR⁶R⁷, -CONR⁶R⁷ or -SO₂NR⁶R⁷,
Z¹ is -O-, -CO-, -C (OH) R⁸-, -C (=N-OR⁸) -, -S-, -SO-, -SO₂-, -N (COR⁸) -, -N (CO₂R⁹) -, -N (SO₂R⁹) -, -CO-O-, -O-CO-, -CO-NR⁸-, -NR⁸-CO-, -NR⁸-CO₂-_{,} -NR⁸-CO-NH-, -NR⁸-SO₂-_{,} or -NR⁸-C (=NH) -NH-, Z² is -O-, -CO-, -C (OH) R⁸-, -C (=N-OR⁸) -, -S-, -SO-, -SO₂-, -NR⁸-, -N (COR⁸) -, -N (CO₂R⁹) -, -N (SO₂R⁹) -, -CO-O-, -O-CO-, -CO-NR⁸-, -NR⁸-CO-, -NR⁸-CO²-_{,} -NR⁸-CO-NH-, -NR⁸-C (=NH) -NH-, -NR⁸-SO₂-_{,} or -SO₂-NR⁸-,
(CH₂) ₘ and (CH₂) ₙ are optionally substituted by 1 to 5 substituents selected from halogen, optionally halogenated C₁₋₄ alkyl and hydroxy, and when m or n is not less than 2, a subset -CH₂CH₂- of (CH₂) ₘ and (CH₂) ₙ is optionally replaced by -CH=CH-,
R⁶ and R⁷ are the same or different and each is a hydrogen atom or a C₁₋₄ alkyl group, or R⁶ and R⁷ are bonded to form, together with a nitrogen atom, a 3- to 8-membered saturated or unsaturated aliphatic heterocyclic group,
R⁸ is a hydrogen atom or C₁₋₄ alkyl, and R⁹ is C₁₋₄ alkyl, is preferable.

As compound (Ie), a compound wherein
B^{e} is a benzene ring optionally substituted by halogen;
C^{e} is a phenyl group optionally substituted by optionally halogenated C₁₋₄ alkyl; and
R^{2e} is a C₁₋₄ alkyl group optionally substituted by -O- (CH₂) ₙ-OH wherein n is an integer of 1 to 4, is preferable.

Moreover, as compound (Ie), a compound wherein

B^{e} is a benzene ring optionally substituted by halogen;
C^{e} is a phenyl group optionally substituted by optionally halogenated C₁₋₄ alkyl; and
R^{2e} is a C₁₋₄ alkyl group substituted by -O- (CH₂) ₙ-OH wherein n is an integer of 1 to 4, is preferable.

### [Compound (If) ]

A compound represented by the formula: 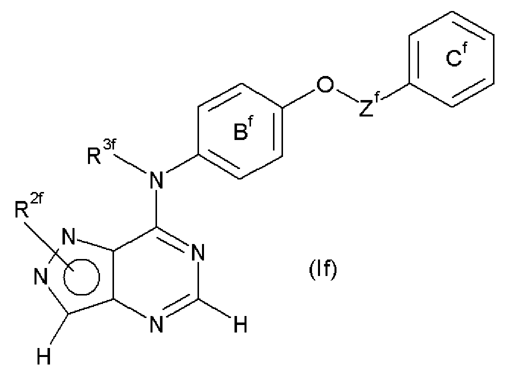 wherein R^{2f} is an optionally substituted group bonded via a carbon atom or a sulfur atom, or
R^{2f} and R^{3f} are optionally bonded to form an optionally substituted ring structure,
R^{3f} is a hydrogen atom or an optionally substituted aliphatic hydrocarbon group, or R^{3f} is optionally bonded to a carbon atom of the adjacent phenyl group to form an optionally substituted ring structure,
B^{f} is an optionally substituted benzene ring, C^{f} is an optionally substituted C₆₋₁₈ aryl group, and
Z^{f} is an optionally substituted C₁₋₃ alkylene group.

As the "optionally substituted group bonded via a carbon atom or a sulfur atom" for R^{2f}, those similar to the "optionally substituted group bonded via a carbon atom or a sulfur atom" for R² can be used.

As the "optionally substituted ring structure" formed by R^{2f} and R^{3f} bonded to each other, those similar to the "optionally substituted ring structure" formed by R² and R³ bonded to each other can be used.

As the "optionally substituted aliphatic hydrocarbon group" for R^{3f}, those similar to the "optionally substituted aliphatic hydrocarbon group" for R³ can be used.

As the "optionally substituted ring structure" formed by R^{3f} and a carbon atom of the adjacent phenyl group, those similar to the "optionally substituted ring structure" formed by R³ and a carbon atom of the adjacent phenyl group can be used.

As the "optionally substituted benzene ring" for B^{f}, those similar to the "optionally substituted benzene ring" for B^{a} can be used.

As the "optionally substituted C₆₋₁₈ aryl group" for C^{f}, those similar to the "optionally substituted C₆₋₁₈ aryl group" for C^{a} can be used.

As the "optionally substituted C₁₋₃ alkylene group" for Z^{f}, those similar to the "optionally substituted C₁₋₃ alkylene group" for Z^{b} can be used.

As R^{2f}, a C₁₋₈ alkyl group, a C₂₋₈ alkenyl group, a C₂₋₈ alkynyl group, a carbamoyl group, a C₁₋₈ alkyl-carbonyl group, a C₁₋₈ alkyl-sulfonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a C₆₋₁₈ aryl-sulfonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted by 1 to 5 substituents selected from
(a) halogen,
(b) oxo,
(c) optionally halogenated C₁₋₄ alkyl,
(d) - (CH₂) ₘ-Q,
(e) - (CH₂) ₘ-Z¹- (optionally halogenated C₁₋₄alkyl),
(f) -(CH₂)ₘ-Z¹-C₃₋₈ cycloalkyl,
(g) - (CH₂) ₘ-Z²- (CH₂) ₙ-Q
(h) -(CH₂) ₘ-Z²-(CH₂) ₙ-Z¹-(optionally halogenated C₁₋₄ alkyl),
(i) - (CH₂) ₘ-Z²- (CH₂) ₙ-Z¹-C₃₋₈ cycloalkyl,
(j) - (CH₂) ₘ-2¹- (optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom),
(k) -(CH₂) ₘ-Z²-C₁₋₄ alkoxy, and
(l) - (CH₂) ₘ-Z²- (CH₂) ₙ-Z¹- (CH₂) ₙ-Z¹-C₁₋₄ alkyl
wherein m is an integer of 0 to 4, n is an integer of 1 to 4, Q is hydroxy, carboxy, cyano, nitro, -NR⁶R⁷, -CONR⁶R⁷ or -SO₂NR⁶R⁷,
Z¹ is -O-, -CO-, -C (OH) R⁸-, -C (=N-OR⁸) -, -S-, -SO-, -SO₂-, -N (COR⁸) -, -N (CO₂R⁹) -, -N (SO₂R⁹) -, -CO-O-, -O-CO-, -CO-NR⁸-, -NR⁸-CO-, -NR⁸-CO²-_{,} -NR⁸-CO-NH-, -NR⁸-SO₂-_{,} or -NR⁸-C (=NH) -NH-, Z² is -O-, -CO-, -C (OH) R⁸-, -C (=N-OR⁸) -, -S-, -SO-, -SO₂-, -NR⁸-, -N (COR⁸)-, -N (CO₂R⁹)-, -N (SO₂R⁹) -, -CO-O-, -O-CO-, -CO-NR⁸-, -NR⁸-CO-, -NR⁸-CO₂-_{,} -NR⁸-CO-NH-, -NR⁸-C (=NH) -NH-, -NR⁸-SO₂-_{,} or -SO₂-NR⁸-,
(CH₂) ₘ and (CH₂) ₙ are optionally substituted by 1 to 5 substituents selected from halogen, optionally halogenated C₁₋₄ alkyl and hydroxy, and when m or n is not less than 2, a subset -CH₂CH₂- of (CH₂) ₘ and (CH₂) ₙ is optionally replaced by -CH=CH-,
R⁶ and R⁷ are the same or different and each is a hydrogen atom or a C₁₋₄ alkyl group, or R⁶ and R⁷ are bonded to form, together with a nitrogen atom, a 3- to 8-membered saturated or unsaturated aliphatic heterocyclic group,
R⁸ is a hydrogen atom or C₁₋₄ alkyl, and R⁹ is C₁₋₄ alkyl, is preferable.

As compound (If), a compound wherein
B^{f} is a benzene ring optionally substituted by halogen;
C^{f} is a phenyl group optionally substituted by halogen;
R^{2f} is
(i) a C₁₋₄ alkyl group optionally substituted by 1 to 5 substituents selected from the group consisting of
   (a) hydroxy,
   (b) -O- (CH₂) ₙ-OH,
   (c) -NR⁸-(CH₂) ₙ-O-C₁₋₄ alkyl,
   (d) -NR⁸- (CH₂) ₙ-heterocyclic group (preferably, said heterocyclic group is a 5- to 8-membered heterocyclic group having 1 to 3 hetero atoms selected from a nitrogen atom, an oxygen atom and a sulfur atom), and
   (e) -NR⁸-(CH₂) ₙ-SO₂-C₁₋₄ alkyl,
      wherein n is an integer of 1 to 4, and R⁸ is a hydrogen atom or a C₁₋₄ alkyl group,
(ii) a C₆₋₁₈ aryl group optionally substituted by 1 to 5 substituents selected from the group consisting of
   (a) C₁₋₄alkyl optionally substituted by substituent(s) selected from hydroxy, -NR⁸- (CH₂) ₙ-OH, -NR⁸-(CH₂) ₙ-O-C₁₋₄ alkyl, -NR⁸- (CH₂) ₙ-heterocyclic group (preferably, said heterocyclic group is a 5- to 8-membered heterocyclic group having 1 to 3 hetero atoms selected from a nitrogen atom, an oxygen atom and a sulfur atom) and -NR⁸- (CH₂) ₙ-SO₂-C₁₋₄ alkyl, and
   (b) -CO-NR⁸-(CH₂) ₙ-O-C₁₋₄ alkyl,
      wherein n is an integer of 1 to 4, and R⁸ is a hydrogen atom or a C₁₋₄ alkyl group, or
(iii) a C₆₋₁₈ aryl-C₁₋₄ alkyl group optionally substituted by 1 to 5 substituents selected from the group consisting of
   (a) carboxy,
   (b) C₁₋₄ alkoxy-carbonyl, and
   (c) -CO-NR⁸- (CH₂) ₙ-O-C₁₋₄ alkyl,
wherein n is an integer of 1 to 4, and R⁸ is a hydrogen atom or a C₁₋₄ alkyl group,
R^{3f} is a hydrogen atom or a C₁₋₆ alkyl group; and
Z^{f} is a C₁₋₃ alkylene group; or
R^{2f} and R^{3f} are optionally bonded to form C₂₋₄ alkylene, is preferable.

As R⁸, a hydrogen atom, methyl, ethyl and the like are preferably, and a hydrogen atom is particularly preferable.

Moreover, as compound (If), a compound wherein
B^{f} is a benzene ring optionally substituted by halogen;
C^{f} is a phenyl group optionally substituted by halogen;
R^{2f} is a C₁₋₄ alkyl group optionally substituted by 1 to 5 substituents selected from the group consisting of
(a) hydroxy, and
(b) -O- (CH₂) ₙ-OH wherein n is an integer of 1 to 4;
R^{3f} is a hydrogen atom or a C₁₋₆ alkyl group;
Z^{f} is methylene, is preferable, and particularly, a compound wherein R^{2f} is a C₁₋₄ alkyl group substituted by -O-(CH₂) ₙ-OH wherein n is an integer of 1 to 4, is preferable.

### [Compound (Ig) ]

A compound represented by the formula: 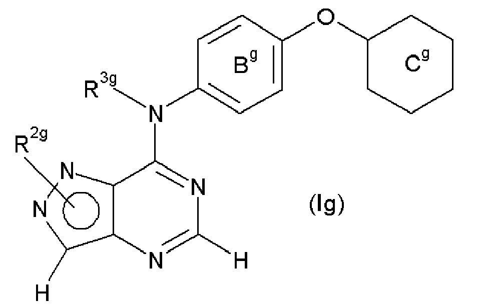 wherein R^{2g} is an optionally substituted group bonded via a carbon atom or a sulfur atom, or
R^{2g} and R^{3g} are optionally bonded to form an optionally substituted ring structure,
R^{3g} is a hydrogen atom or an optionally substituted aliphatic hydrocarbon group, or R^{3g} is optionally bonded to a carbon atom of the adjacent phenyl group to form an optionally substituted ring structure,
B^{g} is an optionally substituted benzene ring, and C^{g} is an optionally substituted heterocyclic group.

As the "optionally substituted group bonded via a carbon atom or a sulfur atom" for R^{2g}, those similar to the "optionally substituted group bonded via a carbon atom or a sulfur atom" for R² can be used.

As the "optionally substituted ring structure" formed by R^{2g} and R^{3g} bonded to each other, those similar to the "optionally substituted ring structure" formed by R² and R³ bonded to each other can be used.

As the "optionally substituted aliphatic hydrocarbon group" for R^{3g}, those similar to the "optionally substituted aliphatic hydrocarbon group" for R³ can be used.

As the "optionally substituted ring structure" formed by R^{3g} and a carbon atom of the adjacent phenyl group, those similar to the "optionally substituted ring structure" formed by R³ and a carbon atom of the adjacent phenyl group can be used.

As the "optionally substituted benzene ring" for B^{g}, those similar to the "optionally substituted benzene ring" for B^{a} can be used.

As the "optionally substituted heterocyclic group" for C^{g}, those similar to the "optionally substituted heterocyclic group" for C^{c} can be used.

As R^{2g}, a C₁₋₈ alkyl group, a C₂₋₈ alkenyl group, a C₂₋₈ alkynyl group, a carbamoyl group, a C₁₋₈ alkyl-carbonyl group, a C₁₋₈ alkyl-sulfonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a C₆₋₁₈ aryl-sulfonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted by 1 to 5 substituents selected from the group consisting of
(a) halogen,
(b) oxo,
(c) optionally halogenated C₁₋₄ alkyl,
(d) - (CH₂) ₘ-Q,
(e) -(CH₂) ₘ-Z¹-(optionally halogenated C₁₋₄ alkyl),
(f) -(CH₂) ₘ-Z¹-C₃₋₈ cycloalkyl,
(g) -(CH₂) m-Z²-(CH₂) ₙ-Q,
(h) -(CH²) ₘ-Z²-(CH₂) ₙ-Z¹-(optionally halogenated C₁₋₄ alkyl),
(i) - (CH₂) ₘ-Z²- (CH₂) ₙ-Z¹-C₃₋₈ cycloalkyl,
(j) - (CH₂) ₘ-Z¹- (optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom),
(k) -(CH₂) ₘ-Z²-C₁₋₄ alkoxy, and
(l) - (CH₂) ₘ-Z²- (CH₂) ₙ-Z¹- (CH₂) ₙ-Z¹-C₁₋₄ alkyl
wherein m is an integer of 0 to 4, n is an integer of 1 to 4,
Q is hydroxy, carboxy, cyano, nitro, -NR⁶R⁷, -CONR⁶R⁷ or -SO₂NR⁶R⁷,
Z¹ is -O-, -CO-, -C (OH) R⁸-, -C (=N-OR⁸) -, -S-, -SO-, -SO₂-, -N (COR⁸)-, -N (CO₂R⁹) -, -N (SO₂R⁹) -, -CO-O-, -O-CO-, -CO-NR⁸-, -NR⁸-CO-, -NR⁸-CO₂-_{,} -NR⁸-CO-NH-, -NR⁸-SO₂-_{,} or -NR⁸-C (=NH) -NH-, Z² is -O-, -CO-, -C (OH) R⁸-, -C (=N-OR⁸) -, -S-, -SO-, -SO₂-, -NR⁸-, -N COR⁸)-, -N (CO₂R₉)-, -N (SO₂R₉) -, -CO-O-, -O-CO-, -CO-NR⁸-, -NR⁸-CO-, -NR⁸-CO₂-_{,} -NR⁸-CO-NH-, -NR⁸-C (=NH) -NH-, -NR⁸-SO₂-_{,} or -SO₂-NR⁸-,
(CH₂) ₘ and (CH₂) ₙ are optionally substituted by 1 to 5 substituents selected from halogen, optionally halogenated C₁₋₄ alkyl and hydroxy, and when m or n is not less than 2, a subset -CH₂CH₂- of (CH₂) ₘ and (CH₂) ₙ is optionally replaced by -CH=CH-.
R⁶ and R⁷ are the same or different and each is a hydrogen atom or a C₁₋₄ alkyl group, or R⁶ and R⁷ are bonded to form, together with a nitrogen atom, a 3- to 8-membered saturated or unsaturated aliphatic heterocyclic group,
R⁸ is a hydrogen atom or C₁₋₄ alkyl, and R⁹ is C₁₋₄ alkyl, is preferable.

As compound (Ig), a compound wherein
B^{g} is a benzene ring optionally substituted by C₁₋₄ alkyl;
C^{g} is a 5- to 8-membered heterocyclic group having 1 to 3 hetero atoms selected from a nitrogen atom, an oxygen atom and a sulfur atom, which is optionally substituted by C₁₋₄ alkyl; R^{2g} is
(i) a C₁₋₄ alkyl group optionally substituted by hydroxy,
(ii) a C₆₋₁₈ aryl group optionally substituted by substituent(s) selected from
   (a) nitro,
   (b) amino,
   (c) -CO-NR⁸- (CH₂) ₙ-O-C₁₋₄ alkyl,
   (d) -NR⁸-CO- (CH₂) ₙ-O-C₁₋₄ alkyl,
   (e) -NR⁸-CO- (CH₂) ₙ-NR⁶R⁷,
   (f) -NR⁸-CO- (CH₂) ₙ-COOH,
   (g) -NR⁸-CO- (CH₂) ₙ-CO₂-C₁₋₄ alkyl, and
   (h) -NR⁸-CO- (CH₂) ₘ-O- (CH₂) ₙ-O-C₁₋₄ alkyl,
      wherein m is an integer of 0 to 4, n is an integer of 1 to 4, R⁶ and R⁷ are the same or different and each is a hydrogen atom or a C₁₋₄ alkyl group, and R⁸ is a hydrogen atom or a C₁₋₄ alkyl group, or
(iii) a C₆₋₁₈ aryl-C₁₋₄ alkyl group optionally substituted by substituent(s) selected from
   (a) carboxy,
   (b) C₁₋₄ alkoxy-carbonyl, and
   (c) -CO-NR⁸-(CH₂) ₙ-O-C₁₋₄ alkyl,
wherein n is an integer of 1 to 4, and R⁸ is a hydrogen atom or a C₁₋₄ alkyl group;
R^{3g} is a hydrogen atom or a C₁₋₄ alkyl group; or
R^{2g} and R^{3g} are optionally bonded to form C₂₋₄ alkylene, is preferable.

As compound (Ig), a compound wherein R^{2g} is
(i) a C₆₋₁₈ aryl group optionally substituted by substituent(s) selected from
   (a) nitro,
   (b) amino,
   (c) -CO-NR⁸- (CH₂) ₙ-O-C₁₋₄ alkyl,
   (d) -NR⁸-CO- (CH₂) ₙ-O-C₁₋₄ alkyl,
   (e) -NR⁸-CO- (CH₂) ₙ-NR⁶R⁷,
   (f) -NR⁸-CO- (CH₂) ₙ-COOH,
   (g) -NR⁸-CO- (CH₂) ₙ-CO₂-C₁₋₄ alkyl, and
   (h) -NR⁸-CO- (CH₂) ₘ-O- (CH₂) ₙ-O-C₁₋₄ alkyl,
      wherein m is an integer of 0 to 4, n is an integer of 1 to 4, R⁶ and R⁷ are the same or different and each is a hydrogen atom or a C₁₋₄ alkyl group, and R⁸ is a hydrogen atom or a C₁₋₄ alkyl group, or
(ii) a C₆₋₁₈ aryl-C₁₋₄ alkyl group substituted by substituent(s) selected from
   (a) carboxy,
   (b) C₁₋₄ alkoxy-carbonyl, and
   (c) -CO-NR⁸-(CH₂) ₙ-O-C₁₋₄ alkyl,
wherein n is an integer of 1 to 4, and R⁸ is a hydrogen atom or a C₁₋₄ alkyl group, is preferable.

As R⁸, a hydrogen atom, methyl, ethyl and the like are preferable, and a hydrogen atom is particularly preferable.

### [Compound (Ih) ]

A compound (I) selected from the following (A) to (H).
(A) A compound (I) wherein W is C (R¹) ;
   A is a phenoxy-C₆₋₁₈ aryl group wherein the phenyloxy moiety is optionally substituted by 1 to 5 substituents selected from
   (i) halogen,
   (ii) optionally halogenated C₁₋₄ alkyl,
   (iii) hydroxy-C₁₋₄ alkyl,
   (iv) heterocyclyl-C₁₋₄ alkyl (preferably, 5- to 8-membered heterocyclyl-C₁₋₄ alkyl, said 5- to 8-membered heterocycle has 1 to 3 hetero atoms selected from a nitrogen atom, an oxygen atom and an optionally oxidized sulfur atom, such as imidazolyl, triazolyl and the like),
   (v) optionally halogenated C₁₋₄ alkoxy,
   (vi) C₁₋₄ alkyl-carbonyl,
   (vii) cyano,
   (viii) carbamoyl optionally substituted by C₁₋₈ alkyl, and
   (ix) C₁₋₄ alkoxy-carbonyl, and
   the C₆₋₁₈ aryl moiety is optionally further substituted by 1 to 4 substituents selected from halogen, C₁₋₄ alkyl, hydroxy-C₁₋₄ alkyl, C₁₋₄ alkoxy, carboxy and C₁₋₄ alkoxy-carbonyl;
   X¹ is -NR^{3'} - wherein R³' is a hydrogen atom or a C₁₋₆ alkyl group; R¹ is
   (i) a hydrogen atom,
   (ii) a cyano group, or
   (iii) a C₁₋₄ alkyl group or a C₂₋₄ alkenyl group, each of which is optionally substituted by -NR⁸-CO-(CH₂) ₙ-NR⁶R⁷ wherein n is an integer of 1 to 4, R⁶ and R⁷ are the same or different and each is a hydrogen atom or a C₁₋₄ alkyl group, R⁸ is a hydrogen atom or a C₁₋₄ alkyl group, and when n is not less than 2, a subset -CH₂CH₂- of (CH₂) ₙ is optionally replaced by - CH=CH-;
   R² is (i) a hydrogen atom or
   (ii) a C₁₋₈ kyl group, a C₂₋₈ alkenyl group or a C₂₋₈ alkynyl group, each of which is optionally substituted by substituent(s) selected from
      (a) hydroxy,
      (b) carboxy,
      (c) cyano,
      (d) optionally halogenated C₁₋₄ alkoxy,
      (e) -O-(CH₂) ₙ-OH,
      (f) -0- (CH₂) ₙ-O-CO-NH₂,
      (g) -O- (CH₂) ₙ-O- (optionally halogenated C₁₋₄ alkyl) ,
      (h) -O- (CH₂) ₙ-SO₂- (optionally halogenated C₁₋₄ alkyl) ,
      (i) -O- (CH₂) ₙ-SO₂-C₆₋₁₈ aryl,
      (j) -O- (CH₂) ₙ-SO₂- (CH₂) ₙ-OH,
      (k) -O- (CH₂) ₙ-NR⁸ -CO-C₁₋₄ alkyl,
      (l) -O- (CH₂) ₙ-NR⁸-CO- (CH₂) ₙ-SO₂-C₁₋₄ alkyl,
      (m) -O- (CH₂) ₙ-NR⁸-SO₂- (optionally halogenated C₁₋₄ alkyl),
      (n) -CO-NR⁸- (CH₂) ₙ-OH,
      (o) -CO-NR⁸- (CH₂) ₙ-SO₂- (optionally halogenated C₁₋₄ alkyl),
      (p) -CO-NR⁸-O-C₁₋₄ alkyl,
      (q) -NR⁶R⁷,
      (r) -NR⁸- (CH₂) ₙ-OH,
      (s) -NR⁸-(CH₂) n-SO₂-C₁₋₄ al kyl ,
      (t) -NR⁸-CO- (optionally halogenated C₁₋₄ alkyl) ,
      (u) -NR⁸-CO- (CH₂) ₙ-OH,
      (v) -NR⁸-CO- (CH₂) ₙ-CN,
      (w) -NR⁸-CO- (CH₂) ₙ-NR⁶R⁷,
      (x) -NR⁸-CO- (CH₂) ₙ-O-C₁₋₄ alkyl,
      (y) -NR⁸-CO- (CH₂) ₙ-SO- (optionally halogenated C₁₋₄ alkyl),
      (z) -NR⁸-CO- (CH₂) ₙ-SO₂- (optionally halogenated C₁₋₄ alkyl),

      (aa) -NR⁸-CO- (CH₂) ₙ-SO₂-C₃₋₈ cycloalkyl,
      (bb) -NR⁸-CO- (CH₂) ₙ-NR⁸-SO₂-C₁₋₄ alkyl,
      (cc) -NR⁸-CO₂- (CH₂) ₙ-SO₂-C₁₋₄ alkyl,
      (dd) -NR⁸-CO-NH- (CH₂) ₙ-SO₂-C₁₋₄ alkyl,
      (ee) -NR⁸-CO-NH-O-C₁₋₄ alkyl,
      (ff) -NR⁸-CO-NH- (CH₂) ₙ-O-C₁₋₄ alkyl,
      (gg) -NR⁸-C (=NH) -NH-C₁₋₄ alkyl,
      (hh) -NR⁸-SO₂- (CH₂) ₙ-SO₂-C₁₋₄ alkyl,
      (ii) -S- (CH₂) ₙ-OH,
      (jj) -SO- (CH₂) ₙ-OH,
      (kk) -SO₂- (CH₂) ₙ-OH, and
      (11) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-O-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
   wherein n is an integer of 1 to 4, R⁶ and R⁷ are the same or different and each is a hydrogen atom or a C₁₋₄ alkyl group, R⁸ is a hydrogen atom or a C₁₋₄ alkyl group, (CH₂) ₙ is optionally substituted by optionally halogenated C₁₋₄ alkyl or hydroxy, and when n is not less than 2, and a subset -CH₂CH₂- of (CH₂) ₙ is optionally replaced by -CH=CH-; or
   R¹ and R² are optionally bonded to form 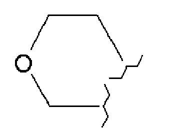 R² and R ³ are optionally bonded to form C₂₋₄ alkylene optionally substituted by an imino group,
   particularly preferably, R^{2a} is a C₁₋₈ alkyl group, a C₂₋₈ alkenyl group or a C₂₋₈ alkynyl group (particularly, a C₁₋₈ alkyl group), each of which is optionally substituted by substituent(s) selected from
   (a) hydroxy,
   (b) carboxy,
   (c) cyano,
   (d) optionally halogenated C₁₋₄ alkoxy,
   (e) -O-(CH₂) ₙ-OH (wherein (CH₂) ₙ is optionally substituted by hydroxy),
   (f) -O- (CH₂) ₙ-O-CO-NH₂,
   (g) -O- (CH₂) ₙ-O- (optionally halogenated C₁₋₄ alkyl) ,
   (h) -O- (CH₂) ₙ-SO₂- (optionally halogenated C₁₋₄ alkyl) ,
   (i) -O- (CH₂) ₙ-SO₂-C₆₋₁₈ aryl,
   (j) -O- (CH₂) ₙ-SO₂- (CH₂) ₙ-OH,
   (k) -O- (CH₂) ₙ-NR⁸-CO-C₁₋₄ alkyl,
   (l) -O- (CH₂) ₙ-NR⁸ -CO- (CH₂) ₙ-SO₂-C₁₋₄ alkyl,
   (m) -O- (CH₂) ₙ-NR⁸-SO₂- (optionally halogenated C₁₋₄ alkyl),
   (n) -CO-NR⁸- (CH₂) ₙ-OH,
   (o) -CO-NR⁸- (CH₂) ₙ-SO₂- (optionally halogenated C₁₋₄ alkyl),
   (p) -CO-NR⁸-O-C₁₋₄ alkyl,
   (q) -NR⁶R⁷,
   (r) -NR⁸- (CH₂) ₙ-OH,
   (s) -NR⁸- (CH₂) ₙ-SO₂-C₁₋₄ alkyl,
   (t) -NR⁸-CO- (optionally halogenated C₁₋₄ alkyl) ,
   (u) -NR⁸-CO- (CH₂) ₙ-OH (wherein (CH₂) ₙ is optionally substituted by optionally halogenated C₁₋₄ alkyl or hydroxy),
   (v) -NR⁸-CO- (CH₂) ₙ-CN,
   (w) -NR⁸-CO- (CH₂) ₙ-NR⁶R⁷ (when n is not less than 2, a subset -CH₂CH₂- of (CH₂) ₙ is optionally replaced by -CH=CH-),
   (x) -NR⁸-CO- (CH₂) ₙ-O-C₁₋₄ alkyl,
   (y) -NR⁸-CO- (CH₂) ₙ-SO- (optionally halogenated C₁₋₄ alkyl),
   (z) -NR⁸-CO- (CH₂) ₙ-SO₂- (optionally halogenated C₁₋₄ alkyl) (wherein (CH₂) ₙ is optionally substituted by C₁₋₄ alkyl),

   (aa) -NR⁸-CO- (CH₂) ₙ-SO₂-C₃₋₈ cycloalkyl,
   (bb) -NR⁸-CO- (CH₂) ₙ-NR⁸- SO₂-C₁₋₄ alkyl,
   (cc) -NR⁸-CO₂- (CH₂) ₙ-SO₂-C₁₋₄ alkyl,
   (dd) -NR⁸-CO-NH- (CH₂) ₙ-SO₂-C₁₋₄ alkyl,
   (ee) -NR⁸-CO-NH-O-C₁₋₄ alkyl,
   (ff) -NR⁸-CO-NH-(CH₂) ₙ-O-C₁₋₄ alkyl,
   (gg) -NR⁸-C (=NH) -NH-C₁₋₄ alkyl,
   (hh) -NR⁸-SO₂- (CH₂) ₙ-SO₂-C₁₋₄ alkyl,
   (ii) -S-(CH₂) ₙ-OH,
   (jj) -SO- (CH₂) ₙ-OH,
   (kk) -SO₂- (CH₂) ₙ-OH, and
   (11) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-O-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
   wherein n is an integer of 1 to 4, R⁶ and R⁷ are the same or different and each is a hydrogen atom or a C₁₋₄ alkyl group, and R⁸ is a hydrogen atom or a C₁₋₄ alkyl group.
(B) A compound (I) wherein W is C(R¹) ;
   A is a phenyl-C₁₋₃ alkoxy-C₆₋₁₈ aryl group wherein the phenyl moiety is optionally substituted by 1 to 5 substituents selected from halogen, optionally halogenated C₁₋₄ alkyl and cyano, and
   the C₆₋₁₈ aryl moiety is optionally further substituted by 1 to 4 substituents selected from halogen, C₁₋₄ alkyl optionally having hydroxy and C₁₋₄ alkoxy;
   X¹ is -NR^{3'} - wherein R^{3'} is a hydrogen atom or a C₁₋₆ alkyl group;
   R¹ is (i) a hydrogen atom, or
   (ii) a C₁₋₄ alkyl group or a C₂₋₄ alkenyl group, each of which is optionally substituted by substituent(s) selected from
      (a) hydroxy,
      (b) amino, and
      (c) -NR⁸-CO-(CH₂) ₙ-NR⁶R⁷,
         wherein n is an integer of 1 to 4, R⁶ and R⁷ are the same or different and each is a hydrogen atom or a C₁₋₄ alkyl group, and R⁸ is a hydrogen atom or a C₁₋₄ alkyl group,
   (iii) a C₆₋₁₈ aryl group optionally substituted by substituent(s) selected from
      (a) amino,
      (b) carboxy,
      (c) -NR⁸-CO- (CH₂) ₙ-O-C₁₋₄ alkyl, and
      (d) -NR⁸-CO- (CH₂) ₙ-O-C₁₋₄ alkyl,
         wherein n is an integer of 1 to 4, and R⁸ is a hydrogen atom or a C₁₋₄ alkyl group, and when n is not less than 2, a subset -CH₂-CH₂- of (CH₂) ₙ is optionally replaced by -CH=CH-, or
   (iv) a 5- to 8-membered heterocyclic group having 1 to 3 hetero atoms selected from a nitrogen atom, an oxygen atom and a sulfur atom;
      R² is (i) a hydrogen atom,
   (ii) a C₁₋₈ alkyl group optionally substituted by substituent(s) selected from
      (a) halogen,
      (b) hydroxy,
      (c) C₁₋₄ alkoxy,
      (d) -O- (CH₂) ₙ-OH,
      (e) -O- (CH₂) ₙ-O-C₁₋₄ alkyl,
      (f) -CO-NR⁸- (CH₂) ₙ-OH,
      (g) -NR⁶R⁷, and
      (h) -NR⁸- (CH₂) ₙ-OH,
      wherein n is an integer of 1 to 4, R⁶ and R⁷ are the same or different and each is a hydrogen atom or a C₁₋₄ alkyl group, and R⁸ is a hydrogen atom or a C₁₋₄ alkyl group,
   (iii) a C₆₋₁₈ aryl-C₁₋₄ alkyl group optionally substituted by substituent(s) selected from
      (a) C₁₋₄ alkyl optionally having hydroxy,
      (b) carboxy,
      (c) C₁₋₄ alkoxy-carbonyl,
      (d) 5- to 8-membered heterocyclyl-carbonyl having 1 to 3 hetero atoms selected from a nitrogen atom, an oxygen atom and a sulfur atom, which optionally has substituent(s) selected from hydroxy and C₁₋₄ alkyl, and
      (e) C₁₋₄ alkyl-carbamoyl optionally having substituent(s) selected from hydroxy and carbamoyl,
   (iv) a C₆₋₁₈ aryl-carbonyl group optionally substituted by C₁₋₄ alkoxy,
   (v) a C₆₋₁₈ aryl-sulfonyl group optionally substituted by C₁₋₄ alkoxy, or
   (vi) a 5- to 8-membered heterocyclyl-C₁₋₄ alkyl group having 1 to 3 hetero atoms selected from a nitrogen atom, an oxygen atom and a sulfur atom, which is optionally substituted by substituent(s) selected from
      (a) carboxy, and
      (b) C₁₋₄ alkoxy-carbonyl; or
   R² and R³ are optionally bonded to form C₂₋₄ alkylene.
(C) A compound (I) wherein W is C (R¹) ;
   A is a 5- to 8-membered heterocycleoxy-C₆₋₁₈ aryl group containing 1 to 3 hetero atoms selected from a nitrogen atom, an oxygen atom and a sulfur atom, wherein the heterocycleoxy moiety is optionally substituted by 1 to 5 substituents selected from
   (i) halogen,
   (ii) C₁₋₄ alkyl,
   (iii) C₁₋₄ lkyl-carbonyl,
   (iv) optionally halogenated C₁₋₄ alkoxy-carbonyl,
   (v) C₃₋₈ cycloalkyl-carbonyl, and
   (vi) a carbamoyl group optionally substituted by substituent(s) selected from
      (a) optionally halogenated C₁₋₈ alkyl,
      (b) C₃₋₈ cycloalkyl, and
      (c) C₆₋₁₈ aryl optionally substituted by substituent(s) selected from halogen, C₁₋₄ alkyl and C₁₋₄ alkoxy, and
         the C₆₋₁₈ aryl moiety is optionally further substituted by 1 to 4 substituents selected from halogen and optionally halogenated C₁₋₄ alkyl;
         X¹ is -NR^{3'} - wherein R^{3'} is a hydrogen atom or a C₁₋₆ alkyl group; R¹ is (i) a hydrogen atom,
   (ii) a C₁₋₄ alkyl group or a C₂₋₄ alkenyl group, each of which is optionally substituted by substituent(s) selected from
      (a) hydroxy,
      (b) amino,
      (c) -NR⁸-CO- (CH₂) ₙ-NR⁶R⁷, and
      (d) -NR⁸-CO- (CH₂) ₙ-O-C₁₋₄ alkyl,
         wherein n is an integer of 1 to 4, R⁶ and R⁷ are the same or different and each is a hydrogen atom or a C₁₋₄ alkyl group, R⁸ is a hydrogen atom or a C₁₋₄ alkyl group, and when n is not less than 2, a subset -CH₂CH₂- of (CH₂) ₙ is optionally replaced by -CH=CH-,
   (iii) a C₆₋₁₈ aryl group optionally substituted by substituent(s) selected from
      (a) C₁₋₄ alkyl optionally substituted by substituent(s) selected from hydroxy, -NR⁸- (CH₂) ₙ-SO₂-C₁₋₄ alkyl and -NR⁸-CO- (CH₂) ₙ-O-C₁₋₄ alkyl,
      (b) amino,
      (c) C₁₋₄ alkoxy,
      (d) carboxy, and
      (e) -NR⁸-CO- (CH₂) ₙ-O-C₁₋₄ alkyl,
         wherein n is an integer of 1 to 4, and R⁸ is a hydrogen atom or a C₁₋₄ alkyl group, or
   (iv) a 5- to 8-membered heterocyclic group having 1 to 3 hetero atoms selected from a nitrogen atom, an oxygen atom and a sulfur atom;
      R² is (i) a hydrogen atom,
   (ii) a C₁₋₄ alkyl group optionally substituted by substituent(s) selected from
      (a) halogen,
      (b) hydroxy,
      (c) C₁₋₄ alkoxy,
      (d) carboxy,
      (e) C₁₋₄ alkoxy-carbonyl,
      (f) -O- (CH₂) ₙ-OH,
      (g) -O- (CH₂) ₙ-O-C₁₋₄ alkyl,
      (h) -CO-NR⁸- (CH₂) ₙ-OH, and
      (i) -NR⁸-CO- (CH₂) ₙ-SO₂-C₁₋₄ alkyl
      wherein n is an integer of 1 to 4, and R⁸ is a hydrogen atom or a C₁₋₄ alkyl group, or
   (iii) a C₆₋₁₈ aryl-C₁₋₄ alkyl group optionally substituted by C₁₋₄ alkyl optionally having hydroxy; or
   R² and R^{3'} are optionally bonded to form C₂₋₄ alkylene.
(D) A compound (I) wherein W is C (R¹) ;
   A is 5- to 8-membered heterocyclyl-C₁₋₃ alkoxy-C₆₋₁₈ aryl group containing 1 to 3 hetero atoms selected from a nitrogen atom, an oxygen atom and a sulfur atom;
   wherein the C₆₋₁₈ aryl moiety is optionally further substituted by halogen;
   X¹ is -NR^{3'}_ wherein R^{3'} is a hydrogen atom or a C₁₋₆ alkyl group;
   R¹ is (i) a hydrogen atom or
   (ii) a 5- to 8-membered heterocyclic group having 1 to 3 hetero atoms selected from a nitrogen atom, an oxygen atom and a sulfur atom;
      R² is (i) a hydrogen atom,
   (ii) C₁₋₄ alkyl optionally substituted by substituent(s) selected from
      (a) C₁₋₄ alkoxy,
      (b) -O-(CH₂) ₙ-OH, and
      (c) -NR⁸-CO- (CH₂) ₙ-SO₂-C₁₋₄ alkyl,
         wherein n is an integer of 1 to 4, and R⁸ is a hydrogen atom or a C₁₋₄ alkyl group, or
   (iii) a 5- to 8-membered heterocyclyl-C₁₋₄ alkyl group having 1 to 3 hetero atoms selected from a nitrogen atom, an oxygen atom and a sulfur atom, which is optionally substituted by substituent(s) selected from
      (a) carboxy, and
      (b) C₁₋₄ alkoxy-carbonyl.
(E) A compound (I) wherein W is N;
   A is a phenoxy-C₆₋₁₈ aryl group wherein the phenyloxy moiety is optionally substituted by 1 to 5 substituents selected from optionally halogenated C₁₋₄ alkyl and cyano, and
   the C₆₋₁₈ aryl moiety is optionally further substituted by 1 to 4 substituents selected from halogen and C₁₋₄ alkyl;
   X¹ is -NR^{3'} - wherein R^{3'} is a hydrogen atom or a C₁₋₆ alkyl group;
   R² is (i) a hydrogen atom or
   (ii) a C₁₋₄ alkyl group optionally substituted by -O- (CH₂) ₙ-OH wherein n is an integer of 1 to 4.
(F) A compound (I) wherein W is N;
   A is a phenyl-C₁₋₃ alkoxy-C₆₋₁₈ aryl group wherein the phenyl moiety is optionally substituted by 1 to 5 substituents selected from halogen and cyano, and
   the C₆₋₁₈ aryl moiety is optionally further substituted by 1 to 5 substituents selected from halogen and C₁₋₄ alkyl;
   X¹ is -NR^{3'} - wherein R^{3'} is a hydrogen atom or a C₁₋₆ alkyl group;
   R² is (i) a hydrogen atom,
   (ii) a C₁₋₄ alkyl group optionally substituted by 1 to 5 substituents selected from the group consisting of
      (a) hydroxy,
      (b) -O- (CH₂) ₙ-OH,
      (c) -NR⁸- (CH₂) ₙ-O-C₁₋₄ alkyl,
      (d) -NR⁸- (CH₂) ₙ-heterocyclic group (preferably, said heterocyclic group is a 5- to 8-membered heterocyclic group having 1 to 3 hetero atoms selected from a nitrogen atom, an oxygen atom and a sulfur atom), and
      (e) -NR⁸- (CH₂) ₙ-SO₂-C₁₋₄ alkyl,
         wherein n is an integer of 1 to 4, and R⁸ is a hydrogen atom or a C₁₋₄ alkyl group,
   (iii) a C₆₋₁₈ aryl group optionally substituted by C₁₋₄ alkyl optionally substituted by substituent(s) selected from hydroxy, -NR⁸-(CH₂) ₙ-OH, -NR⁸- (CH₂) ₙ-heterocyclic group (preferably, said heterocyclic group is a 5- to 8-membered heterocyclic group having 1 to 3 hetero atoms selected from a nitrogen atom, an oxygen atom and a sulfur atom) and -NR⁸- (CH₂) ₙ-SO₂-C₁₋₄ alkyl, wherein n is an integer of 1 to 4 and R⁸ is a hydrogen atom or a C₁₋₄ alkyl group, or
   (iv) a C₆₋₁₈ aryl-C₁₋₄ alkyl group optionally substituted by 1 to 5 substituents selected from the group consisting of
      (a) carboxy,
      (b) C₁₋₄ alkoxy-carbonyl, and
      (c) -CO-NR⁸- (CH₂) ₙ-O-C₁₋₄ alkyl,
   wherein n is an integer of 1 to 4, and R⁸ is a hydrogen atom or a C₁₋₄ alkyl group; or
   R² and R³ are optionally bonded to form C₂₋₄ alkylene.
(G) A compound (I) wherein W is N;
   A is a 5- to 8-membered heterocycleoxy-C₆₋₁₈ aryl group containing 1 to 3 hetero atoms selected from a nitrogen atom, an oxygen atom and a sulfur atom, wherein the heterocycleoxy moiety is optionally substituted by C₁₋₄ alkyl, and the C₆₋₁₈ aryl moiety is optionally further substituted by C₁₋₄ alkyl;
   X¹ is -NR^{3'} - wherein R^{3'} is a hydrogen atom or a C₁₋₆ alkyl group;
   R² is (i) a hydrogen atom,
   (ii) a C₁₋₄ alkyl group optionally substituted by hydroxy,
   (iii) a C₆₋₁₈ aryl group optionally substituted by substituent(s) selected from
      (a) nitro,
      (b) amino,
      (c) -CO-NR⁸- (CH₂) ₙ-O-C₁₋₄ alkyl,
      (d) -NR⁸-CO- (CH₂) ₙ-O-C₁₋₄ alkyl,
      (e) -NR⁸-CO- (CH₂) -NR⁶R⁷
      (f) -NR⁸-CO- (CH₂) ₙ-COOH,
      (g) -NR⁸-CO- (CH₂) ₙ-CO₂-C₁₋₄ alkyl, and
      (h) -NR⁸-CO- (CH₂) ₘ-O- (CH₂) ₙ-O-C₁₋₄ alkyl,
         wherein m is an integer of 0 to 4, n is an integer of 1 to 4, R⁶ and R⁷ are the same or different and each is a hydrogen atom or a C₁₋₄ alkyl group, and R⁸ is a hydrogen atom or a C₁₋₄ alkyl group, or
   (iv) a C₆₋₁₈ aryl-C₁₋₄ alkyl group optionally substituted by substituent(s) selected from
      (a) carboxy,
      (b) C₁₋₄ alkoxy-carbonyl, and
      (c) -CO-NR⁸-(CH₂) ₙ-O-C₁₋₄ alkyl,
   wherein n is an integer of 1 to 4, and R⁸ is a hydrogen atom or a C₁₋₄ alkyl group; and
   R² and R³ are optionally bonded to form C₂₋₄ alkylene.
(H) A compound (I) wherein W is CH;
   A is a C₆₋₁₈ aryl group optionally substituted by substituent(s) selected from
   (a) carboxy,
   (b) C₁₋₄ alkoxy-carbonyl,
   (c) a 5- to 8-membered heterocyclyl-carbonyl group containing 1 to 3 hetero atoms selected from a nitrogen atom, an oxygen atom and a sulfur atom (preferably, a 5- to 8-membered cyclic amino-carbonyl group optionally having 1 or 2 hetero atoms selected from a nitrogen atom, an oxygen atom and a sulfur atom), which is optionally substituted by C₆₋₁₈ aryl-C₁₋₄ alkyl,
   (d) a carbamoyl group optionally substituted by C₆₋₁₈ aryl-C₁₋₄ alkyl, and
   (e) a ureido group optionally substituted by C₆₋₁₈ aryl-C₁₋₄ alkyl;
   X¹ is -NR³' - wherein R³' is a hydrogen atom or a C₁₋₆ alkyl group; and
   R² is a hydrogen atom.

### [Compound (Ii)]

A compound (I) wherein A is a C₆₋₁₈ aryl group substituted by substituent(s) selected from
(i) a phenoxy group substituted by 1 to 5 substituents selected from
   (a) halogen,
   (b) optionally halogenated C₁₋₄ alkyl,
   (c) hydroxy-C₁₋₄ alkyl,
   (d) heterocyclyl-C₁₋₄ alkyl (preferably, 5- to 8-membered heterocyclyl-C₁₋₄ alkyl, said 5- to 8-membered heterocycle has 1 to 3 hetero atoms selected from a nitrogen atom, an oxygen atom and an optionally oxidized sulfur atom, such as imidazolyl, triazolyl and the like),
   (e) optionally halogenated C₁₋₄ alkoxy,
   (f) C1₋₄ alkyl-carbonyl,
   (g) cyano,
   (h) carbamoyl optionally substituted by C₁₋₈ alkyl, and
   (i) C₁₋₄ alkoxy-carbonyl,
(ii) a phenyl-C₁₋₃ alkoxy group substituted by 1 to 5 substituents selected from
   (a) halogen,
   (b) optionally halogenated C₁₋₄ alkyl,
   (c) hydroxy-C₁₋₄ alkyl,
   (d) heterocyclyl-C₁₋₄ alkyl (preferably, 5- to 8-membered heterocyclyl-C₁₋₄ alkyl, said 5- to 8-membered heterocycle has 1 to 3 hetero atoms selected from a nitrogen atom, an oxygen atom and an optionally oxidized sulfur atom, such as imidazolyl, triazolyl and the like),
   (e) optionally halogenated C₁₋₄ alkoxy,
   (f) C₁₋₄ alkyl-carbonyl,
   (g) cyano,
   (h) carbamoyl optionally substituted by C₁₋₈ alkyl, and
   (i) C₁₋₄ alkoxy-carbonyl,
(iii) a 5- to 8-membered heterocycleoxy group containing 1 to 3 hetero atoms selected from a nitrogen atom, an oxygen atom and a sulfur atom, which is substituted by 1 to 5 substituents selected from
   (a) halogen,
   (b) optionally halogenated C₁₋₄ alkyl,
   (c) hydroxy-C₁₋₄ alkyl,
   (d) heterocyclyl-C₁₋₄ alkyl (preferably, 5- to 8-membered heterocyclyl-C₁₋₄ alkyl, said 5- to 8-membered heterocycle has 1 to 3 hetero atoms selected from a nitrogen atom, an oxygen atom and an optionally oxidized sulfur atom, such as imidazolyl, triazolyl and the like),
   (e) optionally halogenated C₁₋₄ alkoxy,
   (f) C1₋₄ alkyl-carbonyl,
   (g) cyano,
   (h) carbamoyl optionally substituted by C₁₋₈ alkyl, and
   (i) C₁₋₄ alkoxy-carbonyl, and
(iv) 5- to 8-membered heterocyclyl-C₁₋₃ alkoxy containing 1 to 3 hetero atoms selected from a nitrogen atom, an oxygen atom and a sulfur atom, which is substituted by 1 to 5 substituents selected from
   (a) halogen,
   (b) optionally halogenated C₁₋₄ alkyl,
   (c) hydroxy-C₁₋₄ alkyl,
   (d) heterocyclyl-C₁₋₄ alkyl (preferably, 5- to 8-membered heterocyclyl-C₁₋₄ alkyl, said 5- to 8-membered heterocycle has 1 to 3 hetero atoms selected from a nitrogen atom, an oxygen atom and an optionally oxidized sulfur atom, such as imidazolyl, triazolyl and the like),
   (e) optionally halogenated C₁₋₄ alkoxy,
   (f) C₁₋₄ alkyl-carbonyl,
   (g) cyano,
   (h) carbamoyl optionally substituted by C₁₋₈ alkyl, and
   (i) C₁₋₄ alkoxy-carbonyl;
   wherein the C₆₋₁₈ aryl group is optionally further substituted by 1 to 4 substituents selected from halogen and optionally halogenated C₁₋₄ alkyl;
   R¹ is a hydrogen atom;
   R² is a C₁₋₈ alkyl group, a C₂₋₈ alkenyl group or a C₂₋₈ alkynyl group, each of which is substituted by substituent(s) selected from
   (a) hydroxy,
   (b) carboxy,
   (c) cyano,
   (d) optionally halogenated C₁₋₄ alkoxy,
   (e) -O- (CH₂)ₙ-OH,
   (f) -0- (CH₂)ₙ-O-CO-NH₂,
   (g) -O- (CH₂)ₙ-O- (optionally halogenated C₁₋₄ alkyl),
   (h) -O- (CH₂)ₙ-SO₂- (optionally halogenated C₁₋₄ alkyl),
   (i) -O- (CH₂)ₙ-SO₂-C₆₋₁₈ aryl,
   (j) -O- (CH₂)ₙ-SO₂- (CH₂)ₙ-OH,
   (k) -O- (CH₂) ₙ-NR⁸-CO-C₁₋₄ alkyl,
   (l) -O- (CH₂) ₙ-NR⁸-CO- (CH₂) ₙ-SO₂-C₁₋₄ alkyl,
   (m) -O- (CH₂) ₙ-NR⁸-SO₂- (optionally halogenated C₁₋₄ alkyl),
   (n) -CO-NR⁸- (CH₂) ₙ-OH,
   (o) -CO-NR⁸- (CH₂) ₙ-SO₂- (optionally halogenated C₁₋₄ alkyl),
   (p) -CO-NR⁸-O-C₁₋₄ alkyl,
   (q) - NR⁶R⁷ ,
   (r) -NR⁸- (CH₂)ₙ-OH,
   (s) -NR⁸-(CH₂)ₙ-SO₂-C₁₋₄ alkyl,
   (t) -NR⁸-CO- (optionally halogenated C₁₋₄ alkyl),
   (u) -NR⁸-CO- (CH₂ ) ₙ-OH,
   (v) -NR⁸-CO- (CH₂) ₙ-CN,
   (w) -NR⁸-CO- (CH₂)ₙ-NR⁶R⁷,
   (x) -NR⁸-CO- (CH₂) ₙ-O-C₁₋₄ alkyl,
   (y) -NR⁸-CO- (CH₂) ₙ-SO- (optionally halogenated C₁-₄ alkyl),
   (z) -NR⁸-CO- (CH₂) ₙ-SO₂- (optionally halogenated C₁₋₄ alkyl),

   (aa) -NR⁸-CO- (CH₂) ₙ-SO₂-C₃₋₈ cycloalkyl,
   (bb) -NR⁸-CO- (CH₂) ₙ-NR⁸-SO₂-C₁₋₄ alkyl,
   (cc) -NR⁸-CO₂- (CH₂) ₙ-SO₂-C₁₋₄ alkyl,
   (dd) -NR⁸-CO-NH- (CH₂) ₙ-SO₂-C₁₋₄ alkyl,
   (ee) -NR⁸-CO-NH-O-C₁₋₄ alkyl,
   (ff) -NR⁸-CO-NH-(CH₂)ₙ-O-C₁₋₄ alkyl,
   (gg) -NR⁸-C (=NH) -NH-C₁₋₄ alkyl,
   (hh) -NR⁸-SO₂- (CH₂) ₙ-SO₂-C₁₋₄ alkyl,
   (ii) -S- (CH₂) ₙ-OH,
   (JJ) -SO- (CH₂) ₙ-OH,
   (kk) -SO₂- (CH_{2) n}-OH, and
   (11) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-O-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
   wherein n is an integer of 1 to 4, R⁶ and R⁷ are the same or different and each is a hydrogen atom or a C₁₋₄ alkyl group, R⁸ is a hydrogen atom or a C₁₋₄ alkyl group,
   (CH₂)ₙ is optionally substituted by optionally halogenated C₁₋₄ alkyl or hydroxy, and when n is not less than 2, a subset -CH₂CH₂- of (CH₂)ₙ is optionally replaced by -CH=CH-;
   R³ is a hydrogen atom or a C₁₋₆ alkyl group; or
   R¹ and R² are optionally bonded to form 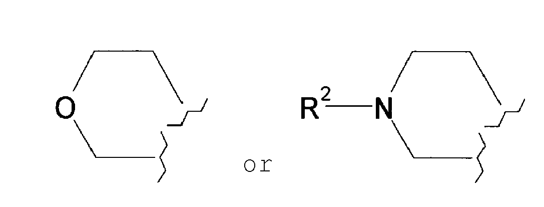
R² and R³ are optionally bonded to form C₂₋₄ alkylene optionally substituted by an imino group,
particularly preferably, R² is a C₁₋₈ alkyl group, a C₂₋₈ alkenyl group or a C₂₋₈ alkynyl group (particularly, a C₁₋₈ alkyl group), each of which is optionally substituted by substituent(s) selected from
(a) hydroxy,
(b) carboxy,
(c) cyano,
(d) optionally halogenated C₁₋₄ alkoxy,
(e) -O- (CH_{2) n}-OH (wherein (CH₂)ₙ is optionally substituted by hydroxy),
(f) -O- (CH₂) ₙ-O-CO-NH₂,
(g) -O- (CH₂) ₙ-O- (optionally halogenated C₁₋₄ alkyl),
(h) -O- (CH₂) ₙSO₂- (optionally halogenated C₁₋₄ alkyl),
(i) -O- (CH2₎ n-SO₂-C₆₋₁₈ aryl,
(j) -O- (CH₂) ₙ-SO₂- (CH₂) ₙ-OH,
(k) -O- (CH₂) ₙ-NR⁸-CO-C₁₋₄ alkyl,
(l) -O-(CH₂)ₙ-NR⁸-CO-(CH₂)ₙ-SO₂-C₁₋₄ alkyl,
(m) -O- (CH₂) ₙ-NR⁸-SO₂- (optionally halogenated C₁₋₄ alkyl),
(n) -CO-NR⁸- (CH₂) ₙ-OH,
(o) -CO-NR⁸- (CH₂) ₙ-SO₂- (optionally halogenated C₁₋₄ alkyl) ,
(p) -CO-NR⁸-O-C₁₋₄ alkyl,
(q) - NR⁶R⁷,
(r) -NR⁸- (CH₂) ₙ-OH,
(s) -NR⁸- (CH₂) ₙ-SO₂-C₁₋₄ alkyl,
(t) -NR⁸-CO- (optionally halogenated C₁₋₄ alkyl),
(u) -NR⁸-CO- (CH_{2) n}-OH (wherein (CH₂)ₙ is optionally substituted by optionally halogenated C₁₋₄ alkyl or hydroxy),
(v) -NR⁸-CO- (CH₂) ₙ-CN,
(w) -NR⁸-CO-(CH₂)ₙ-NR⁶R⁷ (when n is not less than 2, a subset -CH₂CH₂- of (CH₂)ₙ is optionally replaced by -CH=CH-),
(x) -NR⁸-CO- (CH₂) ₙ-O-C₁₋₄ alkyl,
(y) -NR⁸-CO- (CH₂) ₙ-SO- (optionally halogenated C₁₋₄ alkyl),
(z) -NR⁸-CO-(CH₂)ₙ-SO₂- (optionally halogenated C₁₋₄ alkyl)
   (wherein (CH₂)ₙ is optionally substituted by C₁₋₄ alkyl),

(aa) -NR⁸-CO- (CH₂) ₙ-SO₂-C₃₋₈ cycloalkyl,
(bb) -NR⁸-CO- (CH₂) ₙ-NR⁸- SO₂-C₁₋₄ alkyl,
(cc) -NR⁸-CO₂- (CH₂) ₙ-SO₂-C₁₋₄ alkyl,
(dd) -NR⁸-CO-NH- (CH₂) ₙ-SO₂-C₁₋₄ alkyl,
(ee) -NR⁸-CO-NH-O-C₁₋₄ alkyl,
(ff) -NR⁸-CO-NH-(CH₂)ₙ-O-C₁₋₄ alkyl,
(gg) -NR⁸-C (=NH) -NH-C₁₋₄ alkyl,
(hh) -NR⁸-SO₂- (CH₂) ₙ-SO₂-C₁₋₄ alkyl,
(ii) -S-(CH₂) ₙ-OH,
(jj) -SO- (CH₂) ₙ-OH,
(kk) -SO₂- (CH₂) ₙ-OH, and
(11) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-O-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
wherein n is an integer of 1 to 4, R⁶ and R⁷ are the same or different and each is a hydrogen atom or a C₁₋₄ alkyl group, and R⁸ is a hydrogen atom or a C₁₋₄ alkyl group.

### [Compound (Ij)]

A compound (I) wherein
A is a C₆₋₁₈ aryl group substituted by substituent (s) selected from
(i) a phenoxy group substituted by 1 to 5 substituents selected from
   (a) halogen,
   (b) optionally halogenated C₁₋₄ alkyl,
   (c) hydroxy-C₁₋₄ alkyl,
   (d) heterocyclyl-C₁₋₄ alkyl (preferably, 5- to 8-membered heterocyclyl-C₁₋₄ alkyl, said 5- to 8-membered heterocycle has 1 to 3 hetero atoms selected from a nitrogen atom, an oxygen atom and an optionally oxidized sulfur atom, such as imidazolyl and the like),
   (e) optionally halogenated C₁₋₄ alkoxy,
   (f) cyano,
   (g) carbamoyl optionally substituted by C₁₋₈ alkyl, and
   (h) C₁₋₄ alkoxy-carbonyl,
(ii) a phenyl-C₁₋₃ alkoxy group substituted by 1 to 5 substituents selected from
   (a) halogen,
   (b) optionally halogenated C₁₋₄ alkyl,
   (c) hydroxy-C₁₋₄ alkyl,
   (d) heterocyclyl-C₁₋₄ alkyl (preferably, 5- to 8-membered heterocyclyl-C₁₋₄ alkyl, said 5- to 8-membered heterocycle has 1 to 3 hetero atoms selected from a nitrogen atom, an oxygen atom and an optionally oxidized sulfur atom, such as imidazolyl and the like),
   (e) optionally halogenated C₁₋₄ alkoxy,
   (f) cyano,
   (g) carbamoyl optionally substituted by C₁₋₈ alkyl, and
   (h) C₁₋₄ alkoxy-carbonyl,
(iii) a 5- to 8-membered heterocycleoxy group containing 1 to 3 hetero atoms selected from a nitrogen atom, an oxygen atom and a sulfur atom, which is substituted by 1 to 5 substituents selected from
   (a) halogen,
   (b) optionally halogenated C₁₋₄ alkyl,
   (c) hydroxy-C₁₋₄ alkyl,
   (d) heterocyclyl-C₁₋₄ alkyl (preferably, 5- to 8-membered heterocyclyl-C₁₋₄ alkyl, said 5- to 8-membered heterocycle has 1 to 3 hetero atoms selected from a nitrogen atom, an oxygen atom and an optionally oxidized sulfur atom, such as imidazolyl and the like),
   (e) optionally halogenated C₁₋₄ alkoxy,
   (f) cyano,
   (g) carbamoyl optionally substituted by C₁₋₈ alkyl, and
   (h) C₁₋₄ alkoxy-carbonyl, and
(iv) 5- to 8-membered heterocyclyl-C₁₋₃ alkoxy containing 1 to 3 hetero atoms selected from a nitrogen atom, an oxygen atom and a sulfur atom, which is substituted by 1 to 5 substituents selected from
   (a) halogen,
   (b) optionally halogenated C₁₋₄ alkyl,
   (c) hydroxy-C₁₋₄ alkyl,
   (d) heterocyclyl-C₁₋₄ alkyl (preferably, 5- to 8-membered heterocyclyl-C₁₋₄ alkyl having 1 to 3 hetero atoms selected from a nitrogen atom, an oxygen atom and an optionally oxidized sulfur atom, such as imidazolyl and the like),
   (e) optionally halogenated C₁₋₄ alkoxy,
   (f) cyano,
   (g) carbamoyl optionally substituted by C₁₋₈ alkyl, and
   (h) C₁₋₄ alkoxy-carbonyl;
   wherein the C₆₋₁₈ aryl group is optionally further substituted by 1 to 4 substituents selected from halogen and optionally halogenated C₁₋₄ alkyl;
   R¹ is a hydrogen atom;
   R² is a C₁₋₈ alkyl group, a C₂₋₈ alkenyl group or a C₂₋₈ alkynyl group, each of which is substituted by substituent(s) selected from
   (a) hydroxy,
   (b) optionally halogenated C₁₋₄ alkoxy,
   (c) -O-(CH₂)ₙ-OH,
   (d) -O- (CH₂) ₙ-O-CO-NH₂,
   (e) -O- (CH₂) n-O-C₁₋₄ alkyl,
   (f) -O- (CH₂) ₙ-SO2- (optionally halogenated C₁₋₄ alkyl),
   (g) -O- (CH₂) ₙ-SO₂-C₆₋₁₈ aryl,
   (h) -O- (CH₂) ₙ-SO₂- (CH₂) ₙ-OH,
   (i) -O- (CH₂) ₙ-NR⁸-SO₂- (optionally halogenated C₁₋₄ alkyl),
   (j) -CO-NR⁸- (CH₂) ₙ-OH,
   (k) -CO-NR⁸- (CH₂) ₙ-SO₂- (optionally halogenated C₁₋₄ alkyl),
   (l) -NR⁶R⁷,
   (m) -NR⁸- (CH₂) ₙ-OH,
   (n) -NR⁸- (CH₂) ₙ-SO₂-C₁₋₄ alkyl,
   (o) -NR⁸-CO- (CH₂) ₙ-OH,
   (p) -NR⁸-CO- (CH₂) ₙ-O-C₁₋₄ alkyl,
   (q) -NR⁸-CO- (CH₂) ₙ-SO- (optionally halogenated C₁₋₄ alkyl) ,
   (r) -NR⁸-CO- (CH₂) ₙ-SO₂- (optionally halogenated C₁₋₄ alkyl),
   (s) -NR⁸-CO- (CH₂) ₙ-SO₂-C₃₋₈ cycloalkyl,
   (t) -NR⁸-CO₂- (CH₂) ₙ-SO₂-C₁₋₄ alkyl,
   (u) -NR⁸-CO-NH- (CH₂) ₙ-SO₂-C₁₋₄ alkyl,
   (v) -NR⁸-SO₂- (CH₂) ₙ-SO₂-C₁₋₄ alkyl,
   (w) -S- (CH₂) ₙ-OH,
   (x) -SO- (CH₂) ₙ-OH,
   (y) -SO₂- (CH₂) ₙ-OH, and
   (z) -NR⁸-CO-(optionally substituted heterocyclic group)
      (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
   wherein n is an integer of 1 to 4, R⁶ and R⁷ are the same or different and each is a hydrogen atom or a C₁₋₄ alkyl group, R⁸ is a hydrogen atom or a C₁₋₄ alkyl group, and (CH₂) is optionally substituted by C₁₋₄ alkyl or hydroxy;
   R³ is a hydrogen atom or a C₁₋₆ alkyl group; or
   R¹ and R² are optionally bonded to form 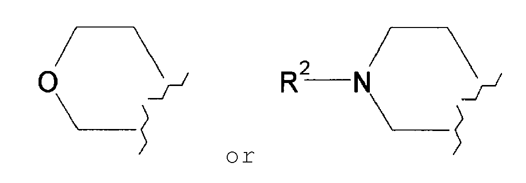 R² and R³ are optionally bonded to form C₂₋₄ alkylene.

Particularly preferably, R² is a C₁₋₈ alkyl group, a C₂₋₈ alkenyl group or a C₂₋₈ alkynyl group (particularly, a C₁₋₈ alkyl group), each of which is substituted by substituent(s) selected from
(a) hydroxy,
(b) optionally halogenated C₁₋₄ alkoxy,
(c) -O- (CH₂) ₙ-OH (wherein (CH₂)ₙ is optionally substituted by hydroxy),
(d) -O- (CH₂) ₙ-O-CO-NH₂,
(e) -O- (CH₂) ₙ-O-C₁₋₄ alkyl,
(f) -O- (CH₂) ₙ-SO₂- (optionally halogenated C₁₋₄ alkyl),
(g) -O- (CH₂) ₙ-SO₂-C₆₋₁₈ aryl,
(h) -O- (CH₂) ₙ-SO₂- (CH₂) ₙ-OH,
(i) -O- (CH₂) ₙ-NR⁸-SO₂- (optionally halogenated C₁₋₄ alkyl),
(j) -CO-NR⁸- (CH₂) ₙ-OH,
(k) -CO-NR⁸- (CH₂) ₙ-SO₂- (optionally halogenated C₁₋₄ alkyl) ,
(l) - NR ⁶R⁷ ,
(m) -NR⁸- (CH₂) ₙ-OH,
(n) -NR⁸-(CH₂)ₙ-SO₂-C₁₋₄ alkyl,
(o) -NR⁸-CO- (CH_{2) n}-OH (wherein (CH₂)ₙ is optionally substituted by C₁₋₄ alkyl),
(p) -NR⁸-CO- (CH₂) ₙ-O-C₁₋₄ alkyl,
(q) -NR⁸-CO- (CH_{2) n}-SO- (optionally halogenated C₁₋₄ alkyl),
(r) -NR⁸-CO- (CH₂) ₙ-SO₂- (optionally halogenated C₁₋₄ alkyl)
   (wherein (CH₂)ₙ is optionally substituted by C₁₋₄ alkyl),
(s) -NR⁸-CO- (CH₂) ₙ-SO₂-C₃₋₈ cycloalkyl,
(t) -NR⁸ -CO₂- (CH₂) ₙ-SO₂-C₁₋₄ alkyl,
(u) -NR⁸-CO-NH- (CH₂) ₙ-SO₂-C₁₋₄ alkyl,
(v) -NR⁸ -SO₂- (CH₂) ₙ-SO₂-C₁₋₄ alkyl,
(w) -S- (CH₂) ₙ-OH,
(x) -SO- (CH₂) ₙ-OH,
(y) -SO₂- (CH₂) ₙ-OH, and
(z) -NR⁸-CO-(optionally substituted heterocyclic group) (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
   wherein n is an integer of 1 to 4, R⁶ and R⁷ are the same or different and each is a hydrogen atom or a C₁₋₄ alkyl group, and R⁸ is a hydrogen atom or a C₁₋₄ alkyl group, and the like is preferable.

### [Compound (Ik)]

A compound (I) wherein
R² is (i) a C₅₋₈ alkyl group substituted by hydroxy,
(ii) a C₁₋₈ alkyl group substituted by substituent(s) selected from
   (a) halogenated C₁₋₄ alkoxy,
   (b) -O- (CH₂) ₙ-OH,
   (c) -O- (CH₂) ₙ-O-CO-NH₂ ,
   (d) -O- (CH₂) ₙ-O- (optionally halogenated C₁₋₄ alkyl),
   (e) -O- (CH₂) n-SO₂- (optionally halogenated C₁₋₄ alkyl),
   (f) -O- (CH₂) ₙ-SO₂-C₆₋₁₈ aryl,
   (g) -O- (CH₂) ₙ-NR⁸-SO₂- (optionally halogenated C₁₋₄ alkyl),
   (h) -CO-NR⁸- (CH₂) ₙ-OH,
   (i) -CO-NR⁸- (CH₂) ₙ-SO₂- (optionally halogenated C₁₋₄ alkyl),
   (j) -NR⁸- (CH₂) ₙ-SO₂-C₁₋₄ alkyl,
   (k) -NR⁸-CO- (CH₂) ₙ-OH,
   (l) -NR⁸-CO- (CH₂) ₙ-O-C₁₋₄ alkyl,
   (m) -NR⁸-CO- (CH₂) ₙ-SO- (optionally halogenated C₁₋₄ alkyl),
   (n) -NR⁸-CO- (CH₂) ₙ-SO₂- (optionally halogenated C₁₋₄ alkyl),
   (o) -NR⁸-CO- (CH₂) ₙ-SO₂-C₃₋₈ cycloalkyl,
   (p) -NR⁸-CO₂- (CH₂) ₙ-SO₂-C₁₋₄ alkyl,
   (q) -NR⁸-CO-NH- (CH₂) ₙ-SO₂-C₁₋₄ alkyl,
   (r) -NR⁸-SO₂- (CH₂) ₙ-SO₂-C₁₋₄ alkyl,
   (s) -S- (CH_{2) n}-OH,
   (t) -SO- (CH₂) ₙ-OH,
   (u) -SO₂- (CH₂) ₙ-OH, and
   (v) -NR⁸-CO-(optionally substituted heterocyclic group)
      (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
   wherein n is an integer of 1 to 4, R⁸ is a hydrogen atom or a C₁₋₄ alkyl group, and (CH₂) is optionally substituted by C₁₋₄ alkyl,
(iii) a C₂₋₈ alkenyl group optionally substituted by hydroxy, or
(iv) a C₂₋₈ alkynyl group optionally substituted by hydroxy.

Particularly preferably, R² is (i) a C₅₋₈ alkyl group substituted by hydroxy,
(ii) a C₁₋₈ alkyl group substituted by substituent(s) selected from
   (a) halogenated C₁₋₄ alkoxy,
   (b) -O- (CH_{2) n}-OH (wherein (CH₂)ₙ is optionally substituted by hydroxy),
   (c) -O- (CH₂) ₙ-O-CO-NH₂ ,
   (d) -O- (CH₂) ₙ-O- (optionally halogenated C₁₋₄ alkyl),
   (e) -O- (CH₂) ₙ-SO₂- (optionally halogenated C₁₋₄ alkyl),
   (f) -O- (CH₂) ₙ-SO₂-C₆₋₁₈ aryl,
   (g) -O- (CH₂) ₙ-NR⁸-SO₂- (optionally halogenated C₁₋₄ alkyl),
   (h) -CO-NR⁸- (CH₂) ₙ-OH,
   (i) -CO-NR⁸- (CH₂) ₙ-SO₂- (optionally halogenated C₁₋₄ alkyl),
   (j) -NR⁸- (CH₂) ₙ-SO₂-C₁₋₄ alkyl,
   (k) -NR⁸-CO-(CH₂)ₙ-OH (wherein (CH₂)ₙ is optionally substituted by C₁₋₄ alkyl),
   (l) -NR⁸-CO- (CH₂)ₙ-O-C₁₋₄ alkyl,
   (m) -NR⁸-CO- (CH₂) ₙ-SO- (optionally halogenated C₁₋₄ alkyl),
   (n) -NR⁸-CO- (CH₂) ₙ-SO₂- (optionally halogenated C₁₋₄ alkyl
      (wherein (CH₂)ₙ is optionally substituted by C₁₋₄ alkyl),
   (o) -NR⁸-CO- (CH₂) ₙ-SO₂-C₃₋₈ cycloalkyl,
   (p) -NR⁸-CO₂- (CH₂) ₙ-SO₂-C₁₋₄ alkyl,
   (q) -NR₈-CO-NH- (CH₂) ₙ-SO₂-C₁₋₄ alkyl,
   (r) -NR⁸-SO₂- (CH₂) ₙ-SO₂-C₁₋₄ alkyl,
   (s) -S- (CH₂) ₙ-OH,
   (t) -SO- (CH₂) ₙ-OH,
   (u) -SO₂- (CH₂) ₙ-OH, and
   (v) -NR⁸-CO-(optionally substituted heterocyclic group)
      (preferably, said heterocyclic group is a 5- to 8-membered heterocyclic group having 1 to 3 hetero atoms selected from a nitrogen atom, an oxygen atom and an optionally oxidized sulfur atom, which is optionally substituted by substituent(s) selected from hydroxy, C₁₋₄ alkyl, optionally oxidized C₁₋₄ alkylthio, -CO-C₁₋₄ alkyl, -CO-NH-C₁₋₄ alkyl, -CONH₂, -SO₂-C₁₋₄ alkyl, -SO₂-NH-C₁₋₄ alkyl, -SO₂NH₂ and the like),
   wherein n is an integer of 1 to 4, and R⁸ is a hydrogen atom or a C₁₋₄ alkyl group,

(iii) a C₂₋₈ alkenyl group optionally substituted by hydroxy, or
(iv) a C₂₋₈ alkynyl group optionally substituted by hydroxy.

As the salts of the compound (I), for example, metal salt, ammonium salt, salts with organic base, salts with inorganic acid, salts with organic acid, salts with basic or acidic amino acid and the like can be mentioned. As preferable examples of the metal salt, for example, alkali metal salts such as sodium salt, potassium salt and the like; alkaline earth metal salts such as calcium salt, magnesium salt, barium salt and the like; aluminum salt and the like can be mentioned. As preferable examples of the salts with organic base, for example, salts with trimethylamine, triethylamine, pyridine, picoline, 2,6-lutidine, ethanolamine, diethanolamine, triethanolamine, tromethamine [tris(hydroxymethyl)methylamine], t-butylamine, cyclohexylamine, dicyclohexylamine, N,N'-dibenzylethylenediamine and the like can be mentioned. As preferable examples of salts with inorganic acid, for example, salts with hydrochloric acid, hydrobromic acid, nitric acid, sulfuric acid, phosphoric acid and the like can be mentioned. As preferable examples of the salts with organic acid, for example, salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like can be mentioned. As preferable examples of the salts with basic amino acid, for example, salts with arginine, lysine, ornithine and the like can be mentioned, and as preferable examples of the salts with acidic amino acid, for example, salts with aspartic acid, glutamic acid and the like can be mentioned.

Of these, pharmaceutically acceptable salts are preferable. For example, when a compound contains an acidic functional group, inorganic salts such as alkali metal salts (e.g., sodium salt, potassium salt etc.), alkaline earth metal salts (e.g., calcium salt, magnesium salt, barium salt etc.) and the like, ammonium salt and the like, and when a compound contains a basic functional group, for example, salts with inorganic acid such as hydrochloric acid, hydrobromic acid, nitric acid, sulfuric acid, phosphoric acid and the like, or salts with organic acid such as acetic acid, phthalic acid, fumaric acid, oxalic acid, tartaric acid, maleic acid, citric acid, succinic acid, methanesulfonic acid, p-toluenesulfonic acid and the like can be mentioned.

As compound (I), preferred is a compound wherein A is an aryl group substituted by a group of the formula -Y²-B and optionally further substituted, wherein Y² is a single bond, -O-, -OCH₂-, -NH- or -S-, and B is an aryl group, a heterocyclic group, a C₃₋₈ cycloalkyl group, a carbamoyl group, a ureido group, a C₆₋₁₈ aryl-carbonyl group or a C₆₋₁₈ aryl-C₁₋₄ alkyl-carbonyl group, each of which is optionally substituted.

As a preferable embodiment of compound (I), a compound wherein W is C (R¹) ;
A is an aryl group substituted by a group of the formula -Y²-B, and optionally further substituted, wherein Y² is a single bond, -O-, -OCH₂-, -NH- or -S-, and B is an aryl group, a heterocyclic group, a C₃₋₈ cycloalkyl group, a carbamoyl group, a ureido group, a C₆₋₁₈ aryl-carbonyl group or a C₆₋₁₈ aryl-C₁₋₄ alkyl-carbonyl group, each of which is optionally substituted; R¹ is a hydrogen atom or a group of the formula -X²-R⁴ wherein x² is a single bond, -NH- or -O-, and R⁴ is a hydrogen atom or a C₁₋₈ alkyl group, a C₂₋₈ alkenyl group, a C₂₋₈ alkynyl group, a carbamoyl group, a C₁₋₈ alkyl-carbonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted;
R² is a hydrogen atom or a C₁₋₈ alkyl group, a C₂₋₈ alkenyl group, a C₂₋₈ alkynyl group, a carbamoyl group, a C₁₋₈ alkyl-carbonyl group, a C₁₋₈ alkyl-sulfonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a C₆₋₁₈ aryl-sulfonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted; and
X¹ is -NR³- wherein R³ is a hydrogen atom or an optionally substituted aliphatic hydrocarbon group, can be mentioned.

As another preferably embodiment of compound (I), a compound wherein
W is N;
X¹ is -NR³- wherein R³ is a hydrogen atom or an optionally substituted aliphatic hydrocarbon group;
A is an aryl group substituted by a group of the formula -Y²-B, and optionally further substituted, wherein Y² is a single bond, -O-, -OCH₂-, -NH- or -S-, and B is an aryl group, a heterocyclic group, a C₃₋₈ cycloalkyl group, a carbamoyl group, a ureido group, a C₆₋₁₈ aryl-carbonyl group or a C₆₋₁₈ aryl-C₁₋₄ alkyl-carbonyl group, each of which is optionally substituted; R² is a hydrogen atom or a C₁₋₈ alkyl group, a C₂₋₈ alkenyl group, a C₂₋₈ alkynyl group, a carbamoyl group, a C₁₋₈ alkyl-carbonyl group, a C₁₋₈ alkyl-sulfonyl group, a C₃₋₈ cycloalkyl group, a C₆₋₁₈ aryl group, a C₆₋₁₈ aryl-C₁₋₄ alkyl group, a C₆₋₁₈ aryl-carbonyl group, a C₆₋₁₈ aryl-C₁₋₄ alkyl-carbonyl group, a C₆₋18 aryl-sulfonyl group, a heterocyclic group, a heterocyclyl-C₁₋₄ alkyl group, a heterocyclyl-carbonyl group or a heterocyclyl-C₁₋₄ alkyl-carbonyl group, each of which is optionally substituted, can be mentioned.

As another preferable embodiment of compound (I), a compound wherein W is N;
X¹ is -NR³-;
A is an aryl group substituted by a group of the formula -Y²-B, and optionally further substituted, wherein Y² is a single bond, -O-, -OCH₂-, -NH- or -S-, and B is an aryl group, a heterocyclic group, a C₃₋₈ cycloalkyl group, a carbamoyl group, a ureido group, a C₆₋₁₈ aryl-carbonyl group or a C₆₋₁₈ aryl-C₁₋₄ alkyl-carbonyl group, each of which is optionally substituted; and R² and R³ are bonded to form an optionally substituted ring structure, can be mentioned.

As compound (I),
(i) 2-{2-[4-({3-chloro-4-[(3-fluorobenzyl)oxy]phenyl}amino)-5H-pyrrolo[3,2-d]pyrimidin-5-yl]ethoxy}ethanol,
(ii) 2-{2-[4-({3-chloro-4-[3-(trifluoromethyl)phenoxy]phenyl}amino)-5H-pyrrolo[3,2-d]pyrimidin-5-yl]ethoxy}ethanol,
(iii) N-{2- [4- ({3-chloro-4- [3-(trifluoromethyl)phenoxy]phenyl}amino)-5H-pyrrolo[3,2-d]pyrimidin-5-yl]ethyl}-3-hydroxy-3-methylbutanamide,
(iv) N-{2-[4-({3-chloro-4-[3-(trifluoromethyl)phenoxy]phenyl}amino)-5H-pyrrolo[3,2-d]pyrimidin-5-yl]ethyl}-2-(methylsulfonyl)acetamide,
(v) N-{2-[4-({3-chloro-4-[3-(trifluoromethyl)phenoxy]phenyl}amino)-5H-pyrrolo[3,2-d]pyrimidin-5-yl]ethyl}-2-methyl-2-(methylsulfonyl)propanamide,
(vi) 5-{2-[2-(tert-butylsulfonyl)ethoxy]ethyl}-N-{3-chloro-4-[3-(trifluoromethyl)phenoxy]phenyl}-5H-pyrrolo[3,2-d]pyrimidine-4-amine,
(vii) 2- (methylsulfonyl) -N-{2- [4- ({3-methyl-4- [3-(trifluoromethoxy)phenoxy]phenyl}amino)-5H-pyrrolo[3,2-d]pyrimidin-5-yl]ethyl}acetamide,
(viii) N-[2-(4-{[3-chloro-4-(3-chlorophenoxy)phenyl]amino}-5H-pyrrolo[3,2-d]pyrimidin-5-yl)ethyl]-2-(methylsulfonyl)acetamide, and
(ix) N-{2-[4-({3-chloro-4-[3-(trifluoromethoxy)phenoxy]phenyl}amino)-5H-pyrrolo[3,2-d]pyrimidin-5-yl]ethyl}-2-(methylsulfonyl)acetamide and the like are preferable. Particularly, N-{2-[4-({3-chloro-4-[3-(trifluoromethyl)phenoxy]phenyl}amino)-5H-pyrrolo[3,2-d]pyrimidin-5-yl]ethyl}-3-hydroxy-3-methylbutanamide is preferable.

Compound (I) and a salt thereof can be produced according to the method described in W02005/118588.

When compound (I) has isomers such as optical isomer, stereoisomer, positional isomer, rotational isomer and the like, and any isomers and mixtures are encompassed in the compound (I). For example, when compound (I) has an optical isomer, an optical isomer separated from a racemate is also encompassed in the compound (I). These isomers can be obtained as independent products by a synthesis means or a separation means (concentration, solvent extraction, column chromatography, recrystallization and the like) known *per se.*

The compound (I) may be a crystal, and both a single crystal and crystal mixtures are encompassed in the compound (I). Crystals can be produced by crystallization according to crystallization methods known *per se.*

The compound (I) may be a solvate (e.g., hydrate etc.) or a non-solvate, both of which are encompassed in the compound (I).

A compound labeled with an isotope (e.g., ³ H, ¹⁴C, ³⁵S, ¹²⁵I and the like) is also encompassed in the compound (I).

A prodrug of the compound (I) or a salt thereof (hereinafter referred to as compound (I)) means a compound which is converted to the compound (I) with a reaction due to an enzyme, an gastric acid, etc. under the physiological condition in the living body, that is, a compound which is converted to the compound (I) with oxidation, reduction, hydrolysis, etc. according to an enzyme; a compound which is converted to the compound (I) by hydrolysis etc. due to gastric acid, etc. A prodrug for compound (I) may be a compound obtained by subjecting an amino group in compound (I) to an acylation, alkylation or phosphorylation (e.g., a compound obtained by subjecting an amino group in compound (I) to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation and tert-butylation, etc.); a compound obtained by subjecting a hydroxy group in compound (I) to an acylation, alkylation, phosphorylation or boration (e.g., a compound obtained by subjecting an hydroxy group in compound (I) to an acetylation, palmitoylation, propanoylation, pivaloylation, succinylation, fumarylation, alanylation, dimethylaminomethylcarbonylation, etc.); a compound obtained by subjecting a carboxyl group in compound (I) to an esterification or amidation (e.g., a compound obtained by subjecting a carboxyl group in compound (I) to an ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification and methylamidation, etc.) and the like. Any of these compounds can be produced from compound (I) by a method known *per se.*

A prodrug for compound (I) may also be one which is converted into compound (I) under a physiological condition, such as those described in IYAKUHIN no KAIHATSU (Development of Pharmaceuticals), Vol. 7, Design of Molecules, p.163-198, Published by HIROKAWA SHOTEN (1990).

In the present specification, as examples of the "hormonal therapeutic agents" there may be mentioned fosfestrol, diethylstylbestrol, chlorotrianisene, medroxyprogesterone acetate, megestrol acetate, chlormadinone acetate, cyproterone acetate, danazol, dienogest, asoprisnil, allylestrenol, gestrinone, nomegestrol, Tadenan, mepartricin, raloxifene, ormeloxifene, levormeloxifene, anti-estrogens (e.g., tamoxifen citrate, toremifene citrate, and the like), ER down-regulator (e.g., fulvestrant (Faslodex (trademark)) and the like), human menopausal gonadotrophin, follicle stimulating hormone, pill preparations, mepitiostane, testrolactone, aminoglutethimide, LH-RH agonists (e.g., goserelin acetate, buserelin, leuprorelin, and the like), droloxifene, epitiostanol, ethinylestradiol sulfonate, aromatase inhibitors (e.g., fadrozole hydrochloride, anastrozole, retrozole, exemestane, vorozole, formestane, and the like), anti-androgens (e.g., flutamide, bicartamide, nilutamide, and the like), 5α-reductase inhibitors (e.g., finasteride, dutasteride, epristeride, and the like), adrenocorticohormone drugs (e.g., dexamethasone, prednisolone, betamethasone, triamcinolone, and the like), androgen synthesis inhibitors (e.g., abiraterone, and the like), retinoid and drugs that retard retinoid metabolism (e.g., liarozole, and the like), etc. and LH-RH agonists (e.g., goserelin acetate, buserelin, leuprorelin) and ER down-regulator (e.g., fulvestrant (Faslodex (trademark)) and the like) are preferable.

In the present specification, as the "anti-cancer agent", for example, chemotherapeutic agent, immunotherapeutic agent, a pharmaceutical agent that inhibits the action of cell growth factor and a receptor thereof and the like can be mentioned.

As examples of said "chemotherapeutic agents", there may be mentioned alkylating agents, antimetabolites, anticancer antibiotics, plant-derived anticancer agents, and the like.

As examples of "alkylating agents", there may be mentioned nitrogen mustard, nitrogen mustard-N-oxide hydrochloride, chlorambutyl, cyclophosphamide, ifosfamide, thiotepa, carboquone, improsulfan tosylate, busulfan, nimustine hydrochloride, mitobronitol, melphalan, dacarbazine, ranimustine, sodium estramustine phosphate, triethylenemelamine, carmustine, lomustine, streptozocin, pipobroman, etoglucid, carboplatin, cisplatin, miboplatin, nedaplatin, oxaliplatin, altretamine, ambamustine, dibrospidium hydrochloride, fotemustine, prednimustine, pumitepa, ribomustin, temozolomide, treosulphan, trophosphamide, zinostatin stimalamer, adozelesin, cystemustine, bizelesin, and the like.

As examples of "antimetabolites", there may be mentioned mercaptopurine, 6-mercaptopurine riboside, thioinosine, methotrexate, enocitabine, cytarabine, cytarabine ocfosfate, ancitabine hydrochloride, 5-FU drugs (e.g., fluorouracil, tegafur, UFT, doxifluridine, carmofur, gallocitabine, emmitefur, and the like), aminopterine, leucovorin calcium, tabloid, butocine, folinate calcium, levofolinate calcium, cladribine, emitefur, fludarabine, gemcitabine, hydroxycarbamide, pentostatin, piritrexim, idoxuridine, mitoguazone, thiazophrine, ambamustine, pemetrexed disodium (Alimta (trademark)) and the like.

As examples of "anticancer antibiotics", there may be mentioned actinomycin-D, actinomycin-C, mitomycin-C, chromomycin-A3, bleomycin hydrochloride, bleomycin sulfate, peplomycin sulfate, daunorubicin hydrochloride, doxorubicin hydrochloride (Adriacin (trademark)), aclarubicin hydrochloride, pirarubicin hydrochloride, epirubicin hydrochloride, neocarzinostatin, mithramycin, sarcomycin, carzinophilin, mitotane, zorubicin hydrochloride, mitoxantrone hydrochloride, idarubicin hydrochloride, and the like.

As examples of "plant-derived anticancer agents", there may be mentioned etoposide, etoposide phosphate, vinblastine sulfate, vincristine sulfate, vindesine sulfate, teniposide, paclitaxel (Taxol (trademark), docetaxel, vinorelbine, and the like.

As examples of said "immunotherapeutic agents (BRM)", there may be mentioned picibanil, krestin, sizofiran, lentinan, ubenimex, interferons, interleukins, macrophage colony-stimulating factor, granulocyte colony-stimulating factor, erythropoietin, lymphotoxin, BCG vaccine, *Corynebacterium parvum,* levamisole, polysaccharide K, procodazole, and the like.

As the "growth factor" in said "pharmaceutical agents inhibiting the action of cell growth factors or cell growth factor receptors", there may be mentioned any substances that promote cell proliferation, which are normally peptides having a molecular weight of not more than 20,000 that are capable of exhibiting their activity at low concentrations by binding to a receptor, including (1) EGF (epidermal growth factor) or substances possessing substantially the same activity as it [e.g., EGF, heregulin, and the like], (2) insulin or substances possessing substantially the same activity as it [e.g., insulin, IGF (insulin-like growth factor)-1, IGF-2, and the like], (3) FGF (fibroblast growth factor) or substances possessing substantially the same activity as it [e.g., acidic FGF, basic FGF, KGF (keratinocyte growth factor), FGF-10, and the like], (4) other cell growth factors [e.g., CSF (colony stimulating factor), EPO (erythropoietin), IL-2 (interleukin-2), NGF (nerve growth factor), PDGF (platelet-derived growth factor), TGFβ (transforming growth factor β), HGF (hepatocyte growth factor), VEGF (vascular endothelial growth factor), and the like], and the like.

As examples of said "growth factor receptors", there may be mentioned any receptors capable of binding to the aforementioned growth factors, including EGF receptor, heregulin receptor (HER2), insulin receptor, IGF receptor, FGF receptor-1 or FGF receptor-2, and the like.

As examples of said "pharmaceutical agent that inhibits the action of cell growth factor", HER2 antibody (trastuzumab (Herceptin (trademark)) etc.), imatinib mesylate, ZD1839 or EGFR antibody (cetuximab (Erbitux) (trademark)) etc.), antibody to VEGF (e.g., bevacizumab (Avastin)(trademark)), VEGFR antibody, VEGFR inhibitor, EGFR inhibitor (erlotinib (Tarceva)(trademark)), gefitinib (Iressa (trademark)) etc.) can be mentioned.

In addition to the aforementioned drugs, mTOR inhibitors (temsirolimus, rapamycin, and the like), Akt inhibitors, PI3 kinase inhibitors, L-asparaginase, aceglatone, procarbazine hydrochloride, protoporphyrin-cobalt complex salt, mercuric hematoporphyrin-sodium, topoisomerase I inhibitors (e.g., irinotecan hydrochloride (Topotecin (trademark), Campto (trademark), topotecan, and the like), topoisomerase II inhibitors (e.g., sobuzoxane, and the like), differentiation inducers (e.g., retinoid, vitamin D, and the like), angiogenesis inhibitors (e.g., thalidomide, SU11248, and the like), α-blockers (e.g., tamsulosin hydrochloride, naftopidil, urapidil, alfuzosin, terazosin, prazosin, silodosin, and the like) serine/threonine kinase inhibitor, endothelin receptor antagonist (e.g., atrasentan, and the like), proteasome inhibitor (e.g., bortezomib, and the like), Hsp 90 inhibitor (e.g., 17-AAG, and the like), spironolactone, minoxidil, 11α-hydroxyprogesterone, bone resorption inhibiting/metastasis suppressing agent (e.g., zoledronic acid, alendronic acid, pamidronic acid, etidronic acid, ibandronic acid, clodronic acid) and the like can be used.

Of those mentioned above, a hormonal therapeutic agent or anti-cancer agent (hereinafter to be abbreviated as a concomitant drug), ER down-regulator (for example, fulvestrant (Faslodex (trademark)) etc.), HER2 antibody (trastuzumab (Herceptin (trademark)) etc.), EGFR antibody (cetuximab (Erbitux (trademark) etc.), EGFR inhibitor (erlotinib (Tarceva (trademark), gefitinib (Iressa (trademark)) etc.), VEGFR inhibitor or a chemotherapeutic agent (paclitaxel (Taxol (trademark) etc.) is preferable.

Particularly, fulvestrant (Faslodex (trademark)), trastuzumab (Herceptin (trademark)), cetuximab (Erbitux (trademark)), erlotinib (Tarceva (trademark)), gefitinib (Iressa (trademark)), paclitaxel (Taxol (trademark)) and the like are preferable.

In addition, doxorubicin hydrochloride (Adriacin (trademark)), irinotecan hydrochloride (Topotecin (trademark), Campto (trademark)), 5FU, docetaxel and methotrexate are among the preferable examples.

When (1) an HER2 inhibitor having a pyrrolopyrimidine skeleton or pyrazolopyrimidine skeleton (hereinafter abbreviated as HER2 inhibitor) and (2) a concomitant drug are used in combination, the administration time of the HER2 inhibitor and the concomitant drug is not restricted, and the HER2 inhibitor and the concomitant drug can be administered to an administration subject simultaneously, or may be administered at staggered times. The dosage of the concomitant drug may be determined according to the dose clinically used, and can be appropriately selected depending on an administration subject, administration route, disease, combination and the like.

The administration mode of the HER2 inhibitor and the concomitant drug is not particularly restricted, and it is sufficient that the HER2 inhibitor and the concomitant drug are combined in administration. Examples of such administration mode include the following methods:
(1) The HER2 inhibitor and the concomitant drug are simultaneously produced to give a single preparation which is administered. (2) The HER2 inhibitor and the concomitant drug are separately produced to give two kinds of preparations which are administered simultaneously by the same administration route. (3) The HER2 inhibitor and the concomitant drug are separately produced to give two kinds of preparations which are administered by the same administration route only at the different times. (4) The HER2 inhibitor and the concomitant drug are separately produced to give two kinds of preparations which are administered simultaneously by different administration routes. (5) The HER2 inhibitor and the concomitant drug are separately produced to give two kinds of preparations which are administered by different administration routes at different times (e.g., the HER2 inhibitor and the concomitant drug are administered in this order, or in the reverse order).

The combination drug of the present invention is useful as a therapeutic agent suppressing growth of cancer expressing HER2 and/or EGFR kinase, and as an agent preventing hormone dependent cancer and hormone dependent cancer from transferring to hormone independent cancer. In addition, the combination drug is useful as a pharmaceutical agent since it shows low toxicity (e.g., acute toxicity, chronic toxicity, genetic toxicity, reproductive toxicity, cardiotoxicity, drug interaction, carcinogenicity and the like), high water solubility, and superior stability, in vivo kinetics (absorbability, distribution, metabolism, excretion and the like) and efficacy expression.

That is, the combination drug of the present invention can be used as a safe agent for the prophylaxis or treatment of diseases due to abnormal cell proliferation such as various cancers (particularly, breast cancer (e.g., invasive ductal carcinoma, ductal cancer in situ, inflammatory breast cancer etc.), prostate cancer (e.g., hormone-dependent prostate cancer, non-hormone dependent prostate cancer etc.), pancreatic cancer (e.g., pancreatic duct cancer etc.), gastric cancer (e.g., papillary adenocarcinoma, mucinous adenocarcinoma, adenosquamous carcinoma etc.), lung cancer (e.g., non-small cell lung cancer, small cell lung cancer, malignant mesothelioma etc.), colon cancer (e.g., gastrointestinal stromal tumor etc.), rectal cancer (e.g., gastrointestinal stromal tumor etc.), colorectal cancer (e.g., familial colorectal cancer, hereditary nonpolyposis colorectal cancer, gastrointestinal stromal tumor etc.), small intestinal cancer (e.g., non-Hodgkin lymphoma, gastrointestinal stromal tumor, etc.), esophagus cancer, duodenal cancer, cancer of the tongue, cancer of pharynx (e.g., nasopharyngeal carcinoma, oropharyngeal cancer, hypopharyngeal cancer etc.), salivary gland cancer, brain tumor (e.g., pineal astrocytoma, pilocytic astrocytoma, diffuse astrocytoma, anaplastic astrocytoma etc.), neurinoma, liver cancer (e.g., primary liver cancer, Extrahepatic Bile Duct Cancer etc.), kidney cancer (e.g., renal cell carcinoma, transitional cell cancers of renal pelvis and ureter etc.), cancer of the bile duct, endometrial carcinoma, cancer of the uterine cervix, ovarian cancer (e.g., ovarian epithelial cancer, extragonadal germ cell tumor, ovarian germ cell tumor, ovarian low malignant potential tumor etc.), urinary bladder cancer, urethral cancer, skin cancer (e.g., ocular melanoma, Merkel cell carcinoma etc.), hemangioma, malignant lymphoma, malignant melanoma, thyroid cancer (e.g., medullary thyroid carcinoma etc.), parathyroid cancer, nasal cavity cancer, paranasal sinus cancer, bone tumors (e.g., osteosarcoma, Ewing's tumor, uterus sarcoma, soft tissue sarcoma etc.), vascular fibroma, retinoblastoma, penile cancer, testis tumor, solid cancer in childhood (e.g., Wilms' tumor, childhood kidney tumor, etc.), Kaposi's sarcoma, Kaposi's sarcoma derived from AIDS, maxillary tumor, fibrous histiocytoma, leiomyosarcoma, rhabdomyosarcoma, leukemia (e.g., acute myeloid leukemia, acute lymphoblastic leukemia etc.) etc.), atherosclerosis, angiogenesis (e.g., angiogenesis associated with growth of solid cancer and sarcoma, angiogenesis associated with tumor metastasis, angiogenesis associated with diabetic retinopathy, etc.), and viral diseases (HIV infection etc.) and the like.

The tyrosine kinase dependent disease further includes cardiovascular diseases related to abnormal tyrosine kinase enzyme activity. Accordingly, the combination drug of the present invention can also be used as an agent for the prophylaxis or treatment of cardiovascular disease such as restenosis.

The combination drug of the present invention is useful as an anti-cancer agent for the prophylaxis or treatment of cancer, particularly breast cancer, ovarian cancer, prostate cancer, lung cancer, pancreatic cancer, kidney cancer, colorectal cancer, small intestinal cancer, esophagus cancer and gastric cancer and the like.

The combination drug of the present invention shows low toxicity and can be directly used as it is as a pharmaceutical agent or as a pharmaceutical composition containing a pharmaceutically acceptable carrier known *per* se etc. for a mammal (e.g., human, horse, bovine, dog, cat, rat, mouse, rabbit, swine, monkey etc.).

The combination drug of the present invention can be safely administered orally or parenterally (e.g., topical, rectal, intravenous administration etc.), for example, as a pharmaceutical composition obtained by mixing an HER2 inhibitor and/or the above-mentioned concomitant drug with a pharmacologically acceptable carrier according to a method known *per se,* such as tablet (including sugar-coated tablet, film-coated tablet), powder, granule, capsule (including soft capsule), liquid, injection, suppository, sustained-release preparation and the like. Injection can be administered intravenously, intramuscularly, subcutaneously, or by intraorgan administration or direct administration to the lesion.

As the pharmacologically acceptable carrier that may be used for the production of the combination drug of the present invention, various organic or inorganic carrier substances conventionally used as a preparation material can be mentioned. For example, excipient, lubricant, binder and disintegrant for solid preparations, solvent, dissolution aids, suspending agent, isotonicity agent, buffer and soothing agent for liquid preparations and the like can be mentioned. Where necessary, conventional additives such as preservatives, antioxidants, coloring agents, sweetening agents, adsorbing agents, wetting agents and the like can be used as appropriate in suitable amounts.

As the excipient, for example, lactose, sucrose, D-mannitol, starch, corn starch, crystalline cellulose, light anhydrous silicic acid and the like can be mentioned.

As the lubricant, for example, magnesium stearate, calcium stearate, talc, colloidal silica and the like can be mentioned.

As the binder, for example, crystalline cellulose, sucrose, D-mannitol, dextrin, hydroxypropylcellulose, hydroxypropylmethylcellulose, polyvinylpyrrolidone, starch, sucrose, gelatin, methylcellulose, carboxymethylcellulose sodium and the like can be mentioned.

As the disintegrant, for example, starch, carboxymethylcellulose, carboxymethylcellulose calcium, carboxymethyl starch sodium, L-hydroxypropylcellulose and the like can be mentioned.

As the solvent, for example, water for injection, alcohol, propylene glycol, macrogol, sesame oil, corn oil, olive oil and the like can be mentioned.

As the dissolution aids, for example, polyethylene glycol, propylene glycol, D-mannitol, benzyl benzoate, ethanol, trisaminomethane, cholesterol, triethanolamine, sodium carbonate, sodium citrate and the like can be mentioned.

As the suspending agent, for example, surfactant such as stearyltriethanolamine, sodium lauryl sulfate, lauryl aminopropionic acid, lecithin, benzalkonium chloride, benzethonium chloride, glyceryl monostearate and the like; hydrophilic polymer such as polyvinyl alcohol, polyvinylpyrrolidone, carboxymethylcellulose sodium, methylcellulose, hydroxymethylcellulose, hydroxyethylcellulose, hydroxypropylcellulose and the like; and the like can be mentioned.

As the isotonicity agent, for example, glucose, D-sorbitol, sodium chloride, glycerol, D-mannitol and the like can be mentioned.

As the buffer, for example, phosphate buffer, acetate buffer, carbonate buffer, citrate buffer and the like can be mentioned.

As the soothing agent, for example, benzyl alcohol and the like can be mentioned.

As the preservative, for example, paraoxybenzoic acid esters, chlorobutanol, benzyl alcohol, phenethyl alcohol, dehydroacetic acid, sorbic acid and the like can be mentioned.

As the antioxidant, for example, sulfite, ascorbic acid, α-tocopherol and the like can be mentioned.

The mixing ratio of an HER2 inhibitor and a concomitant drug in the combination drug of the present invention can be appropriately selected according to the subject of administration, administration route, disease and the like.

For example, while the content of the HER2 inhibitor in the combination drug of the present invention varies depending on the form of the preparation, it is generally about 0.01 to 100 wt%, preferably about 0.1 to 50 wt%, more preferably about 0.5 to about 20 wt%, relative to the whole preparation.

While the content of the concomitant drug in the combination drug of the present invention varies depending on the form of the preparation, it is generally about 0.01 to 100 wt%, preferably about 0.1 to 50 wt%, more preferably about 0.5 to about 20 wt%, relative to the whole preparation.

While the content of the additive such as a carrier in the combination drug of the present invention varies depending on the form of the preparation, it is generally about 1 to 99.99 wt%, preferably about 10 to about 90 wt%, relative to the whole preparation.

When an HER2 inhibitor and a concomitant drug are separately made into preparations, similar contents can be employed.

These preparations can be produced by a method known per se, which is generally used for a preparation step.

For example, an HER2 inhibitor or a concomitant drug can be prepared into an injection by forming an aqueous injection together with a dispersing agent (e.g., Tween 80 (manufactured by Atlas Powder, US), HCO 60 (manufactured by Nikko Chemicals), polyethylene glycol, carboxymethylcellulose, sodium alginate, hydroxypropylmethylcellulose, dextrin and the like), stabilizer (e.g., ascorbic acid, sodium pyrosulfite etc.), surfactant (e.g., polysorbate 80, macrogol etc.), solubilizer (e.g., glycerol, ethanol etc.), buffer (e.g., phosphoric acid and alkali metal salt thereof, citric acid and alkali metal salt thereof etc.), isotonicity agent (e.g., sodium chloride, potassium chloride, mannitol, sorbitol, glucose etc.), pH adjusting agent (e.g., hydrochloric acid, sodium hydroxide etc.), preservative (e.g., ethyl parahydroxybenzoate, benzoic acid, methyl parahydroxybenzoate, propyl parahydroxybenzoate, benzyl alcohol etc.), dissolving agent (e.g., concentrated glycerol, meglumine etc.), dissolution aids (e.g., propylene glycol, sucrose etc.), soothing agent (e.g., glucose, benzyl alcohol etc.) and the like, or an oil injection by dissolving, suspending or emulsifying in vegetable oil such as olive oil, sesame oil, cottonseed oil, corn oil and the like, and dissolution aids such as propylene glycol and the like.

A preparation for oral administration can be produced by adding, for example, an excipient (e.g., lactose, sucrose, starch, corn starch and the like), a disintegrating agent (e.g., starch, calcium carbonate and the like), a binder (e.g., starch, gum arabic, carboxymethylcellulose, polyvinylpyrrolidone, hydroxypropylcellulose, gelatin and the like), a lubricant (e.g., talc, magnesium stearate, polyethylene glycol 6000 and the like) and the like to an HER2 inhibitor or a concomitant drug according to a method known *per se,* and compression-molding the mixture, then when desired, coating the molder product by a method known *per* se for the purpose of masking of taste, enteric property or durability, to give a preparation for oral administration. As the coating agent, for example, hydroxypropylmethylcellulose, ethylcellulose, hydroxymethylcellulose, hydroxypropylcellulose, polyoxyethylene glycol, Tween 80, Pluronic F68, cellulose acetate phthalate, hydroxypropylmethylcellulose phthalate, hydroxymethylcellulose acetate succinate, Eudoragit (methacrylic acid acrylic acid copolymer, manufactured by Rohm, Germany), pigment (e.g., red iron oxide, titanium dioxide, etc.) and the like can be used. As the sugar coating, for example, saccharose, talc, gum arabic, pigment (e.g., red iron oxide, titanium dioxide etc.), polishing agent (e.g., beeswax etc.), and the like can be used. The preparation for oral administration may be any of an immediate-release preparation and a sustained-release preparation.

For example, for production of a suppository, an HER2 inhibitor or a concomitant drug can be formulated into an oily or aqueous solid, semi-solid or liquid suppository according to a method known *per se.* As the oily base to be used for the above-mentioned composition, for example, glycerides of higher fatty acids [e.g., cacao butter, Witepsols (manufactured by Dynamit Nobel, Germany), etc.], medium chain fatty acid [e.g., Miglyols (manufactured by Dynamit Nobel, Germany), etc.], or vegetable oils (e.g., sesame oil, soybean oil, cottonseed oil and the like), and the like are listed. Further, as the aqueous substrate, for example, polyethylene glycols, propylene glycol are listed, and as the aqueous gel substrate, for example, natural gums, cellulose derivatives, vinyl polymers, acrylic acid polymers and the like can be mentioned.

As the above-mentioned sustained-release preparation, sustained-release microcapsule and the like can be mentioned.

A sustained-release microcapsule can be prepared by a method known *per se.*

The HER2 inhibitor is preferably molded into an oral administration preparation such as a solid preparation (e.g., powder, granule, tablet, capsule) and the like, or molded into a rectal administration preparation such as a suppository. Particularly, an oral administration preparation is preferable.

The concomitant drug can be made into the above-mentioned drug form depending on the kind of the drug.

While the dose of the combination drug of the present invention varies depending on the kind of HER2 inhibitor, age, body weight, symptom, dosage form, administration method, administration period and the like, for example, it is generally, as an HER2 inhibitor and a concomitant drug, each within the range of about 0.1 mg - about 500 mg, preferably about 1 mg - about 100 mg, for oral administration, and each about 0.01 mg - about 100 mg, more preferably about 0.1 mg - about 10 mg for parenteral administration, for one patient (adult, body weight about 60 kg). The dose can be administered in 1 - 3 portions a day. Of course, since the dose as described above varies depending on various conditions, amounts smaller than the above-mentioned dosage may sometimes be sufficient, further, amounts over that range sometimes have to be administered.

The amount of the concomitant drug can be set at any value unless side effects are problematical. The daily dosage in terms of the concomitant drug differs depending on the severity of the symptom, age, sex, body weight, sensitivity difference of the subject, administration period, interval, and nature, pharmacy, kind of the pharmaceutical preparation, kind of effective ingredient, and the like, and not particularly restricted, and the amount of a drug is, in the case of oral administration for example, usually from about 0.001 to 2000 mg, preferably from about 0.01 to 500 mg, further preferably from about 0.1 to 100 mg, and in the case of parenteral administration for example, usually from about 0.0001 to 400 mg, preferably from about 0.001 to 200 mg per 1 kg of a mammal and this is usually administered once to 3 times in division a day.

For administration of the combination drug of the present invention, an HER2 inhibitor may be administered after administration of the concomitant drug or the concomitant drug may be administered after administration of an HER2 inhibitor, though they may be administered simultaneously. When administered at a time interval, the interval varies depending on the effective ingredient, dosage form and administration method, and, for example, when the concomitant drug is administered first, a method in which an HER2 inhibitor is administered within time range of from 1 minute to 3 days, preferably from 10 minutes to 1 day, more preferably from 15 minutes to 1 hour, after administration of the concomitant drug is exemplified. When an HER2 inhibitor is administered first, a method in which the concomitant drug is administered within time range of from 1 minute to 1 day, preferably from 10 minutes to 6 hours, more preferably from 15 minutes to 1 hour after administration of an HER2 inhibitor is exemplified.

As the preferable administration method, for example, about 0.001 - 200 mg/kg body weight of a concomitant drug formulated as a parenteral administration preparation is administered by intravenous injection or intramuscular injection and, about 15 min later, about 0.005 - 100 mg/kg body weight of an HER2 inhibitor formulated as an oral administration preparation is orally administered, for a daily dose.

### Examples

While the production method and method of use of the present invention are explained in the following Examples and Experimental Examples, the present invention is not limited to them. Other embodiments within the idea and scope of invention defined in the Claims are also encompassed in the present invention. Compound A in Examples and Experimental Examples means N-{2-[4-({3-chloro-4-[3-(trifluoromethyl)phenoxy]phenyl}amino)-5H-pyrrolo[3,2-d]pyrimidin-5-yl]ethyl}-3-hydroxy-3-methylbutanamide.

### Example 1

| | | |
|---|---|---|
| (1) | compound A | 8.0 g |
| (2) | lactose | 60.0 g |
| (3) | corn starch | 35.0 g |
| (4) | gelatin | 3.0 g |
| (5) | magnesium stearate | 2.0 g |

A mixture of compound A (8.0 g), lactose (60.0 g) and corn starch (35.0 g) is granulated using 10 wt% aqueous gelatin solution (30 mL, 3.0 g as gelatin) and, by passing through a 1 mm mesh sieve, dried at 40°C and sieved again. The obtained granules are mixed with magnesium stearate (2.0 g) and compression molded. The obtained core tablet is coated with a sugar coating of an aqueous suspension of saccharose, titanium dioxide, talc and gum arabic. The coated tablet is polished with beeswax to give 1000 coated tablets.

### Example 2

Trastuzmab (50 mg) is dissolved in Japanese Pharmacopoeia distilled water for injection (50 mL), and the Japanese Pharmacopoeia distilled water for injection is added to 100 mL. This solution is filtrated under sterile conditions. The solution (1 mL) is taken, filled in a vial for injection under sterile conditions, freeze-dried and sealed.

### Example 3

Cetuximab (50 mg) is dissolved in Japanese Pharmacopoeia distilled water for injection (50 mL), and the Japanese Pharmacopoeia distilled water for injection is added to 100 mL. This solution is filtrated under sterile conditions. The solution (1 mL) is taken, filled in a vial for injection under sterile conditions, freeze-dried and sealed.

### Example 4

| | | |
|---|---|---|
| (1) | compound A | 8.0 g |
| (2) | erlotinib | 8.0 g |
| (3) | lactose | 60.0 g |
| (4) | corn starch | 35.0 g |
| (5) | gelatin | 3.0 g |
| (6) | magnesium stearate | 2.0 g |

A mixture of compound A (8.0 g), erlotinib (8.0 g), lactose (60.0 g) and corn starch (35.0 g) is granulated using 10 wt% aqueous gelatin solution (30 mL, 3.0 g as gelatin) and, by passing through a 1 mm mesh sieve, dried at 40°C and sieved again. The obtained granules are mixed with magnesium stearate (2.0 g) and compression molded. The obtained core tablet is coated with a sugar coating of an aqueous suspension of saccharose, titanium dioxide, talc and gum arabic. The coated tablet is polished with beeswax to give 1000 coated tablets.

### Example 5

| | | |
|---|---|---|
| (1) | compound A | 8.0 g |
| (2) | gefitinib | 8.0 g |
| (3) | lactose | 60.0 g |
| (4) | corn starch | 35.0 g |
| (5) | gelatin | 3.0 g |
| (6) | magnesium stearate | 2.0 g |

A mixture of compound A (8.0 g), gefitinib (8.0 g), lactose (60.0 g) and corn starch (35.0 g) is granulated using 10 wt% aqueous gelatin solution (30 mL, 3.0 g as gelatin) and, by passing through a 1 mm mesh sieve, dried at 40°C and sieved again. The obtained granules are mixed with magnesium stearate (2.0 g) and compression molded. The obtained core tablet is coated with a sugar coating of an aqueous suspension of saccharose, titanium dioxide, talc and gum arabic. The coated tablet is polished with beeswax to give 1000 coated tablets.

### Example 6

Paclitaxel (50 mg) is dissolved in Japanese Pharmacopoeia distilled water for injection (50 mL), and the Japanese Pharmacopoeia distilled water for injection is added to 100 mL. This solution is filtrated under sterile conditions. The solution (1 mL) is taken, filled in a vial for injection under sterile conditions, freeze-dried and sealed.

### Example 7

| | | |
|---|---|---|
| (1) | compound A | 8.0 g |
| (2) | fulvestrant | 8.0 g |
| (3) | lactose | 60.0 g |
| (4) | corn starch | 35.0 g |
| (5) | gelatin | 3.0 g |
| (6) | magnesium stearate | 2.0 g |

A mixture of compound A (8.0 g), fulvestrant (8.0 g), lactose (60.0 g) and corn starch (35.0 g) is granulated using 10 wt% aqueous gelatin solution (30 mL, 3.0 g as gelatin) and, by passing through a 1 mm mesh sieve, dried at 40°C and sieved again. The obtained granules are mixed with magnesium stearate (2.0 g) and compression molded. The obtained core tablet is coated with a sugar coating of an aqueous suspension of saccharose, titanium dioxide, talc and gum arabic. The coated tablet is polished with beeswax to give 1000 coated tablets.

### Example 8

Doxorubicin hydrochloride (50 mg) is dissolved in Japanese Pharmacopoeia distilled water for injection (50 mL), and the Japanese Pharmacopoeia distilled water for injection is added to 100 mL. This solution is filtrated under sterile conditions. The solution (1 mL) is taken, filled in a vial for injection under sterile conditions, freeze-dried and sealed.

### Example 9

Irinotecan hydrochloride (50 mg) is dissolved in Japanese Pharmacopoeia distilled water for injection (50 mL), and the Japanese Pharmacopoeia distilled water for injection is added to 100 mL. This solution is filtrated under sterile conditions. The solution (1 mL) is taken, filled in a vial for injection under sterile conditions, freeze-dried and sealed.

### Example 10

| | | |
|---|---|---|
| (1) | compound A | 8.0 g |
| (2) | 5FU | 8.0 g |
| (3) | lactose | 60.0 g |
| (4) | corn starch | 35.0 g |
| (5) | gelatin | 3.0 g |
| (6) | magnesium stearate | 2.0 g |

A mixture of compound A (8.0 g), 5FU (8.0 g), lactose (60.0 g) and corn starch (35.0 g) is granulated using 10 wt% aqueous gelatin solution (30 mL, 3.0 g as gelatin) and, by passing through a 1 mm mesh sieve, dried at 40°C and sieved again. The obtained granules are mixed with magnesium stearate (2.0 g) and compression molded. The obtained core tablet is coated with a sugar coating of an aqueous suspension of saccharose, titanium dioxide, talc and gum arabic. The coated tablet is polished with beeswax to give 1000 coated tablets.

### Example 11

Docetaxel (50 mg) is dissolved in Japanese Pharmacopoeia distilled water for injection (50 mL), and the Japanese Pharmacopoeia distilled water for injection is added to 100 mL. This solution is filtrated under sterile conditions. The solution (1 mL) is taken, filled in a vial for injection under sterile conditions, freeze-dried and sealed.

### Example 12

| | | |
|---|---|---|
| (1) | compound A | 8.0 g |
| (2) | methotrexate | 8.0 g |
| (3) | lactose | 60.0 g |
| (4) | corn starch | 35.0 g |
| (5) | gelatin | 3.0 g |
| (6) | magnesium stearate | 2.0 g |

A mixture of compound A (8.0 g), methotrexate (8.0 g), lactose (60.0 g) and corn starch (35.0 g) is granulated using 10 wt% aqueous gelatin solution (30 mL, 3.0 g as gelatin) and, by passing through a 1 mm mesh sieve, dried at 40°C and sieved again. The obtained granules are mixed with magnesium stearate (2.0 g) and compression molded. The obtained core tablet is coated with a sugar coating of an aqueous suspension of saccharose, titanium dioxide, talc and gum arabic. The coated tablet is polished with beeswax to give 1000 coated tablets.

### Experimental Example 1

### Effect of combined use (in vitro) of compound A and trastuzumab on human breast cancer cell line BT-474

Human breast cancer cell line BT-474 (ATCC (American Type Culture Collection) catalog No.HTB-20, Lasfargues EY, In Vitro 15:723-729(1979)), which is a passage cultured HER2 high expressing cell, was treated with trypsin and suspended in RPMI-1640 medium (GibcoInvitrogen) containing 10% bovine fetus serum (GibcoInvitrogen). The cell density of the cell suspension was measured by cell counter Sysmex CDA500, and the cell density was adjusted to 4 x 10⁴ cell/mL using the aforementioned medium, which was dispensed to each well of a 96 well multi-well culture plate (Nunc) by 0.15 mL and cultured overnight at 37°C, 5% CO₂. Thereto was added a medium containing compound A 40 - 200 nmol/L at a given concentration, a medium containing trastuzumab (Herceptin (trademark) (Genentech, Inc.) catalog No. NDC 50242-134-68, 79 - 400 ng/mL) at a given concentration, or a medium containing compound A 40 - 200 nmol/L and trastuzumab 79 - 400 ng/mL at a given concentration. After culturing at 37°C, 5% CO₂ for 5 days, the cells of each well were fixed, stained and subjected to colorimetry. The effect of the combined use was determined by the method of Chou TC, Talalay P Adv. Enzyme Regul 22: 27-55 (1984) (Fig. 1).

Compound A showed a synergistic effect with trastuzumab on HER2 high expressing BT-474 breast cancer cell.

### Experimental Example 2

### Effect of combined use (in vivo) of compound A and trastuzumab on human breast cancer cell line BT-474

Human breast cancer cell line BT-474 (ATCC (American Type Culture Collection) catalog No.HTB-20, Lasfargues EY, In Vitro 15:723-729(1979)), which is a passage cultured HER2 high expressing cell, was treated with trypsin and suspended in RPMI-1640 medium (GibcoInvitrogen) containing 10% bovine fetus serum (GibcoInvitrogen). The cell density of the cell suspension was measured by cell counter Sysmex CDA500, and the cell density was adjusted to 1 x 10⁸ cell/mL using the aforementioned medium. This was diluted with the same amount of Matrigel (Basement Membrane Matrix, phenol-red free, Cat.No.40234C, Becton Dickinson) to give a cell suspension (5 x 10⁷ cell/mL) for nude mouse transplantation. As a nude mouse for transplantation, BALB/cAJcl-nu/nu, female, 5-week-old mice were purchased from CLEA Japan, Inc. After 1 week of acclimation on receipt of the mice, the above-mentioned cell suspension for transplantation was injected subcutaneously into the abdomen of 6-week-old mouse at a concentration of 1 x 10⁷ cell/200 **µ**L to transplant the cells. During transplantation, estradiol dipropionate (OVAHORMON DEPOT 5 mg ASKA Pharmaceutical Co., Ltd.) was intramuscularly injected into the right hind leg at a dose of 50 **µ**L. Further, at 1 week after the transplantation, estradiol dipropionate was intramuscularly injected similarly into the left hind leg at a dose of 50 **µ**L. The tumor volume was measured over time from 2 weeks after the transplantation and the mouse with a tumor volume of from 200 to 300 mm³ was used for the test.

Compound A was adjusted to a concentration of 10 mg/mL with 0.5% aqueous methylcellulose solution, and orally administered to mouse at a liquid amount of the above-mentioned compound A suspension of 10 mL/kg body weight. Compound A was administered twice a day in the morning and in the afternoon (administration interval was 7 hr or more) for 14 consecutive days. As for trastuzumab (Herceptin (trademark) (Genentech, Inc.) catalog No. NDC 50242-134-68), undiluted solution (21 mg/mL) was diluted to 1 mg/mL with saline, and intraperitoneally administered at a liquid amount of 10 mL/kg body weight. Trastuzumab was administered twice a week, totaling 4 times. The tumor volume was measured every 2 or 3 days, and the final tumor volume was measured on the next day of the completion of the 14 day administration and the effect of combined use was determined (Fig. 2).

Compound A showed a synergistic effect with trastuzumab on HER2 high expressing BT-474 breast cancer cell.

### Experimental Example 3

### Effect of combined use (in vivo) of compound A and trastuzumab on HER2 high expressing clinical gastric cancer cell line (4-1ST)

A tumor mass of 4-1ST (obtained from Central Institute for Experimental Animals), which was HER2 high expressing clinical gastric cancer cell line transplanted and passaged in vivo in nude mouse, was recovered from nude mouse, aseptically cut into 2 to 3 mm square pieces. The above-mentioned tumor piece was fixed on a trocar transplantation tool and transplanted subcutaneously into the abdomen of nude mouse. As a nude mouse for transplantation, BALB/cAJc 1-nu/nu, female, 5-week-old mice were purchased from CLEA Japan, Inc. After 1 week of acclimation on receipt of the mice, the tumor piece was transplanted subcutaneously into the abdomen of 6-week-old mouse. The tumor volume was measured over time from 2 weeks after the transplantation and the mouse with a tumor volume of from 200 to 300 mm³ was used for the test.

Compound A was adjusted to a concentration of 10 mg/mL with 0.5% aqueous methylcellulose solution, and orally administered to mouse at a liquid amount of the above-mentioned compound A suspension of 10 mL/kg body weight. Compound A was administered twice a day in the morning and in the afternoon (administration interval was 7 hr or more) for 14 consecutive days. As for trastuzumab (Herceptin (trademark) (Genentech, Inc.) catalog No. NDC 50242-134-68), undiluted solution (21 mg/mL) was diluted to 1 mg/mL with saline, and intraperitoneally administered at a liquid amount of 10 mL/kg body weight. Trastuzumab was administered twice a week, totaling 4 times. The tumor volume was measured every 2 or 3 days, and the final tumor volume was measured on the next day of the completion of the 14 day administration and the effect of combined use was determined (Fig. 3).

Compound A showed a synergistic effect with trastuzumab on HER2 high expressing 4-1ST gastric cancer tumor.

### Experimental Example 4

### Effect of combined use (in vivo) of compound A and cetuximab on HER2 high expressing clinical gastric cancer cell line (4-1ST)

A tumor mass of 4-1ST (obtained from Central Institute for Experimental Animals), which was HER2 high expressing clinical gastric cancer cell line transplanted and passaged in vivo in nude mouse, was recovered from nude mouse, aseptically cut into 2 to 3 mm square pieces. The above-mentioned tumor piece was fixed on a trocar transplantation tool and transplanted subcutaneously into the abdomen of nude mouse. As a nude mouse for transplantation, BALB/cAJc 1-nu/nu, female, 5-week-old mice were purchased from CLEA Japan, Inc. After 1 week of acclimation on receipt of the mice, the tumor piece was transplanted subcutaneously into the abdomen of 6-week-old mouse. The tumor volume was measured over time from 2 weeks after the transplantation and the mouse with a tumor volume of from 200 to 300 mm³ was used for the test.

Compound A was adjusted to a concentration of 10 mg/mL with 0.5% aqueous methylcellulose solution, and orally administered to mouse at a liquid amount of the above-mentioned compound A suspension of 10 mL/kg body weight. Compound A was administered twice a day in the morning and in the afternoon (administration interval was 7 hr or more) for 14 consecutive days. As for cetuximab (Erbitux (trademark)), undiluted solution (2 mg/mL) was intraperitoneally administered at a liquid amount of 10 mL/kg body weight. Cetuximab was administered twice a week, totaling 4 times. The tumor volume was measured every 2 or 3 days, and the final tumor volume was measured on the next day of the completion of the 14 day administration and the effect of combined use was determined (Fig. 4).

Compound A showed a synergistic effect with cetuximab on HER2 high expressing 4-1ST gastric cancer tumor.

### Experimental Example 5

### Effect of combined use (in vivo) of compound A and erlotinib on HER2 high expressing clinical gastric cancer cell line (4-1ST)

A tumor mass of 4-1ST (obtained from Central Institute for Experimental Animals), which was HER2 high expressing clinical gastric cancer cell line transplanted and passaged in vivo in nude mouse, was recovered from nude mouse, aseptically cut into 2 to 3 mm square pieces. The above-mentioned tumor piece was fixed on a trocar transplantation tool and transplanted subcutaneously into the abdomen of nude mouse. As a nude mouse for transplantation, BALB/cAJc 1-nu/nu, female, 5-week-old mice were purchased from CLEA Japan, Inc. After 1 week of acclimation on receipt of the mice, the tumor piece was transplanted subcutaneously into the abdomen of 6-week-old mouse. The tumor volume was measured over time from 2 weeks after the transplantation and the mouse with a tumor volume of from 200 to 300 mm³ was used for the test.

Compound A was adjusted to a concentration of 10 mg/mL with 0.5% aqueous methylcellulose solution, and orally administered to mouse at a liquid amount of the above-mentioned compound A suspension of 10 mL/kg body weight. Compound A was administered twice a day in the morning and in the afternoon (administration interval was 7 hr or more) for 14 consecutive days. Erlotinib (Tarceva (trademark)) was adjusted to a concentration of 2 mg/mL with 0.5% aqueous methylcellulose solution, and orally administered to mouse at a liquid amount of the above-mentioned erlotinib suspension of 10 mL/kg body weight. Erlotinib was administered twice a day in the morning and in the afternoon (administration interval was 7 hr or more) for 14 consecutive days. The tumor volume was measured every 2 or 3 days, and the final tumor volume was measured on the next day of the completion of the 14 day administration and the effect of combined use was determined (Fig. 5).

Compound A showed a synergistic effect with erlotinib on HER2 high expressing 4-1ST gastric cancer tumor.

### Experimental Example 6

### Effect of combined use (in vivo) of compound A and paclitaxel on human breast cancer cell line BT-474

Human breast cancer cell line BT-474 (ATCC (American Type Culture Collection) catalog No.HTB-20, Lasfargues EY, In Vitro 15:723-729(1979)), which is a passage cultured HER2 high expressing cell, was treated with trypsin and suspended in RPMI-1640 medium (GibcoInvitrogen) containing 10% bovine fetus serum (GibcoInvitrogen). The cell density of the cell suspension was measured by cell counter Sysmex CDA500, and the cell density was adjusted to 1 x 10⁸ cell/mL using the aforementioned medium. This was diluted with the same amount of Matrigel (Basement Membrane Matrix, phenol-red free, Cat.No.40234C, Becton Dickinson) to give a cell suspension (5 x 10⁷ cell/mL) for nude mouse transplantation. As a nude mouse for transplantation, BALB/cAJc 1-nu/nu, female, 5-week-old mice were purchased from CLEA Japan, Inc. After 1 week of acclimation on receipt of the mice, the above-mentioned cell suspension for transplantation was injected subcutaneously into the abdomen of 6-week-old mouse at a concentration of 1 x 10⁷ cell/200 **µ**L to transplant the cells. During transplantation, estradiol dipropionate (OVAHORMON DEPOT 5 mg ASKA Pharmaceutical Co., Ltd.) was intramuscularly injected into the right hind leg at a dose of 50 **µ**L. Further, at 1 week after the transplantation, estradiol dipropionate was intramuscularly injected similarly into the left hind leg at a dose of 50 **µ**L. The tumor volume was measured over time from 2 weeks after the transplantation and the mouse with a tumor volume of from 200 to 300 mm³ was used for the test. Compound A was adjusted to a concentration of 10 mg/mL with 0.5% aqueous methylcellulose solution, and orally administered to mouse at a liquid amount of the above-mentioned compound A suspension of 10 mL/kg body weight. Compound A was administered twice a day in the morning and in the afternoon (administration interval was 7 hr or more) for 14 consecutive days. Paclitaxel (Taxol (trademark)) (Cat.No.163-18614 Wako Pure Chemical Industries, Ltd.) was dissolved in cremophor-ethanol (1:1 v/v) solution to a concentration of 30 mg/mL, and this solution was used as an undiluted solution. Paclitaxel administration liquid was prepared by diluting the undiluted solution with saline to a concentration of 3 mg/mL, and the above-mentioned paclitaxel solution was intraperitoneally administered to a mouse at a liquid amount of 10 mL/kg body weight. Paclitaxel was administered twice a week, totaling 4 times. The tumor volume was measured every 2 or 3 days, and the final tumor volume was measured on the next day of the completion of the 14 day administration and the effect of combined use was determined (Fig. 6). It has been clarified that a stronger action on HER2 high expressing BT-474 breast cancer tumor as compared to the use of each pharmaceutical agent alone was afforded by a combined use of compound A and paclitaxel.

### Experimental Example 7

### Effect of combined use (in vitro) of compound A and fulvestrant on human breast cancer cell line BT-474

Human breast cancer cell line BT-474 was treated with trypsin and suspended in RPMI 1640 medium (Invitrogen) containing 10% bovine fetus serum (Invitrogen). BT-474 cell was plated on a 96 well plate at 6000 cells/100 **µ**L/well. The next day, fulvestrant alone, compound A alone or a mixture of fulvestrant and compound A was serially diluted and added. After addition of the compound, the mixture was stood in a CO₂ incubator for 5 days. 50 **µ**L of 25% glutaraldehyde solution (Wako) was added to 200 **µ**L of medium and the mixture was left standing at room temperature for 15 min. The plate was washed with PBS once, 200 **µ**L of PBS was added, 25 **µ**L of 50% trichloroacetic acid was added, and the mixture was left standing at 4°C for 1 hr or longer. The plate was washed 5 times with tap water, redundant water was removed by slamming the plate against Kimtowel. 50 **µ**L of 0.4% (w/v) Sulforhodamine B (SRB, Sigma)-containing 1% (v/v) acetic acid solution was added to each well and, 15 min later, the plate was washed 3 times with 1% acetic acid solution (v/v). The plate was thoroughly dried, 10 mM Tris solution was added, and the mixture was stirred well with a plate shaker, and the absorbance at 550 nm was measured (Bio-Rad, Benchmark Plus).

In calculation, a control free of a drug was 100% (Fig. 7). The combined use of compound A and fulvestrant revealed stronger cell growth inhibitory action as compared to use of each of them alone.

### Experimental Example 8

### Effect of combined use (in vitro) of compound A and fulvestrant on human breast cancer cell line MCF-7

Human breast cancer cell line MCF-7 cell was plated on a 96 well plate at 3000 cells/100 **µ**L/well. The next day, fulvestrant alone, compound A alone or a mixture of fulvestrant and compound A was serially diluted and added. After addition of the compound, the mixture was stood in a CO₂ incubator for 4 days. 50 **µ**L of 25% glutaraldehyde solution (Wako) was added to 200 **µ**L of medium and the mixture was left standing at room temperature for 15 min. The plate was washed with PBS once, 200 **µ**L of PBS was added, 25 **µ**L of 50% trichloroacetic acid was added, and the mixture was left standing at 4°C for 1 hr or longer. The plate was washed 5 times with tap water, redundant water was removed by slamming the plate against Kimtowel. 50 **µ**L of 0.4% (w/v) Sulforhodamine B (SRB, Sigma)-containing 1% (v/v) acetic acid solution was added to each well and, 15 min later, the plate was washed 3 times with 1% acetic acid solution (v/v). The plate was thoroughly dried, 10 mM Tris solution was added, and the mixture was stirred well with a plate shaker, and the absorbance at 550 nm was measured (Bio-Rad, Benchmark Plus).

In calculation, a control free of a drug was 100% (Fig. 8). The combined use of compound A and fulvestrant revealed stronger cell growth inhibitory action as compared to use of each of them alone.

### Experimental Example 9

### Induction of HER family genes by fulvestrant in human breast cancer cell line MCF-7

MCF-7 cell was plated on a 6 well plate at 8.6x 10⁴ cells/well. The next day, the cells were treated with compound A (1 **µ**M), fulvestrant (1 **µ**M) or compound A (1 **µ**M)+fulvestrant (1 **µ**M). After 120 hr, total RNA was extracted using an RNeasy mini kit (Qiagen). 1 **µ**g of total RNA was subjected to a Reverse Transcriptase reaction using a high capacity cDNA archive kit (P/N 4322171, Applied Biosystems) to give cDNA. The obtained cDNA was diluted 50-fold with Nuclease Free water (Promega), and 5 **µ**L thereof was used for PCR reaction. 2xTaqMan Universal PCR Master Mix (12.5 **µ**L) and 20xProbe & Primer (TaqMan Gene Expression Assays, 1.25 **µ**L) and Nuclease Free water (6.25 **µ**L) were blended. PCR reaction and analysis were performed using GeneAmp 7700 (Applied Biosystems). The reaction conditions were 50**°**Cx2 min, 95**°**Cx10 min, followed by 95**°**Cx15 sec, 60**°**Cx1 min, which cycle was repeated 40 times. For data analysis, calculation was made with GAPDH as an internal control and according to 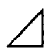 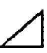 **Ct** method
(see Applied Biosystems) (Fig. 9).

When fulvestrant, which is a hormonal therapeutic agent, is administered alone, the HER family molecule may increase, the stimulation therefrom may be enhanced, and the effect may be attenuated. From the results, however, compound A was found to have an HER signal blocking action. Based on this action, an effect of combined use of compound A and hormonal therapeutic agent can be confirmed.

### Experimental Example 10

### Effect of combined use (in vitro) of compound A and chemotherapeutic agent on human cancer cell line

HER2 high expressing human cancer cell lines, BT-474 (breast cancer), N87 (gastric cancer), Calu3 (lung cancer) and OE19 (esophageal cancer), were purchased from ATCC and used for the experiments. Cell growth determination test was performed using each cell and in accordance with Experimental Example 1, and the growth suppressive action by compound A and various chemotherapeutic agents was examined. The effect of combined use was determined by the method of Chou TC, Talalay P Adv. Enzyme Regul 22:27-55 (1984) (Table 1). In the Table, Combination Index at the time of inhibition of cell growth by 50% is shown.

Compound A showed an additive effect with almost all the chemotherapeutic agents used on HER2 high expressing cancer cells.

**(Table 1)**

| Anti-cancer drug | cell line | | | |
|---|---|---|---|---|
| | BT-474 | N87 | Calu-3 | OE19 |
| paclitaxel | 1.10 | - | 1.08 | 1.22 |
| doxorubicin hydrochloride | 1.65 | 1.01 | 1.39 | 0.96 |
| irinotecan hydrochloride | - | 1.02 | 1. 68 | 0.83 |
| 5FU | - | 0.95 | - | 1.43 |
| docetaxel | 1.10 | 0.57 | - | 0.94 |
| methotrexate | - | 1.44 | 1.70 | 1.29 |

This application is based on patent application Nos. 2006-275841 and 2007-057902 filed in Japan, the contents of which are incorporated in full herein by this reference.

## Claims

1. A pharmaceutical agent comprising, in combination, (1) N-{2-[4-({3-chloro-4-[3-(trifluoromethyl)phenoxy]phenyl}amino)-5H-pyrrolo[3,2-d]pyrimidin-5-yl]ethyl}-3-hydroxy-3-methylbutanamide and (2) an anti-cancer agent selected from an HER2 antibody, an EGFR antibody, an EGFR inhibitor and a chemotherapeutic agent.

2. The pharmaceutical agent of claim 1, wherein the anti-cancer agent is trastuzumab, cetuximab, erlotinib, gefitinib or paclitaxel.

3. The pharmaceutical agent of claim 1, wherein the anti-cancer agent is trastuzumab.

4. The pharmaceutical agent of claim 1, wherein the anti-cancer agent is cetuximab.

5. The pharmaceutical agent of claim 1, wherein the anti-cancer agent is erlotinib.

6. The pharmaceutical agent of claim 1, wherein the anti-cancer agent is gefitinib.

7. The pharmaceutical agent of claim 1, wherein the anti-cancer agent is paclitaxel.
